# EUROPEAN PATENT APPLICATION

(11) **EP 4 321 180 A1**
(43) Date of publication of application: **14.02.2024**
(21) Application number: 22781627.9
(22) Date of filing: 30.03.2022
(51) Int. Cl.: A61K 47/68, A61K 47/65, A61K 31/5517, A61K 38/05, A61K 45/06, A61P 35/00

(54) **ANTIBODY-DRUG CONJUGATE INCLUDING ANTIBODY AGAINST HUMAN CLDN18.2, AND USE THEREOF**

(30) Priority: 30.03.2021 KR 20210041447
(71) Applicant: LegoChem Biosciences, Inc., Daejeon 34002 (KR); Nona Biosciences (Suzhou) Co., Ltd., Suzhou, Jiangsu 215123 (CN)
(72) Inventor: PARK, Chang Sik, Daejeon 34002 (KR); SONG, Ho Young, Daejeon 34002 (KR); JANG, Tae Ik, Daejeon 34002 (KR); CHUNG, Chul-Woong, Daejeon 34002 (KR); JHENG, Ming-jin, Suzhou, Anhui 215123 (CN)
(74) Representative: Vossius & Partner Patentanwälte Rechtsanwälte mbB
(86) International application number: PCT/KR2022/004552
(87) International publication number: WO 2022/211508

(57) **Abstract**

The present disclosure relates to a novel antibody-drug conjugate (ADC) targeting claudin 18 isoform 2 (CLDN18.2), an active metabolite of the ADC, a method of producing the ADC, use of the ADC for the treatment and/or prevention of diseases, and use of the ADC for producing a drug for the treatment and/or prevention of diseases, more specifically hyperproliferative and/or angiogenic diseases, for example, cancer, and more particularly, to an antibody-drug conjugate including a novel antibody or antigen-binding fragment thereof that binds to CLDN18.2, and a pharmaceutical composition including the same.

## Description

### [TECHNICAL FIELD]

The present disclosure relates to a novel antibody-drug conjugate (ADC) targeting claudin 18 isoform 2 (CLDN18.2), an active metabolite of the ADC, a method of producing the ADC, use of the ADC for the treatment and/or prevention of diseases, and use of the ADC for producing a drug for the treatment and/or prevention of diseases, more specifically hyperproliferative and/or angiogenic diseases, for example, cancer, and more particularly, to an antibody-drug conjugate including a novel antibody that binds to CLDN18.2 or an antigen-binding fragment thereof, and a pharmaceutical composition including the same.

### [BACKGROUND ART]

Cancer refers to a disease caused by abnormally grown lumps due to autonomous overgrowth of body tissues, and is the result of uncontrolled cell growth in various tissues. Tumors in early stage can be removed by surgical and radiotherapeutic measures, and metastasized tumors are generally treated palliatively using chemotherapy.

Most chemotherapeutic agents administered parenterally may induce unwanted side effects and even serious toxicity, as a result of systemic administration. Accordingly, the focus of development has been on developing novel chemotherapeutic agents to simultaneously achieve increased efficacy and minimal toxicity/side effects through the improved efficacy and selective application of these chemotherapeutic agents in tumor cells or immediately adjacent tissues.

An antibody-drug conjugate (ADC) is a new targeted technology for conjugating a toxin or a drug to an antibody that binds to an antigen, by which the toxin is released in a cell to cause death of cancer cells and the like. The ADC enables a drug to be accurately delivered to target cancer cells while minimally affecting healthy cells, and to be released only under specific conditions, and thus has excellent efficacy compared to antibody therapeutic agents themselves and can remarkably reduce the risk of side effects compared to existing anticancer agents.

The basic structure of these antibody-drug conjugates is "antibody-linker-small molecule drug (toxin)." In this structure, the linker needs to not only play a functional role in linking the antibody and the drug, but also ensure that the drug is released properly through antibody-drug dissociation (e.g., as a result of hydrolysis by an enzyme) after being circulated through the body and stably reaching target cells, and exhibits efficacy against target cancer cells. That is, the stability of the linker plays a very important role in the efficacy and safety such as systemic toxicity of an antibody-drug conjugate (Discovery Medicine 2010, 10(53): 329-39). 85-8 2018-08-14).

The inventors of the present disclosure developed a linker containing an effective self-immolative group, which is more stable in plasma and also stable in body circulation and enables a drug to be easily released in cancer cells to exhibit efficacy, and have secured patent therefor (Korean Patent Registration No. 1 ,628,872). Meanwhile, use of monoclonal antibodies for cancer treatment is having substantial success. Monoclonal antibodies are suitable for target-directed addressing of tumor tissue and tumor cells. Antibody-drug conjugates have become a novel and powerful option for the treatment of lymphomas and solid cancers, and immunomodulatory antibodies also have recently had considerable success in clinical trials. The development of therapeutic antibodies is based on deep understanding of cancer serology, protein engineering technology and the action thereof, mechanisms of resistance, and interactions between immune systems and cancer cells.

Antigens which are expressed on the surface of human cancer cells are defined as a broad range of targets which are over-expressed compared to normal tissues, mutated and selectively expressed. The key challenge is to identify antigens suitable for antibody-based therapies. These therapeutic agents mediate changes in antigen or receptor function (i.e., function as a stimulant or an antagonist), regulate the immune system through Fc and T cell activation, and exhibit efficacy through the delivery of specific drugs that bind to antibodies targeting specific antigens. Molecular techniques that can alter antibody pharmacokinetics, action function, size and immune stimulation are emerging as key factors in the development of novel antibody-based therapies. Evidences from clinical trials of therapeutic antibodies in cancer patients highlight the importance of approaches for selecting optimized antibodies, including affinity and binding of target antigens and antibodies, selection of an antibody structure, and therapeutic approaches (signaling blockade or immune function).

Human claudin 18 isoform 2 (CLDN18.2) is a transmembrane protein consisting of a full length of 261 amino acids with two extracellular loops and four transmembrane domains. CLDN18.2 differs in a part of the amino acid sequence up to extracellular loop domain 1, from CLDN18.1 expressed in some normal tissues.

CLDN18.2 belongs to the claudin family, which is an important component of tight junctions, and claudin proteins are expressed in various cancer tissues and are known to be potential targets for diagnostic and therapeutic modalities. CLDN18.2 is also known to play an important role in tumorigenesis and inflammatory responses, and changes in the expression of claudin proteins are known to cause dysfunction of tight junctions, affect signaling pathways, and have tumor-promoting action in some epithelial cancers. In addition, CLDN18.2 is specifically expressed in primary and metastatic gastric cancer, except for expression in the apical region of some gastric mucosa, is known as a cancer-specific antigen that is also associated with malignant transformation, and ectopic activation of CLDN18.2 has been reported in pancreatic cancer, esophageal cancer, ovarian cancer, lung cancer, and the like.

Under these technical backgrounds, the inventors of the present disclosure made efforts to develop an antibody that specifically binds to CLDN18.2, which shows tumor-specific expression distinguished from CLDN18.1 expressed also in normal tissues, and as a result, developed an anti-CLDN18.2 antibody that exhibits an excellent ability to bind to CLDN18.2, and confirmed that, by applying a linker containing an effective self-immolative group, which is more stable in plasma and also stable in body circulation to further strengthen the effect of the antibody and enables a drug to be easily released in cancer cells to exhibit efficacy, to an anticancer therapeutic antibody that specifically binds to CLDN18.2, a novel antibody-drug conjugate (ADC) targeting CLDN18.2, which is effective in the treatment and/or prevention of hyperproliferative and/or metastatic cancer diseases, can be provided, thus completing the present disclosure.

### [DETAILED DESCRIPTION OF DISCLOSURE]

### [TECHNICAL PROBLEM]

The present disclosure is to provide a novel antibody-linker-drug conjugate targeting claudin 18 isoform 2 (CLDN18.2), or a slat or solvate thereof.

The present disclosure is to provide an antibody-drug conjugate including an antibody that specifically binds to CLDN18.2 and a drug that binds thereto, and a pharmaceutical composition including the same.

Also, the present disclosure is to provide an antibody-linker-drug (toxin) system which, by applying the technology for a linker containing a self-immolative group, which is more stable in plasma and stable even in body circulation and enables a drug to be easily released in cancer cells to maximize efficacy, enables a drug and/or toxin to stably reach a target cell, and thus effectively exhibits efficacy and can remarkably reduce toxicity.

### [TECHNICAL SOLUTION]

An aspect of the present disclosure provides a conjugate represented by General Formula I below or a pharmaceutically acceptable salt or solvate thereof:

[General Formula I] Ab-[LINKER-(B)ₗ]m

wherein,
Ab is an anti-Claudin 18 isoform 2 antibody or antigen-binding fragment thereof including a heavy chain variable region and a light chain variable region,
wherein the heavy chain variable region may include a heavy chain CDR1 consisting of the amino acid sequence represented by SEQ ID NO: 2, a heavy chain CDR2 consisting of the amino acid sequence represented by SEQ ID NO: 4, and a heavy chain CDR3 consisting of the amino acid sequence represented by SEQ ID NO: 6, and
the light chain variable region may include a light chain CDR1 consisting of the amino acid sequence represented by SEQ ID NO: 9, a light chain CDR2 consisting of the amino acid sequence represented by SEQ ID NO: 11, and a light chain CDR3 consisting of the amino acid sequence represented by SEQ ID NO: 13,
LINKER may be a linker,
B may be an active agent, and
I and m may each independently be an integer selected from 1 to 20,
wherein, when I is an integer of 2 or more, at least two of B may be identical to or different from each other.

In an embodiment of the present disclosure, the anti-Claudin 18 isoform 2 antibody or antigen-binding fragment thereof may includes
a heavy chain variable region including heavy chain CDR1 consisting of the amino acid sequence represented by SEQ ID NO: 2, a heavy chain CDR2 consisting of the amino acid sequence represented by SEQ ID NO: 4, and a heavy chain CDR3 consisting of the amino acid sequence represented by SEQ ID NO: 6, and
a light chain variable region including light chain CDR1 consisting of the amino acid sequence represented by SEQ ID NO: 9, a light chain CDR2 consisting of the amino acid sequence represented by SEQ ID NO: 11, and a light chain CDR3 consisting of the amino acid sequence represented by SEQ ID NO: 13.

In addition, the heavy chain variable region may include a heavy chain FR of a human antibody, and a gene encoding the heavy chain FR may be derived from germline V gene IGHV3-23. Specifically, the heavy chain FR may include a heavy chain FR1 consisting of the amino acid sequence represented by SEQ ID NO: 1, a heavy chain FR2 consisting of the amino acid sequence represented by SEQ ID NO: 3, a heavy chain FR3 consisting of the amino acid sequence represented by SEQ ID NO: 5, and a heavy chain FR4 consisting of the amino acid sequence represented by SEQ ID NO: 7.

In addition, the light chain variable region may include a light chain FR of a human antibody, and a gene encoding the light chain FR may be derived from germline V gene IGKV3-11 or IGKV3-15. Specifically, the light chain FR may include a light chain FR1 consisting of the amino acid sequence represented by SEQ ID NO: 8, a light chain FR2 consisting of the amino acid sequence represented by SEQ ID NO: 10, a light chain FR3 consisting of the amino acid sequence represented by SEQ ID NO: 12, and a light chain FR4 consisting of the amino acid sequence represented by SEQ ID NO: 14.

In addition, the light chain F4 may include a moiety consisting of the amino acid sequence represented by SEQ ID NO: 19 at the C-terminus thereof for binding to a linker.

In an embodiment of the present disclosure, the antibody or antigen-binding fragment thereof that specifically binds to CLDN18.2 may include a heavy chain variable region consisting of: the amino acid sequence represented by SEQ ID NO: 15; or an amino acid sequence with at least 80% sequence identity to the amino acid sequence of SEQ ID NO: 15, and a light chain variable region consisting of: the amino acid sequence represented by SEQ ID NO: 16; or an amino acid sequence with at least 80% sequence identity to the amino acid sequence of SEQ ID NO: 16.

More specifically, the antibody or antigen-binding fragment thereof that specifically binds to CLDN18.2 includes a heavy chain variable region consisting of: the amino acid sequence represented by SEQ ID NO: 15; or an amino acid sequence with at least 80% sequence identity, at least 85% sequence identity, at least 90% sequence identity, or at least 95% sequence identity to the amino acid sequence of SEQ ID NO: 15, and a light chain variable region consisting of: the amino acid sequence represented by SEQ ID NO: 16; or an amino acid sequence with at least 80% sequence identity, at least 85% sequence identity, at least 90% sequence identity, or at least 95% sequence identity to the amino acid sequence of SEQ ID NO: 16, and specifically binds to CLDN18.2.

More specifically, the antibody or antigen-binding fragment thereof that specifically binds to CLDN18.2 includes a heavy chain variable region consisting of: the amino acid sequence represented by SEQ ID NO: 15; or an amino acid sequence with at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, at least 99% sequence identity, or at least 99.5% sequence identity to the amino acid sequence of SEQ ID NO: 15, and a light chain variable region consisting of: the amino acid sequence represented by SEQ ID NO: 16; or an amino acid sequence with at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, at least 99% sequence identity, or at least 99.5% sequence identity to the amino acid sequence of SEQ ID NO: 16, and specifically binds to CLDN18.2.

In an embodiment of the present disclosure, the antibody or antigen-binding fragment thereof that specifically binds to CLDN18.2 includes a heavy chain consisting of: the amino acid sequence represented by SEQ ID NO: 17; or an amino acid sequence with at least 80% sequence identity to the amino acid sequence of SEQ ID NO: 17, and a light chain consisting of: the amino acid sequence represented by SEQ ID NO: 18; or an amino acid sequence with at least 80% sequence identity to the amino acid sequence of SEQ ID NO: 18.

More specifically, the antibody or antigen-binding fragment thereof that specifically binds to CLDN18.2 includes a heavy chain consisting of: the amino acid sequence represented by SEQ ID NO: 17; or an amino acid sequence with at least 80% sequence identity, at least 85% sequence identity, at least 90% sequence identity, or at least 95% sequence identity to the amino acid sequence of SEQ ID NO: 17, and a light chain consisting of: the amino acid sequence represented by SEQ ID NO: 18; or an amino acid sequence with at least 80% sequence identity, at least 85% sequence identity, at least 90% sequence identity, or at least 95% sequence identity to the amino acid sequence of SEQ ID NO: 18, and specifically binds to CLDN18.2.

More specifically, the antibody or antigen-binding fragment thereof that specifically binds to CLDN18.2 includes a heavy chain consisting of: the amino acid sequence represented by SEQ ID NO: 17; or an amino acid sequence with at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, at least 99% sequence identity, or at least 99.5% sequence identity to the amino acid sequence of SEQ ID NO: 17, and a light chain consisting of: the amino acid sequence represented by SEQ ID NO: 18; or an amino acid sequence with at least 80% sequence identity, at least 81% sequence identity, at least 82% sequence identity, at least 83% sequence identity, at least 84% sequence identity, at least 85% sequence identity, at least 86% sequence identity, at least 87% sequence identity, at least 88% sequence identity, at least 89% sequence identity, at least 90% sequence identity, at least 91% sequence identity, at least 92% sequence identity, at least 93% sequence identity, at least 94% sequence identity, at least 95% sequence identity, at least 96% sequence identity, at least 97% sequence identity, at least 98% sequence identity, at least 99% sequence identity, or at least 99.5% sequence identity to the amino acid sequence of SEQ ID NO: 18, and specifically binds to CLDN18.2.

In an embodiment, the heavy chain consisting of an amino acid sequence with at least 80% sequence identity to the amino acid sequence represented by SEQ ID NO: 17 includes L237A and L238A mutations.

In an embodiment of the present disclosure, the antibody or antigen-binding fragment thereof may be any one selected from a monoclonal antibody, a domain antibody (dAb), a single chain antibody (scAb), a Fab fragment, a Fab' fragment, a F(ab')2 fragment, an scFab fragment, an Fv fragment, a dsFv fragment, a single chain variable fragment (scFv), an scFv-Fc fragment, a single domain heavy chain antibody, a single domain light chain antibody, a variant antibody, a multimeric antibody, a minibody, a diabody, a bispecific antibody, and a multispecific antibody.

The term "linker" as used herein refers to a compound that covalently bonds a cytotoxic compound to a ligand.

The linker described herein may be cleavable, non-cleavable, hydrophilic, or hydrophobic.

The cleavable linker is cleavable under intracellular conditions, by which an active agent is released from an antibody construct-active agent conjugate in the intracellular environment.

The cleavable linker can be cleaved by a cleaving agent present in an intracellular environment (e.g., lysosomes, endosomes, or caveolea). The cleavable linker may be, for example, a peptidyl linker that is cleaved by an intracellular peptidase or protease enzyme, including, but not being limited to, a lysosomal or endosomal protease. Generally, the peptidyl linker has a length of at least two amino acids or a length of at least three amino acids. Cleaving agents may include cathepsin B, cathepsin D, and plasmin, all of which are known to hydrolyze dipeptide drug derivatives to release an active drug in target cells (e.g., see Dubowchik and Walker, 1999, Pharm. Therapeutics 83:67-123). The most common are peptidyl linkers cleavable by enzymes present in antigen-expressing cells. For example, peptidyl linkers cleavable by thiol-dependent protease cathepsin-B, which is highly expressed in cancer tissue, may be used (e.g., a Phe-Leu or Gly-Phe-Leu-Gly linker). Other examples of these linkers are described in, for example, U.S. Patent No. 6,214,345. In addition, the peptidyl linker cleavable by an intracellular protease may be, for example, a Val-Cit linker, a Phe-Lys linker (e.g., see U.S. Patent No. 6,214,345, which describes the synthesis of doxorubicin using a Val-Cit linker), or a Val-Ala linker. The Val-Cit linker or the Val-Ala linker may contain a pentafluorophenyl group, and may contain a succinimide group or a maleimide group. Alternatively, the Val-Cit linker or the Val-Ala linker may contain a 4-aminobenzoic acid (PABA) group and a pentafluorophenyl group, may contain a PABA group and a maleimide group, and may contain a PABA group and a succinimide group.

The terms "intracellularly cleaved" and "intracellular cleavage" as used herein refer to a metabolic process or reaction inside a cell on an antibody construct-active agent conjugate, by which the covalent attachment, e.g., the linker, between an active agent (B) and an antibody construct (Ab) is broken, thus causing a free drug or other metabolites of the conjugate dissociated from the antibody inside the cell.

In addition, a cleavable linker may be easily hydrolyzed in a pH-sensitive manner, i.e., at certain pH values. Generally, the pH-sensitive linker may be hydrolyzed under acidic conditions. For example, acid-labile linkers that can be hydrolyzed in lysosomes (e.g., hydrazone, semicarbazone, thiosemicarbazone, cis-aconic amides, orthoesters, acetals, and ketals) may be used (e.g., see: U.S. Patent NOs. 5,122,368, 5,824,805, and 5,622,929; and Dubowchik and Walker, 1999, Pharm. Therapeutics 83:67-123; and Neville et al., 1989, Biol. Chem. 264:14653-14661). These linkers are relatively stable under neutral pH conditions, such as in blood, but are unstable at pH 5.5, which is the approximate pH of lysosomes, or less than pH 5.0. Examples of hydrolysable linkers may include thioether linkers (e.g., thioethers attached to a therapeutic agent via an acylhydrazone bond) (e.g., see U.S. Patent No. 5,622,929).

Also, the linker is cleavable under reducing conditions (e.g., a disulfide linker). For example, various disulfide linkers, including N-succinimidyl-5-acetylthioacetate (SATA), N-succinimidyl-3-(2-pyridyldithio)prop ionate (SPDP), N-succinimidyl-3-(2-pyridyldithio)butyrate (SPDB), and N-succinimidyl-oxycarbonyl-alpha-methyl-alpha-(2-pyridyl-thio)toluene)- (SMPT), and those that can be formed using SPDB and SMPT, are known (e.g., see: Thorpe et al., 1987, Cancer Res. 47:5924-5931; and U.S. Patent No. 4,880,935).

In addition, the linker may be a malonate linker (Johnson et al., 1995, Anticancer Res. 15:1387-93), a maleimidobenzoyl linker (Lau et al., 1995, Bioorg-Med-Chem. 3(10):1299-1304), a 3'-N-amide analogue (Lau et al., 1995, Bioorg-Med-Chem. 3(10):1305-12), a β-glucuronide linker (Jeffery et al., 2006, Bioconjug Chem. 17(3):832-40), or a β-galactosidase linker (Kolodych et al., 2017, Eur J Med Chem. Dec 15;142:376-382).

The non-cleavable linker may be a maleimidocaproyl linker. The maleimidocaproyl linker may include N-maleimidomethylcyclohexane-1-carboxylate. The maleimidocaproyl linker may contain a succinimide group. The maleimidocaproyl linker may contain a pentafluorophenyl group. The linker may be a combination of a maleimide group and one or more polyethylene glycol molecules. The linker may be a combination of a maleimidocaproyl group and one or more polyethylene glycol molecules. The linker may be a maleimide-PEG4 linker. The linker may be a combination of a maleimidocaproyl linker containing a succinimide group and one or more polyethylene glycol molecules. The linker may be a combination of a pentafluorophenyl group and a maleimidocaproyl linker containing one or more polyethylene glycol molecules. The linker may contain a maleimide linked to a polyethylene glycol molecule, wherein the polyethylene glycol allows for more linker flexibility or allows longer linkers to be used. The linker may be a (maleimidocaproyl)-(valine-citrulline)-(para-aminobenzyloxycarbonyl) linker.

In an embodiment of the present disclosure, the linker may be a cleavable linker.

In an embodiment of the present disclosure, the linker may be a protease cleavable linker, an acid-cleavable linker, a disulfide linker, a self-immolative linker or a self-stabilizing linker, a malonate linker, a maleimidobenzoyl linker, a 3'-N-amide analogue, a β-glucuronide linker, or a β-galactoside linker.

In an embodiment of the present disclosure, the protease cleavable linker may include a thiol-reactive spacer or a dipeptide, and more specifically, the protease cleavable linker may include a thiol-reactive maleimidocaproyl spacer, a valine-citrulline dipeptide, or a p-amino-benzyloxycarbonyl spacer.

In an embodiment of the present disclosure, the acid-cleavable linker may be a hydrazine linker or a quaternary ammonium linker.

In an embodiment of the present disclosure, the linker may have a structure of General Formula II: 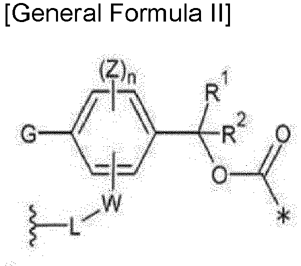 wherein,
a wave symbol denotes a site that is linked to an antibody or antigen-binding fragment thereof that specifically binds to CLDN18.2, and * denotes a site that is linked to an active agent;
G is a glucuronic acid moiety or 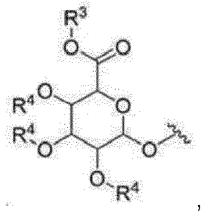 R³ is hydrogen or a carboxyl-protecting group, and each R⁴ is independently hydrogen or a hydroxyl-protecting group;
R¹ and R² are each independently hydrogen, C₁₋₈ alkyl, or C₃₋₈ cycloalkyl;
W is -C(O)-, -C(O)NR'-, -C(O)O-, -SO2NR'-, -P(O)R"NR', -SONR'-, or -PO₂NR'-, wherein C, S, or P is directly bonded to a phenyl ring, and R' and R" are each independently hydrogen, C₁₋₈ alkyl, C₃₋₈ cycloalkyl, C₁₋₈ alkoxy, C₁₋₆ alkylthio, mono- or di-C₁₋₈ alkylamino, C₃₋₂₀ heteroaryl, or C₆₋₂₀ aryl;
Z is independently hydrogen, C₁₋₈ alkyl, halogen, cyano, or nitro;
n is an integer from 0 to 3; and
L is any one selected from A) or B) below:
   A) C₁₋₅₀ alkylene or 1- to 50-membered heteroalkylene, satisfying at least one of the following:
      (i) L includes one or more unsaturated bonds;
      (ii) two atoms in L are substituted with a divalent substituent, which completes a heteroarylene;
      (iii) L is a 1- to 50-membered heteroalkylene;
      (iv) the alkylene is substituted with at least one C₁₋₂₀ alkyl; and
   B) at least one isoprenyl derivative unit of General Formula III below which can be recognized by an isoprenoid transferase:

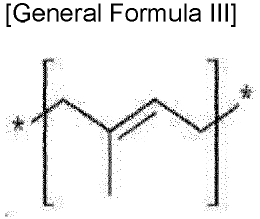

In an embodiment of the present disclosure, the conjugate may have a structure of General Formula IIa. 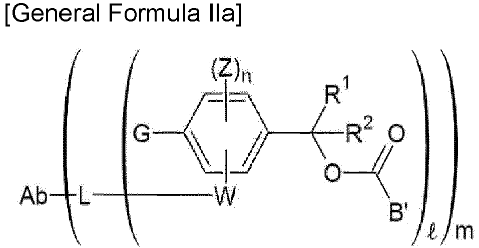 wherein,
Ab is an antibody or antigen-binding fragment thereof that includes a heavy chain variable region and a light chain variable region and specifically binds to CLDN18.2;
each of B' is independently an active agent wherein the active agents are identical to or different from each other;
each G is independently a glucuronic acid moiety or 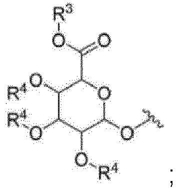
R³ is hydrogen or a carboxyl-protecting group, and each R⁴ is independently hydrogen or a hydroxyl-protecting group;
R¹ and R² are each independently hydrogen, C₁₋₈ alkyl, or C₃₋₈ cycloalkyl;
each W is independently -C(O)-, -C(O)NR'-, -C(O)O-, -SO₂NR'-, -P(O)R"NR', - SONR'-, or -PO₂NR'-, wherein C, S, or P is directly bonded to a phenyl ring, NR' is bonded to L, and R' and R" are each independently hydrogen, C₁₋₈ alkyl, C₃₋₈ cycloalkyl, C₁₋₈ alkoxy, C₁₋₈ alkylthio, mono- or di- C₁₋₈ alkylamino, C₃₋₂₀ heteroaryl, or C₆₋₂₀ aryl;
each Z is independently hydrogen, C₁₋₈ alkyl, halogen, cyano, or nitro;
n is an integer from 0 to 3 wherein, when n is an integer of 2 or more, at least two of Z may be identical to or different from each other;
each L is independently any one selected from A) or B) below:
   A) C₁₋₅₀ alkylene or 1-50 atom heteroalkylene, satisfying at least one of the following:
      (i) L includes one or more unsaturated bonds;
      (ii) two atoms in L are substituted with a bivalent substituent, which completes a heteroarylene;
      (iii) L is a 1-50 atom heteroalkylene; or
      (iv) the alkylene is further substituted with at least one C₁₋₂₀ alkyl; or
   B) at least one isoprenyl derivative unit of General Formula III which can be recognized by an isoprenoid transferase: 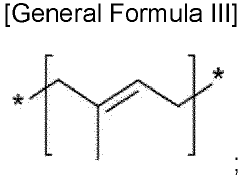 m is an integer from 1 to 20.

The present disclosure also relates to a conjugate represented by General Formula IIa below or a pharmaceutically acceptable salt or solvate thereof: 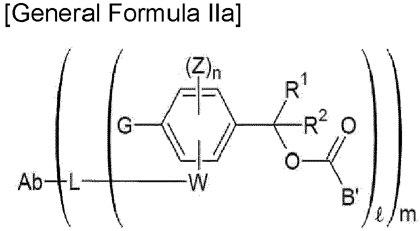 wherein,
Ab is an antibody or antigen-binding fragment thereof that specifically binds to claudin 18 isoform 2 (CLDN18.2);
each G is independently a glucuronic acid moiety or 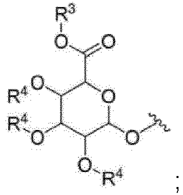
R³ is hydrogen or a carboxyl-protecting group, and each R⁴ is independently hydrogen or a hydroxyl-protecting group;
R¹ and R² are each independently hydrogen, C₁₋₈ alkyl, or C₃₋₈ cycloalkyl;
each W is independently -C(O)-, -C(O)NR'-, -C(O)O-, -SO₂NR'-, -P(O)R"NR', - SONR'-, or -PO₂NR'-, wherein C, S, or P is directly bonded to a phenyl ring, NR' is bonded to L, and R' and R" are each independently hydrogen, C₁₋₈ alkyl, C₃₋₈ cycloalkyl, C₁₋₈ alkoxy, C₁₋₈ alkylthio, mono- or di- C₁₋₈ alkylamino, C₃₋₂₀ heteroaryl, or C₆₋₂₀ aryl;
each Z is independently hydrogen, C₁₋₈ alkyl, halogen, cyano, or nitro;
n is an integer from 0 to 3; and
each L is independently any one selected from A) or B) below:
   A) C₁₋₅₀ alkylene or 1-50 atom heteroalkylene, satisfying at least one of the following:
      (i) L includes one or more unsaturated bonds;
      (ii) two atoms in L are substituted with a bivalent substituent, which completes a heteroarylene;
      (iii) L is a 1-50atom heteroalkylene; or
      (iv) the alkylene is substituted with at least one C₁₋₂₀ alkyl; or
   B) at least one isoprenyl derivative unit of General Formula III which can be recognized by an isoprenoid transferase: 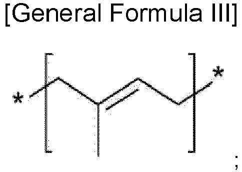
      B' is an active agent;
      Ab is bonded to a site indicated as a wave symbol; and
      I and m are each independently an integer selected from 1 to 20,
      wherein, when I is 2 or more, the active agents may be identical to or different from each other.

In an embodiment of the present disclosure, G is a glucuronic acid moiety or a compound having the structure of Formula (IIIa): 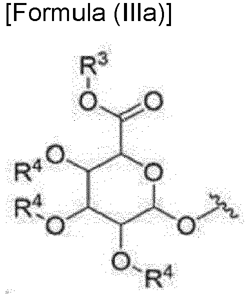 wherein, in Formula (IIIa),
R³ is hydrogen or a carboxyl-protecting group; and
each R⁴ is independently hydrogen or a hydroxyl-protecting group.

In an embodiment of the present disclosure, each of G is independently 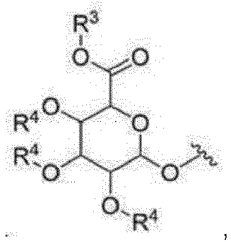
R³ is hydrogen or a carboxyl-protecting group, and
each R⁴ is independently hydrogen or a hydroxyl-protecting group.

In an embodiment of the present disclosure, R³ is hydrogen, and each R⁴ is hydrogen.

In an embodiment of the present disclosure, R¹ and R² are each hydrogen.

In addition, each of Z is independently hydrogen, C₁₋₈ alkyl, halogen, cyano, or nitro.

In addition, W is -C(O)-, -C(O)NR'-, or -C(O)O-, and more specifically, W is - C(O)NR'-, wherein C(O) is linked to a phenyl ring, and NR' is bonded to L. In this case, R' is hydrogen, C₁₋₈ alkyl, C₃₋₈ cycloalkyl, C₁₋₈ alkoxy, C₁₋₈ alkylthio, mono- or di-C₁₋₈ alkylamino, C₃₋₂₀ heteroaryl, or C₆₋₂₀ aryl.

In addition, n is 0, 1, 2, or 3, more particularly 0, 1, or 2, and even more particularly 0.

More specifically, G is a compound having the structure of Formula (IIIa): 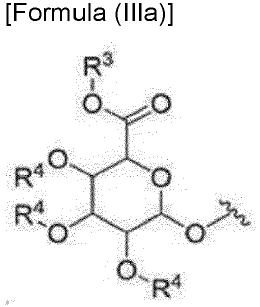 wherein, in Formula (IIIa),
R³ is hydrogen or a carboxyl-protecting group,
each R⁴ is independently hydrogen or a hydroxyl-protecting group,
W is -C(O)NR'-, wherein C(O) is linked to a phenyl ring, NR' is bonded to L, each of Z is C₁₋₈ alkyl, halogen, cyano, or nitro, and n is 0, m is 1, and R¹ and R² are each hydrogen.

In an embodiment of the present disclosure, at least one L is an alkylene having 1 to 100 carbon atoms, wherein the carbon atom(s) of the alkylene may be substituted with one or more heteroatoms selected from the group consisting of N, O, and S, and the alkylene may be further substituted with one or more alkyls having 1 to 20 carbon atoms.

Specifically, at least one L is C₁₋₅₀ alkylene or 1- to 50-membered heteroalkylene, and may satisfy at least one of the following:
(i) L contains one or more unsaturated bonds;
(ii) two atoms in L are substituted with a divalent substituent, which completes a heteroarylene;
(iii) L is a 1- to 50-membered heteroalkylene; and
(iv) the alkylene is substituted with one or more C₁₋₂₀ alkyls.

In addition, the at least one L is a nitrogen-containing 1- to 50-membered heteroalkylene, the linker includes two or more atoms of a hydrophilic amino acid, and the nitrogen may form a peptide bond with a carbonyl of the hydrophilic amino acid.

In an embodiment of the present disclosure, L is C₂₋₅₀ alkylene, C₂₋₅₀ heteroalkylene, a hydrophilic amino acid, -C(O)-, -C(O)NR"-, -C(O)O-, -(CH₂)ₛ-NHC(O)-(CH₂)t-, -(CH₂)ᵤC(O)NH-(CH₂)ᵥ-, -(CH₂)ₛ-NHC(O)-(CH₂)ₜ-C(O)-, -(CH₂)ᵤC(O)NH-(CH₂)ᵥ-C(O)-, -S(O)₂NR"-, -P(O)R‴NR"-, -S(O)NR"-, or -PO₂NR"-,
wherein R" and R‴ are each independently hydrogen, C₁₋₈ alkyl, C₃₋₈ cycloalkyl, C₁₋₈ alkoxy, C₁₋₈ alkylthio, mono- or di-C₁₋₈ alkylamino, C₃₋₂₀ heteroaryl, or C₅₋₂₀ aryl, and s, t, u, and v are each independently an integer from 0 to 10.

In an embodiment of the present disclosure, L is a 1-5 atom heteroalkylene comprising nitrogen, the L includes two or more atoms of a hydrophilic amino acid, and the nitrogen forms a peptide bond with a carbonyl of the hydrophilic amino acid.

In an embodiment of the present disclosure, L is covalently bonded to the antibody via a thioether bond, and the thioether bond includes a sulfur atom of a cysteine of the antibody.

In an embodiment of the present disclosure, at least one L is a hydrophilic amino acid.

In an embodiment of the present disclosure, the hydrophilic amino acid may be arginine, aspartate, asparagine, glutamate, glutamine, histidine, lysine, ornithine, proline, serine, or threonine.

In an embodiment of the preset disclosure, the hydrophilic amino acid may be an amino acid including a side chain having residues that carry a charge at neutral pH in an aqueous solution.

In an embodiment of the present disclosure, the hydrophilic amino acid is aspartate or glutamate.

In an embodiment of the present disclosure, the hydrophilic amino acid is ornithine or lysine.

In an embodiment of the present disclosure, the hydrophilic amino acid is arginine.

In an embodiment of the present disclosure, at least one L is -C(O)-, -C(O)NR"-, -C(O)O-, -S(O)₂NR"-, -P(O)R‴NR"-, -S(O)NR"-, or -PO₂NR"-, and R" and R‴ are each independently hydrogen, C₁₋₈ alkyl, C₃₋₈ cycloalkyl, C₁₋₈ alkoxy, C₁₋₈ alkylthio, mono- or di-C₁₋₈ alkylamino, C₃₋₂₀ heteroaryl, or C₆₋₂₀ aryl.

In an embodiment of the present disclosure, at least one L is -C(O)NR"-, -(CH₂)ₛ-NHC(O)-(CH₂)ₜ-, -(CH₂)ᵤC(O)NH-( CH₂)ᵥ-, -(CH₂)ₛ-NHC(O)-(CH₂)ₜ-C(O)-, or - (CH₂)ᵤC(O)NH-(CH₂)ᵥ-C(O)-, wherein R" is hydrogen, C₁₋₅ alkyl, C₃₋₈ cycloalkyl, C₁₋₅ alkoxy, C₃₋₂₀ heteroaryl, or C₆₋₂₀ aryl, and s, t, u, and v are each independently an integer from 0 to 10.

In an embodiment of the present disclosure, at least one L is -C(O)NR"-, and R" is hydrogen.

In an embodiment of the present disclosure, at least one L is -(CH₂)ₛ-NHCO-, and s is an integer from 1 to 5.

In an embodiment of the present disclosure, at least one L is -(CH₂)ᵤ-C(O)NH-(CH₂)ᵥ-C(O)-, u is an integer from 1 to 5, and v is an integer from 1 to 5.

In an embodiment of the present disclosure, L includes 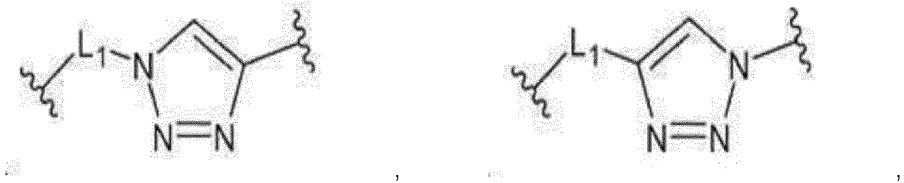 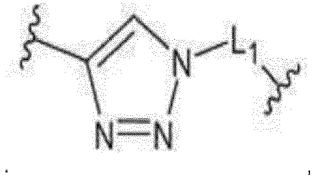 or 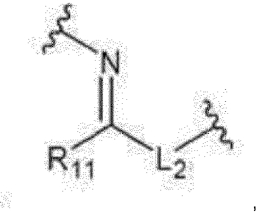 wherein L₁ is a bond or an alkylene having 1 to 30 carbon atoms, R₁₁ is hydrogen or an alkyl having 1 to 10 carbon atoms, and L₂ is an alkylene having 1 to 30 carbon atoms.

In an embodiment of the present disclosure, L further includes a second unit represented by General Formula VIII or General Formula IX.

In an embodiment of the present disclosure, the at least one second unit is represented by General Formula VIII or General Formula IX:

[General Formula VIII] -(CH₂)ᵣ(V(CH₂)ₚ)_{q}-

[General Formula IX] -(CH₂CH₂X)_{w}-

V is a single bond, -O-, -S-, -NR²¹-, -C(O)NR²²-, -NR²³C(O)-, -NR²⁴SO₂-, or - SO₂NR²⁵-;
X is -O-, C₁₋₈ alkylene, or - NR²¹-;
R²¹ to R²⁵ are each independently hydrogen, C₁₋₆ alkyl, C₁₋₆ alkyl C₆₋₂₀ aryl, or C₁₋₆ alkyl C₃₋₂₀ heteroaryl;
r is an integer from 0 to 10;
p is an integer from 0 to 10;
q is an integer from 1 to 20; and
w is an integer from 1 to 20.

In an embodiment of the present disclosure, q may be from 4 to 20, more particularly from 5 to 20. In addition, q may be from 1 to 10 or from 2 to 12, and more particularly, may be 2, 4, 5, or 11. Also, r may be 1 or 2. Also, p may be 1 or 2. Also, V may be -O-.

More specifically, r may be 2, p may be 2, q may be 2, 4, 5, or 11, and V may be -O-.

Also, in an embodiment of the present disclosure, X may be -O-. Also, w may be an integer from 4 to 20.

More specifically, X is -O-, and w may be from 1 to 10 or from 4 to 20.

In an embodiment of the present disclosure, at least one second unit is at least one polyethylene glycol unit, and has the structure of . 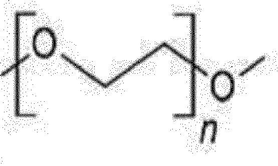 or 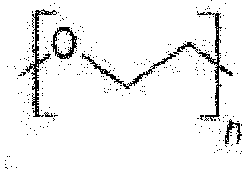 , wherein n ranges from 1 to 12.

In an embodiment of the present disclosure, the at least one second unit is 1 to 12 -OCH₂CH₂- units, 3 to 12 -OCH₂CH₂- units, 5 to 12 -OCH₂CH₂- units, 6 to 12 - OCH₂CH₂- units, or 3 -OCH₂CH₂- units.

In an embodiment of the present disclosure, the at least one second unit is - (CH₂CH₂X)_{w}-,
wherein X is a single bond, -O-, C₁₋₃ alkylene, or -NR₂₁-; and
R₂₁ is hydrogen, C₁₋₆ alkyl, C₁₋₆ alkyl-C₆₋₂₀ aryl, or C₁₋₆ alkyl-C₃₋₂₀ heteroaryl, and w is an integer from 1 to 20, specifically 1, 3, 6, or 12.

In an embodiment of the present disclosure, X is -O-, and w is an integer from 6 to 20.

In a specific embodiment of the present disclosure, L includes an oxime, and at least one polyethylene glycol unit covalently bonds the oxime to the active agent.

In an embodiment of the present disclosure, the linker includes a third unit formed by 1,3-dipolar cycloaddition reactions, hetero-Diels-Alder reactions, nucleophilic substitution reactions, non-aldol type carbonyl reactions, addition to a carbon-carbon multiple bond, oxidation reactions, or click reactions.

In an embodiment of the present disclosure, the third unit is formed by a reaction between an alkyne and an azide, or between an aldehyde or ketone group and a hydrazine or alkoxyamine.

In an embodiment of the present disclosure, L further includes a third unit represented by General Formula IVa, IVb, IVc, IVd or IVe below. 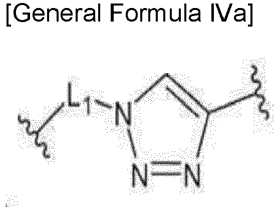 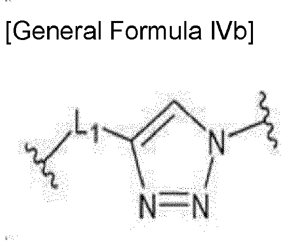 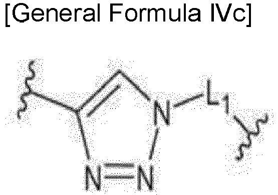 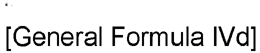 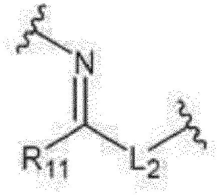 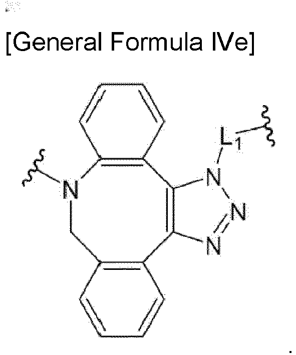
Wherein, L₁ is a single bond or an alkylene having 1 to 30 carbon atoms, and
R₁₁ is hydrogen or C₁₋₁₀ alkyl.

In an embodiment of the present disclosure, the third unit includes 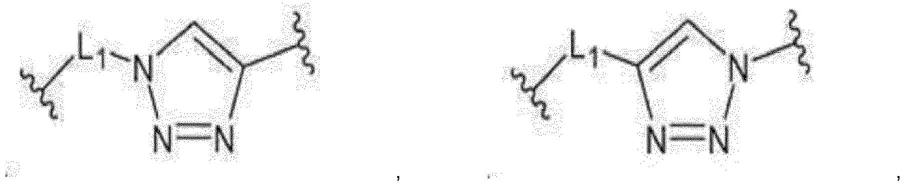 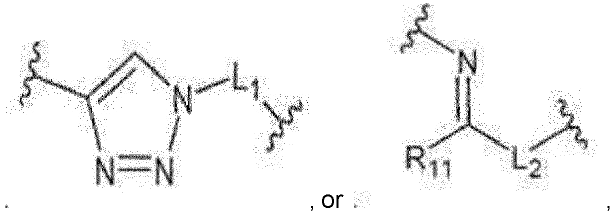 wherein:
L₁ is a single bond or an alkylene having 1 to 30 carbon atoms, preferably an alkylene having 10, 11, 12, 13, 14, 15 or 16 carbon atoms;
R₁₁ is hydrogen or an alkyl having 1 to 10 carbon atoms, specifically methyl; and
L₂ is an alkylene having 1 to 30 carbon atoms.

In an embodiment of the present disclosure, L₁ and L₂ are each independently a single bond or C₁₋₃₀ alkylene, and R₁₁ is hydrogen or C₁₋₁₀ alkyl.

In an embodiment of the present disclosure, L₁ is a single bond, an alkylene having 11 carbon atoms, or an alkylene having 12 carbon atoms.

Also, in an embodiment of the present disclosure, the third unit includes 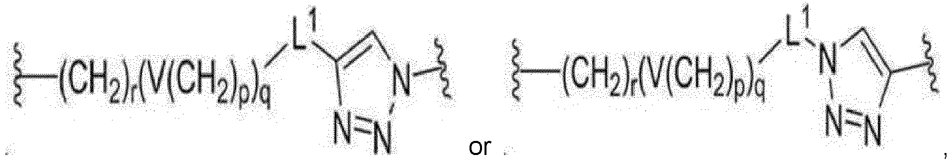
wherein: V is a single bond, -O-, -S-, -NR²¹-, -C(O)NR²²-, -NR²³C(O)-, -NR²⁴SO₂-, or -SO₂NR²⁵-;
R²¹ to R²⁵ are each independently hydrogen, C₁₋₆ alkyl, C₁₋₆ alkyl C₆₋₂₀ aryl, or C₁₋₆ alkyl C₃₋₂₀ heteroaryl;
r is an integer from 1 to 10;
p is an integer from 0 to 10;
q is an integer from 1 to 20; and
L¹ is a single bond.

In an embodiment of the present disclosure, r may be 2 or 3. Also, p may be 1 or 2. Also, q may be 1 to 6.

More specifically, in the third unit, r may be 2 or 3, p may be 1 or 2, and q may be 1 to 6.

In addition, in an embodiment of the present disclosure, the linker including the third unit may be 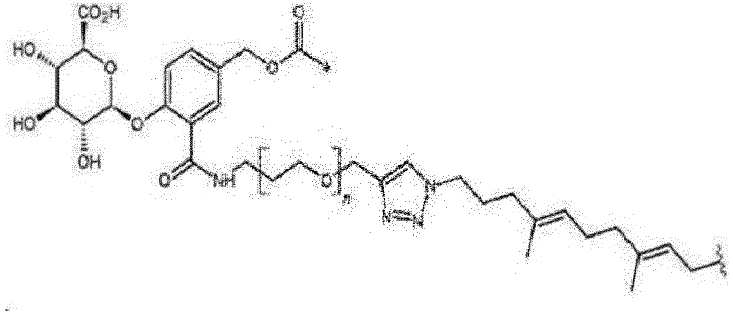 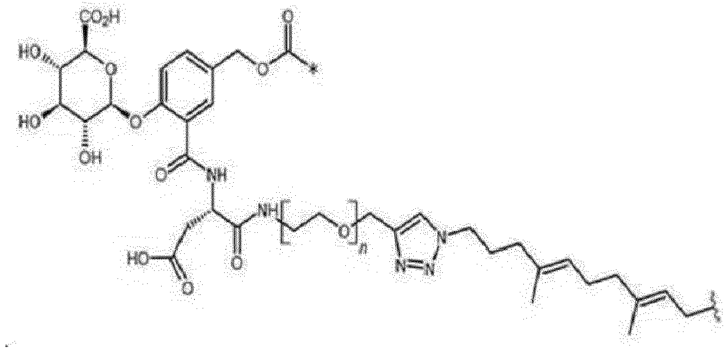 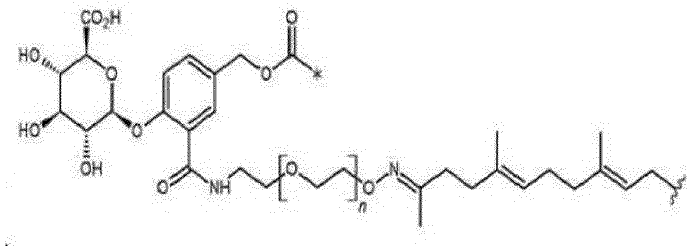 or 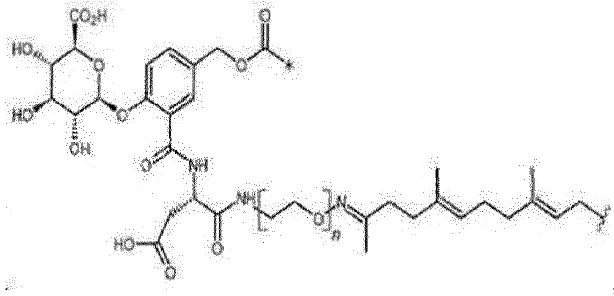 , wherein a wave symbol denotes a binding site for the antibody construct, * denotes a binding site for the active agent, and n is an integer from 0 to 20.

In an embodiment of the present disclosure, the third unit includes 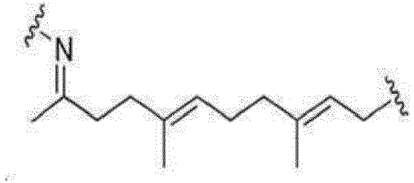 or

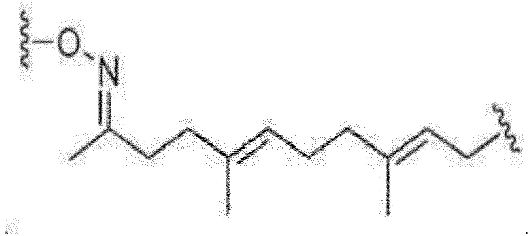

In addition, in an embodiment of the present disclosure, the linker includes 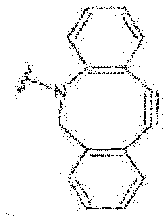 in a precursor state. In addition, in the above structure, the third unit may be formed by a reaction between an alkyne and an azide, particularly a click reaction.

In an embodiment of the present disclosure, the linker includes 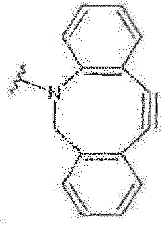 in a precursor state, and may form a third unit through the structure, and the third unit includes

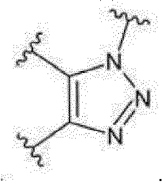

In an embodiment of the present disclosure, L is 3 to 50 heteroalkylene containing an oxime,
the oxygen atom of the oxime is on the side of L linked to W, and the carbon atom of the oxime is on the side of L linked to Ab, or
the carbon atom of the oxime is on the side of L linked to W, and the oxygen atom of the oxime is on the side of L linked to Ab.

In an embodiment of the present disclosure, L includes an oxime, and one or more isoprenyl units covalently bond the oxime to Ab.

In an embodiment of the present disclosure, the linker may further contain at least one isoprenyl unit having the structure of . 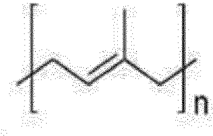 , wherein n is at least 2.

In an embodiment of the present disclosure, at least one isoprenyl unit is a substrate of an isoprenoid transferase or a product of an isoprenoid transferase.

In an embodiment of the present disclosure, the isoprenyl unit of the linker is covalently bonded to the antibody via a thioether bond, and the thioether bond includes a sulfur atom of a cysteine of the antibody construct.

In addition, the isoprenyl unit may covalently bond the oxime included in the linker to the antibody construct.

In an embodiment of the present disclosure, the antibody construct includes an amino acid motif recognized by an isoprenoid transferase, and the thioether bond includes a sulfur atom of a cysteine of the amino acid motif.

In an embodiment of the present disclosure, the antibody construct includes an amino acid motif recognized by an isoprenoid transferase, and the thioether bond includes a sulfur atom of a cysteine of the amino acid motif.

In an embodiment of the present disclosure, the amino acid motif is a sequence selected from the group consisting of CXX, CXC, XCXC, XXCC, and CYYX, wherein: C is cysteine; each of Y is independently an aliphatic amino acid; each of X is independently glutamine, glutamate, serine, cysteine, methionine, alanine, or leucine; and the thioether bond includes a sulfur atom of a cysteine of the amino acid motif.

In an embodiment of the present disclosure, the amino acid motif has a CYYX sequence, and each of Y is independently alanine, isoleucine, leucine, methionine, or valine.

In an embodiment of the present disclosure, the amino acid motif has a CVIM or CVLL sequence.

In an embodiment of the present disclosure, at least one of the 1 to 20 amino acids preceding the amino acid motif may each independently be selected from glycine, arginine, aspartic acid, and serine.

In an embodiment of the present disclosure, at least one of the 1 to 20 amino acids preceding the amino acid motif is glycine.

In an embodiment of the present disclosure, at least one of the 1 to 10 amino acids preceding the amino acid motif may each independently be selected from glycine, arginine, aspartic acid, and serine. For example, in an embodiment of the present disclosure, at least one of the 7 amino acids preceding the amino acid motif is glycine. Alternatively, at least three of the 7 amino acids preceding the amino acid motif are each independently selected from glycine, arginine, aspartic acid, and serine. Alternatively, 1 to 10 amino acids preceding the amino acid motif are glycine, and particularly, at least 1, 2, 3, 4, 5, 6, 7, 8, 9 or 10 amino acids preceding the amino acid motif are glycine.

In an embodiment of the present disclosure, the antibody may include the amino acid sequence of GGGGGGGCVIM.

In an embodiment of the present disclosure, L may include the amino acid sequence GGGGGGGCVIM at the C-terminus.

In another embodiment of the present disclosure, Linker may contain:
(a) one or more L; (b) one or more second units; (c) one or more third units; and (d) one or more fourth units.

In this case, the second unit links L to the third unit, L to the fourth unit, or the third unit to the fourth unit,
the one or more fourth units can release one or more drugs or toxins, and
L links the second unit to the fourth unit, the second unit to the third unit, or the second unit to another second unit.

In an embodiment of the present disclosure, the fourth unit has the structure of Formula (IIb) below: 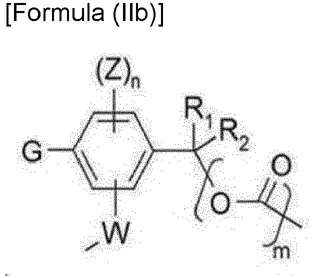 wherein, in Formula (IIb),
G is a sugar, a sugar acid, or a sugar derivative,
W is -C(O)-, -C(O)NR'-, -C(O)O-, -S(O)₂NR'-, -P(O)R"NR'-, -S(O)NR'-, or - PO₂NR'-,
wherein, when C(O), S, or P is directly linked to the phenyl ring, R' and R" are each independently hydrogen, (C₁₋₈)alkyl, (C₃₋₈)cycloalkyl, (C₁₋₈)alkoxy, (C₁₋₈)alkylthio, mono- or di-( C₁₋₈)alkylamino, (C₃₋₂₀)heteroaryl, or (C₆₋₂₀)aryl, and W is linked to L or the second unit,
each of Z is independently hydrogen, (C₁₋₈)alkyl, halogen, cyano, or nitro,
n is an integer from 1 to 3,
m is 0 or 1, and
R¹ and R² are each independently hydrogen, (C₁₋₈)alkyl, or (C₃₋₈)cycloalkyl, or R¹ and R² form a (C₃₋₈)cycloalkyl ring along with a carbon atom to which R¹ and R² are attached.

In an embodiment of the present disclosure, the sugar or the sugar acid is a monosaccharide.

In an embodiment of the present disclosure, G is a compound having the structure of Formula (IIIa) below: 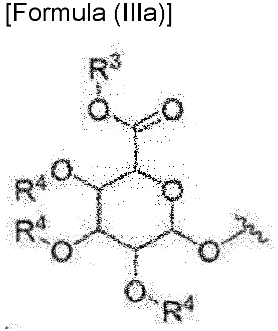 wherein, in Formula (IIIa),
R³ is hydrogen or a carboxyl-protecting group, and
each R⁴ is independently hydrogen or a hydroxyl-protecting group.

In an embodiment of the present disclosure, R³ is hydrogen and each R⁴ is hydrogen.

In an embodiment of the present disclosure, W is -C(O)NR'-, wherein C(O) is linked to the phenyl ring, and NR' is linked to L or the second unit.

In an embodiment of the present disclosure, Z is hydrogen.

In an embodiment of the present disclosure, R¹ and R² are each hydrogen.

In an embodiment of the present disclosure, the second unit is represented by - (CH₂)ᵣ(V(CH₂)ₚ)_{q}-, -((CH₂)ₚV)_{q}-, -(CH₂)ᵣ(V(CH₂)ₚ)_{q}Y-, -((CH₂)ₚV)_{q}(CH₂)ᵣ-, -Y((CH₂)ₚV)_{q}-, or -(CH₂)ᵣ(V(CH₂)ₚ)_{q}YCH₂-,
wherein:
r is an integer from 0 to 10;
p is an integer from 1 to 10; and
q is an integer from 1 to 20;
V and Y are each independently a single bond, -O-, -S-, -NR²¹-, -C(O)NR₂₂-, - NR₂₃C(O)-, -NR²⁴SO₂-, or -SO₂NR₂₅-; and
R₂₁ to R₂₅ are each independently hydrogen, (C₁₋₆)alkyl, (C₁₋₆)alkyl(C₆₋₂₀)aryl, or (C₁₋₆)alkyl(C₃₋₂₀)heteroaryl.

In an embodiment of the present disclosure, r is 2.

In an embodiment of the present disclosure, p is 2.

In an embodiment of the present disclosure, q is an integer from 6 to 20.

In an embodiment of the present disclosure, q is 2, 5, or 11.

In an embodiment of the present disclosure, V and Y are each independently -O-.

In an embodiment of the present disclosure, L or the third unit includes 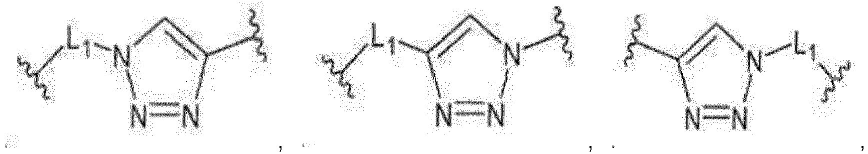 or 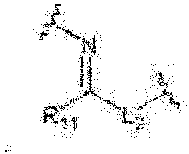 , wherein L₁ is a bond or an alkylene having 1 to 30 carbon atoms, R₁₁ is hydrogen or an alkyl having 1 to 10 carbon atoms, particularly methyl, and L₂ is an alkylene having 1 to 30 carbon atoms.

In an embodiment of the present disclosure, L₁ is an alkylene having 12 carbon atoms.

In an embodiment of the present disclosure, R₁₁ is methyl.

In an embodiment of the present disclosure, L₂ is an alkylene having 11 carbon atoms.

In an embodiment of the present disclosure, the binding unit is covalently bonded to the antibody via a thioether bond, and the thioether bond includes a sulfur atom of a cysteine of the antibody.

In an embodiment of the present disclosure, the antibody includes an amino acid motif recognized by an isoprenoid transferase, and the thioether bond includes a sulfur atom of a cysteine of the amino acid motif.

In an embodiment of the present disclosure, the amino acid motif is a sequence selected from the group consisting of CXX, CXC, XCXC, XXCC, and CYYX, wherein: C is cysteine; each of Y is independently an aliphatic amino acid; each of X is independently glutamine, glutamate, serine, cysteine, methionine, alanine, or leucine; and the thioether bond includes a sulfur atom of a cysteine of the amino acid motif.

In an embodiment of the present disclosure, the amino acid motif has a CYYX sequence, and each of Y is independently alanine, isoleucine, leucine, methionine, or valine.

In an embodiment of the present disclosure, the amino acid motif is a CVIM or CVLL sequence.

In an embodiment of the present disclosure, at least one of the 1 to 20 amino acids preceding the amino acid motif is glycine.

In an embodiment of the present disclosure, at least one of the 1 to 20 amino acids preceding the amino acid motif may each independently be selected from glycine, arginine, aspartic acid, and serine.

In an embodiment of the present disclosure, 1 to 20 amino acids preceding the amino acid motif are glycine, and particularly, at least 1, 2, 3, 4, 5, 6, 7, 8, 9, 10, 11, 12, 13, 14, 15, 16, 17, 18, 19 or 20 amino acids preceding the amino acid motif are glycine.

In an embodiment of the present disclosure, the antibody may include the amino acid sequence of GGGGGGGCVIM.

In an embodiment of the present disclosure, the isoprenoid transferase is farnesyl protein transferase (FTase) or geranylgeranyl transferase (GGTase).

In an embodiment of the present disclosure, L includes one or more branched linkers covalently bonded to Ab, wherein:
i) each branched linker includes a fifth unit covalently bonded to Ab by a primary linker (PL);
ii) each branched linker includes a first branch (B1) in which a first active agent is covalently bonded to the fifth unit by a secondary linker (SL) and a cleavage group (CG); and
iii) each branched linker further includes: a) a second branch (B2) in which a second active agent is covalently bonded to the fifth unit by the secondary linker (SL) and the cleavage group (CG); or b) a second branch (B2) in which a polyethylene glycol moiety is covalently bonded to the fifth unit, and
each cleavage group may be hydrolyzed to release the active agent from an antibody construct-active agent conjugate.

In an embodiment of the present disclosure, i) the branched linker includes a fifth unit (BR) covalently bonded to a reactive moiety by the primary linker (PL),
ii) the fifth unit is covalently bonded to the first branch (B1), and includes a first active agent covalently bonded to the secondary linker (SL) and the cleavage group (CG), and
iii) the fifth unit is covalently bonded to the second branch (B2) and includes a) a second active agent covalently bonded to the secondary linker (SL) and the cleavage group (CG) or b) a polyethylene glycol moiety, wherein each cleavage group can be hydrolyzed to release the active agent.

In an embodiment of the present disclosure, the cleavage group is the same as Formula II above.

In the present disclosure, the secondary linker (e.g., linking the active agent to the fifth unit) may include a third unit formed by a 1,3-dipolar cycloaddition reaction, a hetero-Diels-Alder reaction, a nucleophilic substitution reaction, a non-aldol type carbonyl reaction, addition to a carbon-carbon multiple bond, an oxidation reaction, or a click reaction. In this regard, as described above, the third unit may be formed by the reaction between an alkyne and an azide, or a non-aldol type carbonyl reaction, such as the reaction between an aldehyde or a ketone group and hydrazine or hydroxylamine, which allows the mild coupling between the active agent and/or the cleavage group and L.

In an embodiment of the present disclosure, the fifth unit is . 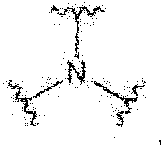 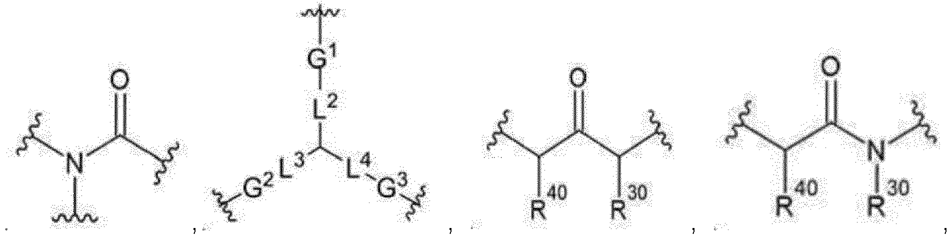 or 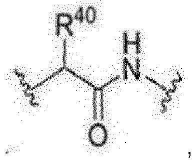
wherein L², L³, and L⁴ are each independently a bond or - CₙH₂ₙ-, wherein n is an integer from 1 to 30,
G¹, G², and G³ are independently a bond, 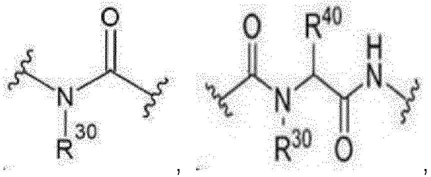 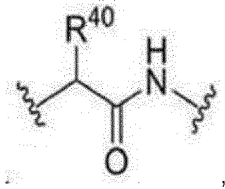 or 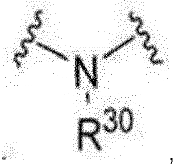
R³⁰ is hydrogen or C₁₋₃₀ alkyl,
R⁴⁰ is hydrogen, C₁₋₁₀ alkyl, or -L⁵-COOR⁵⁰, wherein L⁵ is a bond or -CₙH₂ₙ-,
wherein n is an integer from 1 to 10, and R⁵⁰ is hydrogen or C₁₋₃₀ alkyl.

In an embodiment of the present disclosure, the fifth unit is

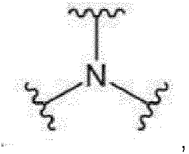

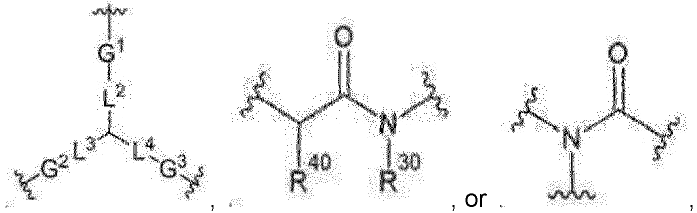

L², L³, L⁴, G¹, G², G³, R³⁰, R⁴⁰, and L⁵ may be the same as described above.

In an embodiment of the present disclosure, the cleavage group is cleavable in a target cell.

In an embodiment of the present disclosure, the cleavage group is capable of releasing one or more active agents.

Also, in an embodiment of the present disclosure, the conjugate includes Ab, and includes 1, 2, 3, 4 or more branched linkers covalently bonded to Ab,
wherein each branched linker is bonded to one or more active agents,
wherein the active agents are identical to or different from each other.

Also, in an embodiment of the present disclosure, each active agent is bonded to the branched linker via a cleavable bond.

In an embodiment of the present disclosure, L is a linker including at least one fifth unit and the primary linker (PL).

In an embodiment of the present disclosure, the primary linker of the antibody-drug conjugate includes an alkylene having 1 to 100 carbon atoms, preferably 1 to 50 carbon atoms, or
the alkylene includes at least one unsaturated bond,
the alkylene includes at least one heteroarylene,
the carbon atom of the alkylene is substituted with one or more heteroatoms selected from nitrogen (N), oxygen (O), and sulfur (S), or
the alkylene is further substituted with one or more alkyls having 1 to 20 carbon atom(s).

Also, in an embodiment of the present disclosure, each of the branched linkers includes a fifth unit, each active agent is bonded to the fifth unit via a secondary linker, and the fifth unit is bonded to the antibody by a primary linker.

In an embodiment of the present disclosure, the fifth unit is an amide, and the primary linker includes a carbonyl of the amide.

In an embodiment of the present disclosure, the fifth unit is a lysine unit.

In a specific embodiment of the present disclosure, at least one carbon atom of the alkylene is substituted with nitrogen, the primary linker includes at least two atoms of a hydrophilic amino acid, and nitrogen forms a peptide bond along with the main chain carbonyl of the hydrophilic amino acid. The hydrophilic amino acid may be, for example, arginine, aspartate, asparagine, glutamate, glutamine, histidine, lysine, ornithine, proline, serine, or threonine.

In an embodiment of the present disclosure, the branched linker of the antibody-active agent includes an amino acid having a side chain with a moiety that carries a charge at neutral pH in an aqueous solution, preferably arginine, aspartate, glutamate, lysine, or ornithine. The amino acid may be located anywhere on the branched linker. For example, the oxime of the branched linker may be covalently bonded to the polyethylene glycol unit of the branched linker. Alternatively or additionally, these amino acids may be present in the secondary linker, optionally within each secondary linker.

In a specific embodiment of the present disclosure, the linker-active agent includes a compound of Structural Formula below: 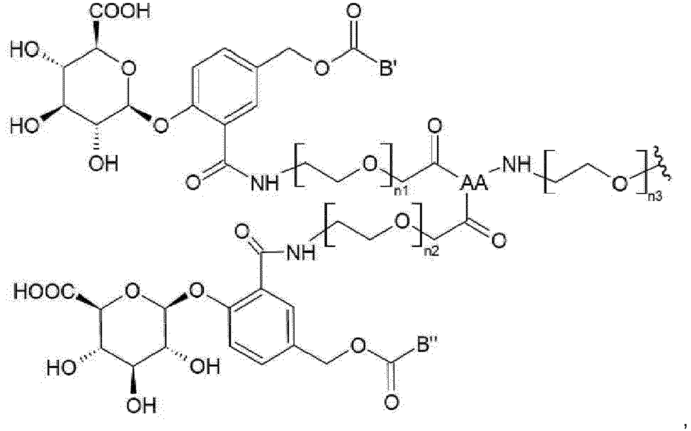
wherein B' and B" are each active agents which may be identical to or different from each other;
n1 to n3 each independently are an integer from 0 to 30; and
AA is an amino acid group.

In an embodiment of the present disclosure, AA is an amino acid group, and the amino acid group is a form in which one or more amino acids are bonded.

In an embodiment of the present disclosure, i) the linker includes a peptide sequence of multiple amino acids, and ii) at least two active agents are covalently bonded to the side chain of an amino acid.

In a specific embodiment of the present disclosure, the amino acid group is a group formed by a main-chain linkage or side-chain linkage of 1 to 20 amino acids.

In a specific embodiment of the present disclosure, the amino acid group is a group formed by a main-chain linkage or side-chain linkage of 1 to 20 arginine, aspartate, asparagine, glutamate, glutamine, histidine, lysine, ornithine, proline, serine or threonine residue(s).

In a specific embodiment of the present disclosure, the amino acid group is a group formed by a main-chain linkage or side-chain linkage of 1 to 20 arginine, aspartate, glutamate, lysine or ornithine residue(s).

In a specific embodiment of the present disclosure, the amino acid group includes at least one lysine of 1 to 20 amino acids.

In addition, the amino acid group includes a main-chain or side-chain linkage of lysine.

In an embodiment of the present disclosure, the branched linker including the fifth unit may be

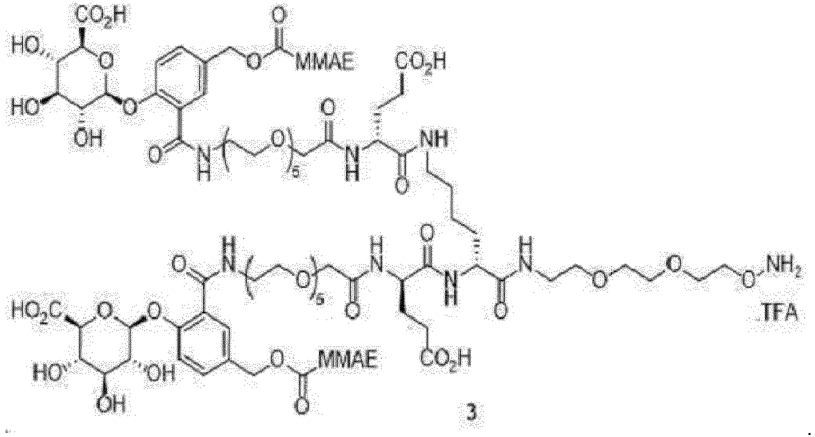

The term "active agent" as used herein refers to a compound that includes an active agent and covalently bonds the active agent to a linker or a part thereof.

In the present disclosure, the active agent moiety may be directly bonded to the linker, and one or more, particularly, two or more, three or more, or four or more active agent moieties may be directly bonded to the linker.

In addition, in the present disclosure, the active agent moiety may include a compound unit for direct binding of the active agent to the linker. In addition, in the present disclosure, a portion of the active agent moiety is cleavable, and the portion thereof is cleaved, thus enabling the active agent to be released in an intracellular environment. In addition, in the present disclosure, the number of bindings between active agent moieties and linkers may be determined in consideration of the DAR, pharmacological activity and the like of the conjugate. In an embodiment, the active agent moiety may refer to the whole including the following structure and an active agent, and thus enables direct binding between the active agent and the linker. However, these are for illustrative purposes and are not intended to limit the present disclosure. 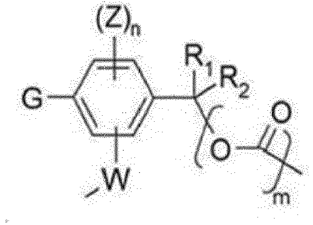 (wherein each moiety is the same as defined above.)

In an embodiment of the present disclosure, active agents are each independently selected from a chemotherapeutic agent and a toxin.

In addition, the active agent may be an immunomodulatory compound, an anticancer agent, an anti-viral agent, an antibacterial agent, an antifungal agent, an antiparasitic agent, or a combination thereof, and may be selected for use from active agents listed below:
(a) erlotinib, bortezomib, fulvestrant, sutent, letrozole, imatinib mesylate, PTK787/ZK 222584, oxaliplatin, 5-fluorouracil, leucovorin, rapamycin, lapatinib, lonafarnib, sorafenib, gefitinib, AG1478, AG1571, thiotepa, cyclophosphamide, busulfan, improsulfan, piposulfan, benzodopa, carboquone, meturedopa, uredopa, ethylenimine, altretamine, triethylenemelamine, triethylenephosphoramide, triethiylenethiophosphoramide, trimethylolomelamine, bullatacin, bullatacinone, camptothecin, topotecan, bryostatin, callystatin, CC-1065, adozelesin, carzelesin, bizelesin, cryptophycin 1, cryptophycin 8, dolastatin, duocarmycin, KW-2189, CB1-TM1, eleutherobin, pancratistatin, sarcodictyin, spongistatin, chlorambucil, chlornaphazine, cholophosphamide, estramustine, ifosfamide, mechlorethamine, melphalan, novembichin, phenesterine, prednimustine, trofosfamide, uracil mustard, carmustine, chlorozotocin, fotemustine, lomustine, nimustine, ranimnustine, calicheamicin, calicheamicin gamma 1, calicheamicin omega 1, dynemicin, dynemicin A, clodronate, esperamicin, neocarzinostatin chromophore, aclacinomysins, actinomycin, antrmycin, azaserine, bleomycins, cactinomycin, carabicin, carninomycin, carzinophilin, chromomycins, dactinomycin, daunorubicin, detorubucin, 6-diazo-5-oxo-L-norleucine, doxorubicin, morpholino-doxorubicin, cyanomorpholino-doxorubicin, 2-pyrrolino-doxorubicin, liposomal doxorubicin, deoxydoxorubicin, epirubicin, esorubicin, marcellomycin, mitomycin C, mycophenolic acid, nogalamycin, olivomycins, peplomycin, potfiromycin, puromycin, quelamycin, rodorubicin, streptomigrin, streptozocin, tubercidin, ubenimex, zinostatin, zorubicin, 5-fluorouracil, denopterin, methotrexate, pteropterin, trimetrexate, fludarabine, 6-mercaptopurine, thiamiprine, thiguanine, ancitabine, azacitidine, 6-azauridine, carmofur, cytarabine, dideoxyuridine, doxifluridine, enocitabine, floxuridine, calusterone, dromostanolone, propionate, epitiostanol, mepitiostane, testolactone, aminoglutethimide, mitotane, trilostane, folinic acid, aceglatone, aldophosphamide glycoside, aminolevulinic acid, eniluracil, amsacrine, bestrabucil, bisantrene, edatraxate, defofamine, demecolcine, diaziquone, elfornithine, elliptinium acetate, etoglucid, gallium nitrate, hydroxyurea, lentinan, lonidainine, maytansine, ansamitocins, mitoguazone, mitoxantrone, mopidanmol, nitraerine, pentostatin, phenamet, pirarubicin, losoxantrone, 2-ethylhydrazide, procarbazine, polysaccharidek, razoxane, rhizoxin, sizofiran, spirogermanium, tenuazonic acid, triaziquone, 2,2',2"-trichlorotriethylamine, T-2 toxin, verracurin A, roridin A, anguidine, urethane, vindesine, dacarbazine, mannomustine, mitobronitol, mitolactol, pipobroman, gacytosine, arabinoside, cyclophosphamide, thiotepa, paclitaxel, albumin-engineered nanoparticle formulation of paclitaxel, docetaxel, chlorambucil, gemcitabine, 6-thioguanine, mercaptopurine, cisplatin, carboplatin , vinblastine, platinum, etoposide, ifosfamide, mitoxantrone, vincristine, vinorelbine, novantrone, teniposide, edatrexate, daunomycin, aminopterin, xeloda, ibandronate, CPT-11, topoisomerase inhibitor RFS 2000, difluoromethylornithine, retinoic acid, capecitabine, or a pharmaceutically acceptable salt, solvate or acid thereof;
(b) monokines, lymphokines, traditional polypeptide hormones, parathyroid hormones, thyroxine, relaxin, prorelaxin, glycoprotein hormone, follicle stimulating hormone, thyroid stimulating hormone, luteinizing hormone, hepatic growth factor, fibroblast growth factor, prolactin, placental lactogen, tumor necrosis factor, tumor necrosis factor-α, tumor necrosis factor-β, mullerian inhibiting substance, mouse gonadotropin-associated peptide, inhibin, activin, vascular endothelial growth factor, thrombopoietin, erythropoietin, osteoinductive factor, interferon, interferon-α, interferon-β, interferon-γ, colony stimulating factor (CSF), macrophage-CSF, granulocyte-macrophage-CSF, granulocyte-CSF, interleukin (IL), IL-1, IL-1α, IL-2, IL-3, IL-4, IL-5, IL-6, IL-7, IL-8, IL-9, IL-10, IL- 11, IL-12, tumor necrosis factor, polypeptide factor, LIF, kit ligand, or a combination thereof;
(c) diphtheria toxin, botulinum toxin, tetanus toxin, decentretoxin, cholera toxin, amanitin, α-amanitin, pyrrolobenzodiazepines, pyrrolobenzodiazepine derivatives, indolinobenzodiazepines, pyridinobenzodiazepines, tetrodotoxin, brevetoxin, ciguatoxin, ricin, AM toxin, auristatin, tubulysin, geldanamycin, maytansinoid, calicheamycin, daunomycin, doxorubicin, methotrexate, vindesine, SG2285, dolastatin, dolastatin analog, cryptophycin, camptothecin, rhizoxin, rhizoxin derivatives, CC-1065, CC-1065, analogs or derivatives, duocarmycin, enediyne antibiotics, esperamicin, epothilone, toxoid, or a combination thereof;
(d) an affinity ligand, wherein the affinity ligand is a substrate, an inhibitor, an active agent, a neurotransmitter, a radioisotope, or a combination thereof;
(e) a radioactive label, 32P, 35S, a fluorescent dye, an electron dense reagent, an enzyme, biotin, streptavidin, dioxigenin, hapten, an immunogenic protein, a nucleic acid molecule with a sequence complementary to a target, or a combination thereof;
(f) an immunomodulatory compound, an anticancer agent, an anti-viral agent, an anti-bacterial agent, an anti-fungal agent, an anti-parasitic agent, or a combination thereof;
(g) tamoxifen, raloxifene, droloxifene, 4-hydroxytamoxifen, trioxifene, keoxifene, LY117018, onapristone, or toremifene;
(h) 4(5)-imidazole, aminoglutethimide, megestrol acetate, exemestane, letrozole, or anastrozole;
(i) flutamide, nilutamide, bicalutamide, leuprolide, goserelin, or troxacitabine;
(j) an aromatase inhibitor;
(k) a protein kinase inhibitor;
(l) a lipid kinase inhibitor;
(m) an antisense oligonucleotide;
(n) a ribozyme;
(o) a vaccine; and
(p) an anti-angiogenic agent.

In an embodiment of the present disclosure, the active agent is 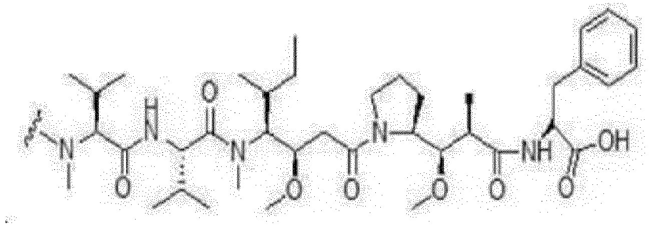 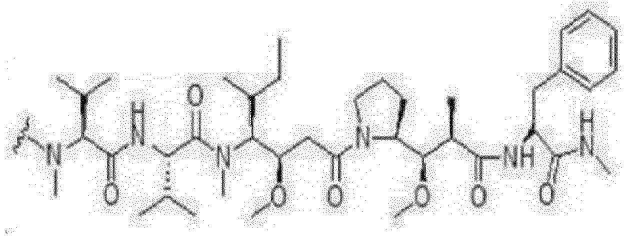 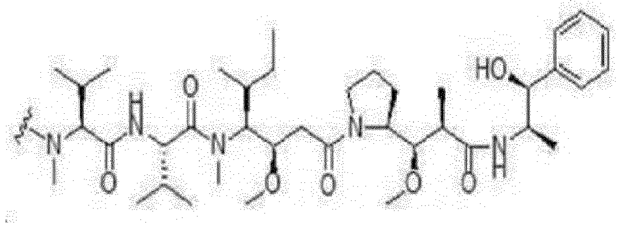 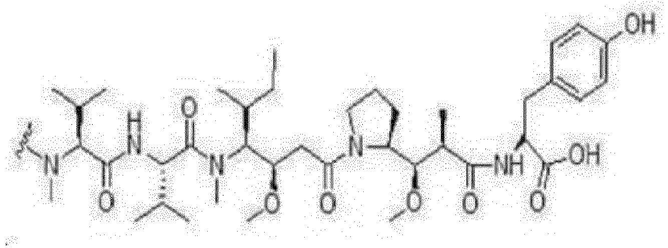 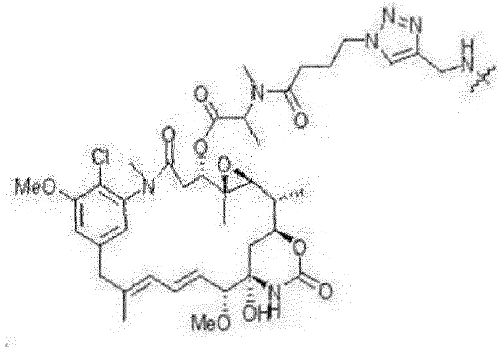 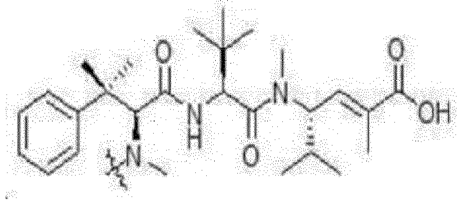 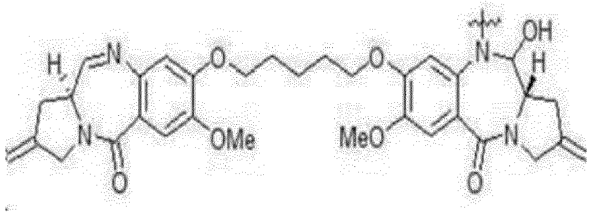 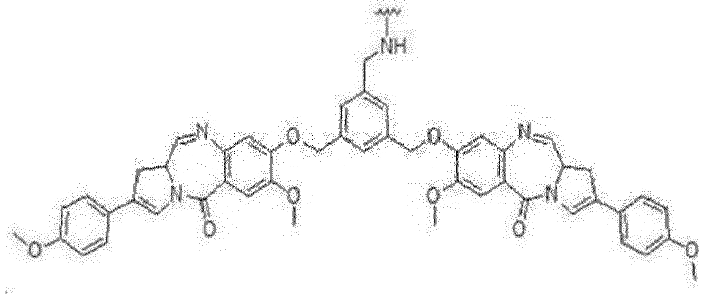 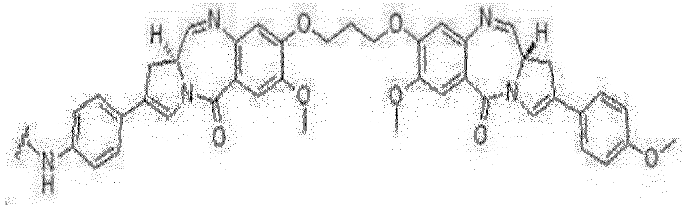 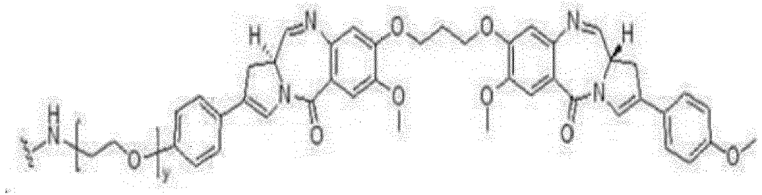 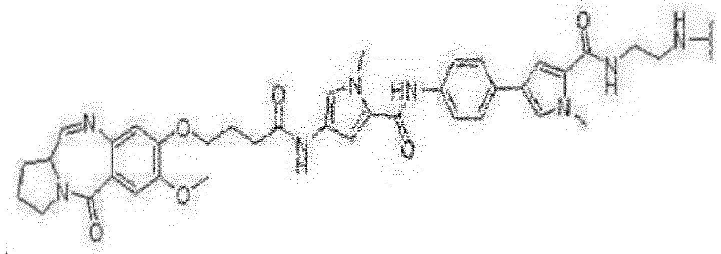 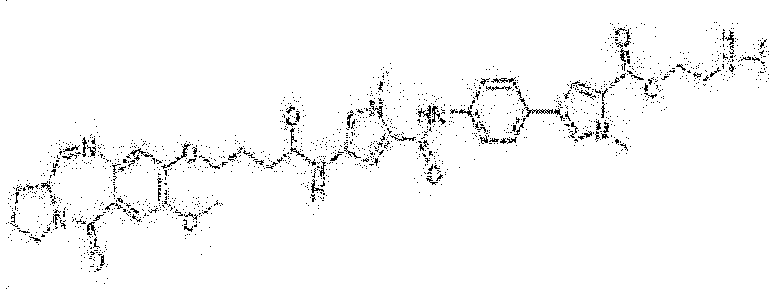 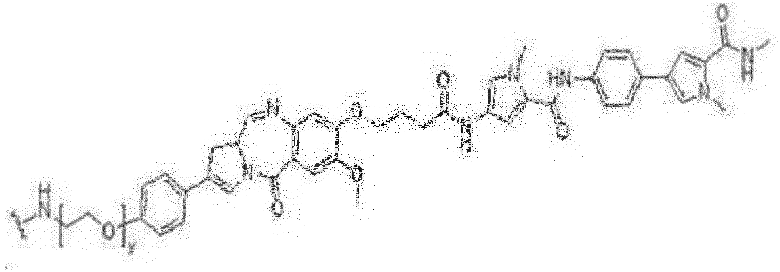 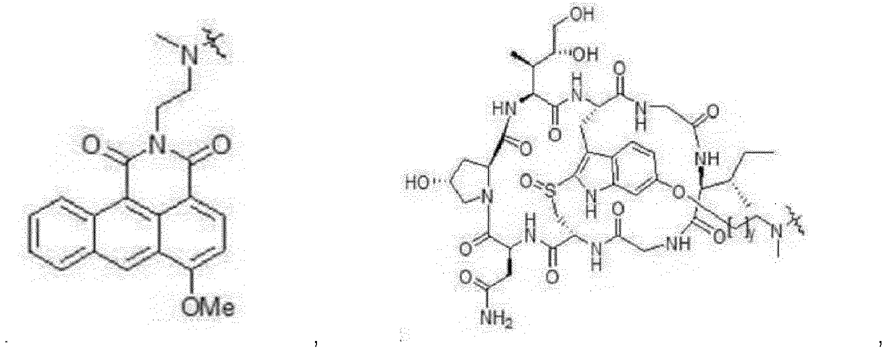 or 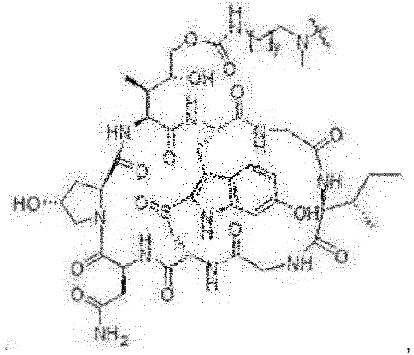 wherein y is an integer from 1 to 10.

Also, in another embodiment of the present disclosure, the active agent is a pyrrolobenzodiazepine dimer, and the linker and the antibody are linked via position N10 or N10' of the pyrrolobenzodiazepine dimer.

Any one selected from -C(O)O-*, -S(O)O-*, -C(O)-*, -C(O)NR-*, -S(O)₂NR-*, - (P(O)R')NR-*, -S(O)NR-*, and -PO₂NR-* is attached to each of positions N10 and N10' of the pyrrolobenzodiazepine dimer,
wherein * is a site to which the linker is attached, and
R and R' are each independently H, OH, N₃, CN, NO₂, SH, NH₂, ONH₂, NHNH₂, halo, substituted or unsubstituted C₁₋₈ alkyl, substituted or unsubstituted C₃₋₈ cycloalkyl, substituted or unsubstituted C₁₋₈ alkoxy, substituted or unsubstituted C₁₋₈ alkylthio, substituted or unsubstituted C₃₋₂₀ heteroaryl, substituted or unsubstituted C₅₋₂₀ aryl, or mono- or di-C₁₋₈ alkylamino,
wherein C₁₋₈ alkyl, C₃₋₈ cycloalkyl, C₁₋₈ alkoxy, C₁₋₈ alkylthio, C₃₋₂₀ heteroaryl, or C₅₋₂₀ aryl is substituted with a substituent selected from the group consisting of H, OH, N₃, CN, NO₂, SH, NH₂, ONH₂, NNH₂, halo, C₁₋₆ alkyl, C₁₋₆ alkoxy, and C₆₋₁₂ aryl.

A pyrrolobenzodiazepine dimer prodrug, or a pharmaceutically acceptable salt or solvate thereof may be used as an active agent.

More specifically, position N10 of the pyrrolobenzodiazepine dimer is substituted with X or position N'10 is substituted with X' wherein X or X' links the pyrrolobenzodiazepine dimer to the linker,
wherein X and X' are each independently selected from -C(O)O*, -S(O)O-*, -C(O)-*, -C(O)NR^{X}-*, -S(O)₂NR^{X}-*, -P(O)R'NR^{X}-*, -S(O)NR^{X}-*, and -PO₂NR^{X}-*,
wherein R^{X} and R^{X'} are independently selected from H, OH, N₃, CN, NO₂, SH, NH₂, ONH₂, NHNH₂, halo, C₁₋₈ alkyl, C₃₋₈ cycloalkyl, C₁₋₈ alkoxy, C₁₋₈ alkylthio, C₃₋₂₀ heteroaryl, C₅₋₂₀ aryl, or mono- or di-C₁₋₈ alkylamino.

In an embodiment of the present disclosure, a pyrrolobenzodiazepine dimer prodrug is provided as the active agent. When administered in the form of the prodrug according to the present disclosure, since it is necessary to convert into an effective drug by an additional reaction when exposed to the blood, the occurrence of unexpected side effects that may occur when the linker is decomposed may be prevented, toxicity to normal cells is reduced, and the drug is more stable, which are advantageous compared to existing PBD drugs.

In addition, in the production of antibody-drug conjugates, an antibody-drug conjugate produced using an existing method has high impurity content and has a risk that a drug having an unwanted structure resulting from a nucleophile attack to an exposed imine group can be produced, whereas the antibody-drug conjugate produced using the method according to the present disclosure has the advantage of easy separation due to high purity thereof, and has been shown to have improved physical properties compared to existing PBDs or PBD dimers.

In an embodiment of the present disclosure, the pyrrolobenzodiazepine dimer prodrug is a pyrrolobenzodiazepine dimer prodrug, or a pharmaceutically acceptable salt or solvate thereof, which have the structure of General Formula X or General Formula XI below: 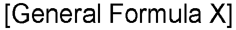 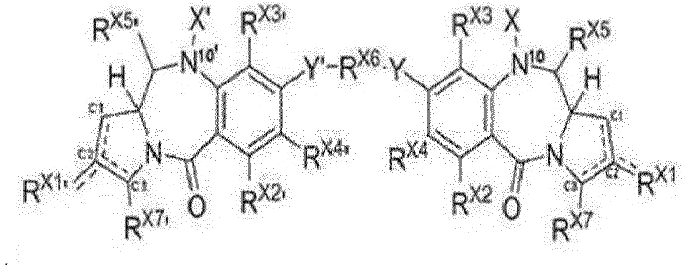 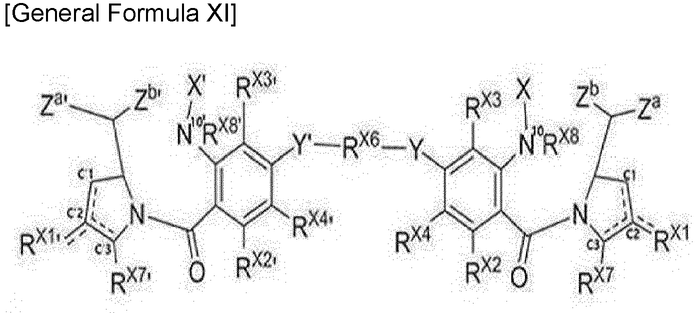 wherein, in General Formulae X and XI,
a dotted line denotes an optional a double bond between C1 and C2, between C2 and C3, between C'1 and C'2, or between C'2 and C'3;
R^{X1} and R^{X1'} are independently selected from H, OH, =O, =CH₂, CN, R^{m}, OR^{m}, =CH-R^{m'}, =C(R^{m'})₂, O-SO₂-R^{m}, CO₂R^{m}, COR^{m}, halo, and dihalo;
R^{m'} is selected from R^{m}, CO₂R^{m}, COR^{m}, CHO, CO₂H, and halo,
each R^{m} is independently selected from the group consisting of C₁₋₁₂ alkyl, C₂₋₁₂ alkenyl, C₂₋₁₂ alkynyl, C₅₋₂₀ aryl, C₅₋₂₀ heteroaryl, C₃₋₆ cycloalkyl, 3- to 7-membered heterocyclyl, 3- to 7-membered heterocycloalkyl, and 5- to 7-membered heteroaryl;
R^{X2}, R^{X2'} R^{X3}, R^{X3'}, R^{X5}, and R^{X5'} are independently selected from H, R^{m}, OH, OR^{m}, SH, SR^{m}, NH₂, NHR^{m}, NR^{m}₂, NO₂, and halo;
R^{X4} and R^{X4'} are independently selected from H, R^{m}, OH, OR^{m}, SH, SR^{m}, NH₂, NHR^{m}, NR^{m}₂, NO₂, halo, C₁₋₆ alkyl C₁₋₆ alkoxy, C₂₋₆ alkenyl, C₂₋₆ alkynyl, C₃₋₆ cycloalkyl, 3- to 7-membered heterocycloalkyl, C₅₋₁₂ aryl, 5- to 7-membered heteroaryl, -CN, -NCO, -ORⁿ, -OC(O)Rⁿ, -OC(O)NRⁿR^{n'}, -OS(O)Rⁿ, -OS(O)₂Rⁿ, -SRⁿ, -S(O)Rⁿ, -S(O)₂Rⁿ, - S(O)NRⁿR^{n'}, -S(O)₂NRⁿR^{n'}, -OS(O)NRⁿR^{n'}, -OS(O)₂NRⁿR^{n'}, -NRⁿR^{n'}, -NRⁿC(O)R^{o}, - NRⁿC(O)OR^{o}, -NRⁿC(O)NR^{o}R^{o'}, -NRⁿS(O)R^{o}, -NRⁿS(O)₂R^{o}, -NRⁿS(O)NR^{o}R^{o'}, - NRⁿS(O)₂NR^{o}R^{o'}, -C(O)Rⁿ, -C(O)ORⁿ, and -C(O)NRⁿR^{n'};
X and X' are independently selected from -C(O)O*, -S(O)O-*, -C(O)-*, -C(O)NR^{X}-*, -S(O)₂NR^{X}-*, -P(O)R'NR^{X}-*, -S(O)NR^{X}-*, or -PO₂NR^{X}-*;
R^{X} and R^{X'} are independently selected from H, OH, N₃, CN, NO₂, SH, NH₂, ONH₂, NHNH₂, halo, C₁₋₈ alkyl, C₃₋₈ cycloalkyl, C₁₋₈ alkoxy, C₁₋₈ alkylthio, C₃₋₂₀ heteroaryl, C₅₋₂₀ aryl, and mono- or di-C₁₋₈ alkylamino;
Y and Y' are independently selected from O, S, and N(H);
R^{X6} is independently selected from C₃₋₁₂ alkylene, C₃₋₁₂ alkenylene, and C₃₋₁₂ heteroalkylene;
R^{X7} and R^{X7'} are independently selected from H, C₁₋₆ alkyl, C₂₋₆ alkenyl, C₂₋₆ alkynyl, C₃₋₆ cycloalkyl, 3- to 7-membered heterocycloalkyl, C₆₋₁₀ aryl, 5- to 7-membered heteroaryl, -OR^{r}, -OC(O)R^{r}, -OC(O)NR^{r}R^{r'}, -OS(O)R^{r}, -OS(O)₂R^{r}, -SR^{r}, -S(O)R^{r}, -S(O)₂R^{r}, -S(O)NR^{r}R^{r'}, -S(O)₂NR^{r}R^{r'}, -OS(O)NR^{r}R^{r'}, -OS(O)₂NR^{r}R^{r'}, -NR^{r}R^{r'}, -NR^{r}C(O)R^{s}, - NR^{r}C(O)OR^{s}, -NR^{r}C(O)NR^{s}R^{s'}, -NRr^{S}(O)R^{s}, -NRr^{S}(O)₂R^{s}, -NRr^{S}(O)NR^{s}R^{s'}, - NRr^{S}(O)₂NR^{s}R^{s}, -C(O)R^{r}, -C(O)OR^{s}, or -C(O)NR^{r}R^{r'};
R^{r}, R^{r'}, R^{s}, and R^{s'} are each independently selected from H, C₁₋₇ alkyl, C₂₋₇ alkenyl, C₂₋₇ alkynyl, C₃₋₁₃ cycloalkyl, 3- to 7-membered heterocycloalkyl, C₅₋₁₀ aryl, and 5- to 7-membered heteroaryl;
R^{X8} and R^{X8'} are each independently selected from H, C₁₋₆ alkyl, C₂₋₆ alkenyl, C₂₋₆ alkynyl, C₃₋₆ heteroalkyl, 3- to 7-membered heterocycloalkyl, C₅₋₁₀ aryl, 5- to 7-membered heteroaryl, -S(O)R^{m}, -S(O)₂R^{m}, -S(O)NR^{m}R^{m'}, -S(O)₂NR^{m}R^{m'}, -NR^{m}R^{m'}, - NR^{m}C(O)R^{m}, -NR^{m}C(O)ORⁿ, -NR^{m}C(O)NRⁿR^{n'}, -NR^{m}S(O)Rⁿ, -NR^{m}S(O)₂Rⁿ, - NR^{m}S(O)NRⁿR^{n'}, -NR^{m}S(O)₂NRⁿR^{n'}, -C(O)R^{m}, -C(O)OR^{m}, and -C(O)NR^{m}R^{m'};
Z^{a} is selected from OR^{X12a} NR^{X12a}R^{X12a}, or SR^{X12a};
Z^{b} is selected from OR^{X13a}, NR^{X13a}R^{X13a}, or SR^{X13a};
Z^{a'} is selected from OR^{X12a'}, NR^{X12a'}R^{X12a'} , or SR^{X12a'};
Z^{b'} is selected from OR^{X13a'} , NR^{X13'}R^{X13a'}, or SR^{X13a'};
R^{X12a}, R^{X12a'}, R^{X13a}, and R^{X13a'} are each independently selected from H, C₁₋₆ alkyl, C₂₋₆ alkenyl, C₂₋₆ alkynyl, C₃₋₆ cycloalkyl, 3- to 7-membered heterocycloalkyl, C₅₋₁₀ aryl, 5-to 7-membered heteroaryl, -C(O)R^{X15a}, -C(O)OR^{X15a}, and -C(O)NR^{X15a}R^{X15a'};
R^{X15a} and R^{X15a'} are each independently selected from C₁₋₁₂ alkyl, C₂₋₁₂ alkenyl, C₂₋₁₂ alkynyl, C₅₋₂₀ aryl, C₅₋₂₀ heteroaryl, C₃₋₆ cycloalkyl, 3- to 7-membered heterocycle, 3-to 7-membered heterocycloalkyl, and 5- to 7-membered heteroaryl;
Z^{a} and Z^{b} are optionally bonded together with the atoms to which Z^{a} and Z^{a} are attached, to form a 3- to 7-membered heterocyclyl, 3- to 7-membered heterocycloalkyl, or 3- to 7-membered heteroaryl, and Z^{a'} and Z^{b'} are optionally bonded together with the atoms to which Z^{a'} and Z^{b'} are attached, to form a 3- to 7-membered heterocyclyl, 3- to 7-membered heterocycloalkyl, or 3- to 7-membered heteroaryl; and
Each Rⁿ, R^{n'}, R°, R^{o'}, R^{p}, and R^{p'} is independently selected from H, C₁₋₇ alkyl, C₂₋₇ alkenyl, C₂₋₇ alkynyl, C₃₋₁₃ cycloalkyl, 3- to 7-membered heterocycloalkyl, C₅₋₁₀ aryl, and 5- to 7-membered heteroaryl.

In an embodiment of the present disclosure, R^{m} is independently selected from the group consisting of C₁₋₁₂ alkyl, C₂₋₁₂ alkenyl, C₂₋₁₂ alkynyl, C₅₋₂₀ aryl, C₅₋₂₀ heteroaryl, C₃₋₆ cycloalkyl, 3- to 7-membered heterocyclyl, 3- to 7-membered heterocycloalkyl, and 5- to 7-membered heteroaryl.

In an embodiment of the present disclosure, R^{m} is additionally substituted with C₁₋₁₂ alkyl, C₂₋₁₂ alkenyl, C₂₋₁₂ alkynyl, C₅₋₂₀ aryl, C₅₋₂₀ heteroaryl, C₃₋₆ cycloalkyl, 3- to 7-membered heterocyclyl, 3- to 7-membered heterocycloalkyl, or 5- to 7-membered heteroaryl.

In an embodiment of the present disclosure, R^{X4} and R^{X4'} are independently selected from H, R^{m}, OH, OR^{m}, SH, SR^{m}, NH₂, NHR^{m}, NR^{m}R^{m'}, NO₂, halo, C₁₋₆ alkyl C₁₋₆ alkoxy, C₂₋₆ alkenyl, C₂₋₆ alkynyl, C₃₋₆ cycloalkyl, 3- to 7-membered heterocycloalkyl, C₅₋₁₂ aryl, 5- to 7-membered heteroaryl, -CN, -NCO, -ORⁿ, -OC(O)Rⁿ, -OC(O)NRⁿR^{n'}, - OS(O)Rⁿ, -OS(O)₂Rⁿ, -SRⁿ, -S(O)Rⁿ, -S(O)₂Rⁿ, -S(O)NRⁿR^{n'}, -S(O)₂NRⁿR^{n'}, - OS(O)NRⁿR^{n'}, -OS(O)₂NRⁿR^{n'}, -NRⁿR^{n'}, -NRⁿC(O)Ro, -NRⁿC(O)ORo, -NRⁿC(O)NR^{o}R^{o'}, - NRⁿS(O)R^{o}, -NRⁿS(O)₂R^{o}, -NRⁿS(O)NR^{o}R^{o'}, -NRⁿS(O)₂NR^{o}R^{o'}, -C(O)Rⁿ, -C(O)ORⁿ, and -C(O)NRⁿR^{n'},

R^{X4} or R^{X4'} is C₁₋₆ alkyl, C₁₋₆ alkoxy, C₂₋₆ alkenyl, C₂₋₆ alkynyl, C₃₋₆ cycloalkyl, 3- to 7-membered heterocycloalkyl, C₅₋₁₂ aryl, or 5- to 7-memebered heteroaryl, and is additionally substituted with at least one C₁₋₆ alkyl, C₁₋₆ alkoxy, C₂₋₆ alkenyl, C₂₋₆ alkynyl, C₃₋₆ cycloalkyl, 3- to 7-membered heterocycloalkyl, C₅₋₁₀ aryl, 5- to 7-membered heteroaryl, -OR^{p}, -OC(O)R^{p}, -OC(O)NR^{p}R^{p'}, -OS(O)R^{p}, -OS(O)₂R^{p}, -SR^{p}, -S(O)R^{p}, - S(O)₂R^{p}, -S(O)NR^{p}R^{p'}, -S(O)₂NR^{p}R^{p'}, -OS(O)NR^{p}R^{p'}, -OS(O)₂NR^{p}R^{p'}, -NR^{p}R^{p'}, - NR^{p}C(O)R^{q}, -NR^{p}C(O)OR^{q}, -NR^{p}C(O)NR^{q}R^{q'}, -NR^{p}S(O)R^{q}, -NR^{p}S(O)₂R^{q}, - NR^{p}S(O)NR^{q}R^{q'}, -NR^{p}S(O)₂NR^{q}R^{q'}, -C(O)R^{p}, -C(O)OR^{p}, or -C(O)NR^{p}R^{p}.

In an embodiment of the present disclosure, R^{X7} and R^{X7'} are independently selected from H, C₁₋₆ alkyl, C₂₋₆ alkenyl, C₂₋₆ alkynyl, C₃₋₆ cycloalkyl, 3- to 7-membered heterocycloalkyl, C₆₋₁₀ aryl, 5- to 7-membered heteroaryl, -OR', -OC(O)R^{r}, -OC(O)NR^{r}R^{r'}, -OS(O)R^{r}, -OS(O)₂R^{r}, -SR^{r}, -S(O)R^{r}, -S(O)₂R^{r}, -S(O)NR^{r}R^{r'}, -S(O)₂NR^{r}R^{r'}, -OS(O)NR^{r}R^{r'}, -OS(O)₂NR^{r}R^{r'}, -NR^{r}R^{r'}, -NR^{r}C(O)R^{s}, -NR^{r}C(O)OR^{s}, -NR^{r}C(O)NR^{s}R^{s'}, -NR^{r}S(O)R^{s}, - NR^{r}S(O)₂R^{s}, -NR^{r}S(O)NR^{s}R^{s'}, -NR^{r}S(O)₂NR^{s}R^{s}, -C(O)R^{r}, -C(O)OR^{s}, or -C(O)NR^{r}R^{r'}, and
R^{X7} or R^{X7'} is C₁₋₆ alkyl, C₂₋₆ alkenyl, C₂₋₆ alkynyl, C₃₋₆ cycloalkyl, 3- to 7-membered heterocycloalkyl, C₆₋₁₀ aryl, or 5- to 7-membered heteroaryl, and additionally substituted with C₁₋₆ alkyl, C₂₋₆ alkenyl, C₂₋₆ alkynyl, C₃₋₆ cycloalkyl, 3- to 7-membered heterocycloalkyl, C₆₋₁₀ aryl, 5- to 7-membered heteroaryl, -OR^{t}, -OC(O)R^{t}, -OC(O)NR^{t}R^{t'}, -OS(O)R^{t}, -OS(O)₂R^{t}, -SR^{t}, -S(O)R^{t}, -S(O)₂R^{t}, -S(O)NR^{t}R^{t'}, -S(O)₂NR^{t}R^{t'}, -OS(O)NR^{t}R^{t'}, - OS(O)₂NR^{t}R^{t'}, -NR^{t}R^{t'}, -NR^{t}C(O)R^{u}, -NR^{t}C(O)OR^{u}, -NR^{t}C(O)NR^{u}R^{u'}, -NR^{t}S(O)R^{u}, - NR^{t}S(O)₂R^{u}, -NR^{t}S(O)NR^{u}R^{u'}, -NR^{t}S(O)₂NR^{u}R^{u'}, -C(O)R^{t}, -C(O)OR^{t}, or -C(O)NR^{t}R^{t'},
wherein R^{r}, R^{r'}, R^{s}, R^{s'}, R^{t}, R^{t'}, R^{u,} and R^{u'} are independently selected from H, C₁₋₇ alkyl, C₂₋₇ alkenyl, C₂₋₇ alkynyl, C₃₋₁₃ cycloalkyl, 3- to 7-membered heterocycloalkyl, C₅₋₁₀ aryl, and 5- to 7-membered heteroaryl.

In an embodiment of the present disclosure, R^{X1} and R^{X1'} are independently selected from R^{m}, and
R^{m} is selected from C₁₋₆ alkyl, C₂₋₆ alkenyl, C₅₋₇ aryl, and C₃₋₆ heteroaryl.

In an embodiment of the present disclosure, R^{X2}, R^{X2'}, R^{X3}, R^{X3'}, R^{X5}, and R^{X5'} are independently selected from H or OH.

In an embodiment of the present disclosure, R^{X4} and R^{X4'} are independently selected from R^{m}, and
R^{m} is C₁₋₆ alkoxy.

In an embodiment of the present disclosure, R^{X4} and R^{X4'} are independently any one selected from methoxy, ethoxy, and butoxy.

In an embodiment of the present disclosure, Y and Y' are O.

In an embodiment of the present disclosure, R^{X6} is C₃₋₁₂ alkylene, C₃₋₁₂ alkenylene, or C₃₋₁₂ heteroalkylene, and R^{X6} is substituted with NH₂, -NHR^{m}, -NHC(O)R^{m}, - NHC(O)CH₂-[OCH₂CH₂]ₙ-R^{XX}, or -[CH₂CH₂O]ₙ-R^{XX},
R^{XX} is H, OH, N₃, CN, NO₂, SH, NH₂, ONH₂, NHNH₂, halo, C₁₋₈ alkyl, C₃₋₈ cycloalkyl, C₁₋₈ alkoxy, C₁₋₈ alkylthio, C₃₋₂₀ heteroaryl, C₅₋₂₀ aryl, or mono- or di-C₁₋₈ alkylamino, and
n is an integer from 1 to 6.

In an embodiment of the present disclosure, the pyrrolobenzodiazepine dimer is represented by General Formula X below: 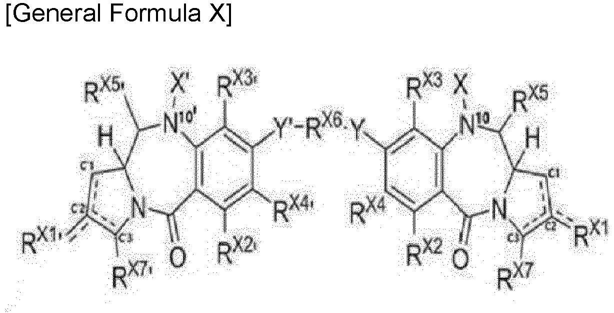 wherein, in General Formula X,
a dotted line denotes an optional double bond between C1 and C2, between C2 and C3, between C'1 and C'2, or between C'2 and C'3;
R^{X1} and R^{X1'} are each independently selected from H, OH, =O, =CH₂, CN, R^{m}, OR^{m}, =CH-R^{m}, =C(R^{m'})₂, OSO₂-R^{m}, CO₂R^{m}, COR^{m}, halo, or dihalo;
R^{X2}, R^{X2'}, R^{X3}, and R^{X3'} are each independently selected from H, R^{m}, OH, OR^{m}, NR^{m}₂, NO₂, and halo;
R^{X4} and R^{X4'} are each independently selected from H, R^{m}, OH, OR^{m}, SH, SR^{m}, NH₂, NHR^{m}, NR^{m}₂, halo, and C₁₋₆ alkyl;
R^{X5} and R^{X5'} are each independently selected from H, R^{m}, OH, OR^{m}, SH, SR^{m}, NH₂, NHR^{m}, NR^{m}₂, -NR^{m}R^{m'}, NO₂, -NR^{m}C(O)R^{m'}, -NR^{m}C(O)OR^{m'}, -NR^{m}C(O)NR^{m}R^{m'}, - S(O)R^{m}, -S(O)₂R^{m}, -S(O)NR^{m}R^{m'}, -S(O)₂NR^{m}R^{m'}, -NR^{m}S(O)R^{m'}, -NR^{m}S(O)₂R^{m'}, -P(O)R^{m}, -P(O)₂R^{m}, -P(O)NR^{m}R^{m'}, -P(O)₂NR^{m}R^{m'}, -NR^{m}P(O)R^{m'}, -NR^{m}P(O)₂R^{m'}, and halo;
Y and Y' are each independently O, S, or N(H);
R^{X6} is independently C₃₋₁₂ alkylene, C₃₋₁₂ alkenylene, or C₃₋₁₂ heteroalkylene; R^{X6} is unsubstituted or substituted with -NH₂, -NHR^{m}, -NHC(O)R^{m}, -NHC(O)CH₂-[OCH₂CH₂]ₙ-R^{XX}, or -[CH₂CH₂O]ₙ-R^{XX};
R^{XX} is H, OH, N₃, CN, NO₂, SH, NH₂, ONH₂, NHNH₂, halo, C₁₋₈ alkyl, C₃₋₈ cycloalkyl, C₁₋₈ alkoxy, C₁₋₈ alkylthio, C₃₋₂₀ heteroaryl, C₅₋₂₀ aryl, or mono- or di-C₁₋₈ alkylamino, wherein n is an integer from 1 to 6;
R^{X7} and R^{X7'} are each independently H, C₁₋₆ alkyl, C₂₋₆ alkenyl, C₂₋₆ alkynyl, - C(O)R^{r}, -C(O)OR^{s}, or -C(O)NR^{r}R^{r'};
R^{r}, R^{r'}, and R^{s} are each independently H, C₁₋₇ alkyl, C₂₋₇ alkenyl, C₂₋₇ alkynyl, C₃₋₁₃ cycloalkyl, 3- to 7-membered heterocycloalkyl, C₅₋₁₀ aryl, or 5- to 7-membered heteroaryl;
each R^{m'} is independently selected from R^{m}, CO₂R^{m}, COR^{m}, CHO, CO₂H, and halo; and
each R^{m} is independently selected from the group consisting of H, OH, C₁₋₁₂alkyl, C₁₋₁₂ alkoxy, C₂₋₁₂ alkenyl, C₂₋₁₂ alkynyl, C₅₋₂₀ aryl, C₅₋₂₀ heteroaryl, C₃₋₆ cycloalkyl, 3- to 7-membered heterocyclyl, 3- to 7-membered heterocycloalkyl, and 5- to 7-membered heteroaryl, wherein at least one hydrogen atom of C₅₋₂₀ aryl, C₅₋₂₀ heteroaryl, C₃₋₆ cycloalkyl, 3- to 7-membered heterocyclyl, 3- to 7-membered heterocycloalkyl, and 5- to 7-membered heteroaryl may be substituted with OH, =O, C₁₋₁₂ alkyl, or C₁₋₁₂ alkoxy.

In an embodiment of the present disclosure, R^{X1} and R^{X1'} are each independently selected from: =CH₂; C₁₋₆ alkyl; C₁₋₆ alkoxy; C₂₋₆ alkenyl; C₅₋₇ aryl; C₃₋₆ heteroaryl; or C₅₋₇ aryl substituted with C₁₋₆ alkyl or C₁₋₆ alkoxy.

In an embodiment of the present disclosure, the R^{X2}, R^{X2'}, R^{X3}, and R^{X3'} are each independently selected from H or OH.

In an embodiment of the present disclosure, R^{X5} and R^{X5'} are each independently selected from H, OH, -S(O)R^{m}, S(O)₂R^{m}, -P(O)R^{m}, and -P(O)₂R^{m}, wherein R^{m} is H, OH, C₁₋₁₂ alkyl, C₁₋₁₂ alkoxy, C₂₋₁₂ alkenyl, or C₂₋₁₂ alkynyl.

In an embodiment of the present disclosure, R^{X4} and R^{X4'} are each independently C₁₋₆ alkoxy.

In an embodiment of the present disclosure, R^{X4} and R^{X4'} are independently any one selected from methoxy, ethoxy, and butoxy.

In an embodiment of the present disclosure, Y and Y' are O.

In an embodiment of the present disclosure, R^{X6} is C₃₋₁₂ alkylene, C₃₋₁₂ alkenylene, or C₃₋₁₂ heteroalkylene, and the R^{X6} is substituted with -NH₂, -NHR^{m}, -NHC(O)R^{m}, - NHC(O)CH₂-[OCH₂CH₂]ₙ-R^{XX}, or -[CH₂CH₂O]ₙ-R^{XX},
R^{XX} is H, OH, N₃, CN, NO₂, SH, NH₂, ONH₂, NHNH₂, halo, C₁₋₈ alkyl, C₃₋₈ cycloalkyl, C₁₋₈ alkoxy, C₁₋₈ alkylthio, C₃₋₂₀ heteroaryl, C₅₋₂₀ aryl, or mono- or di-C₁₋₈ alkylamino, and
n is an integer from 1 to 6.

Also, in an embodiment of the present disclosure, the active agent is a pyrrolobenzodiazepine dimer represented by General Formula XII or General Formula XIII: 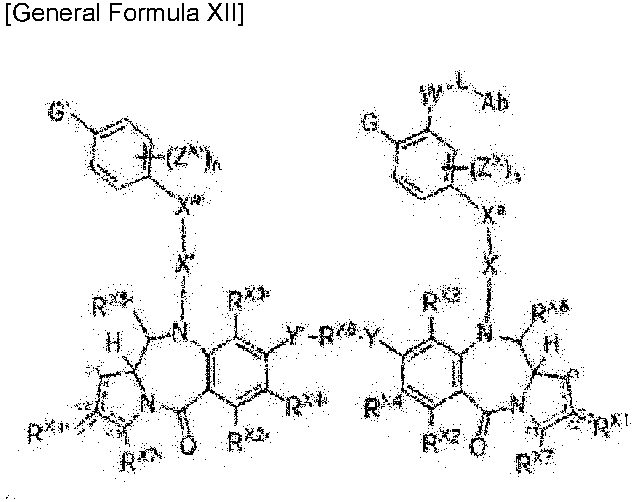 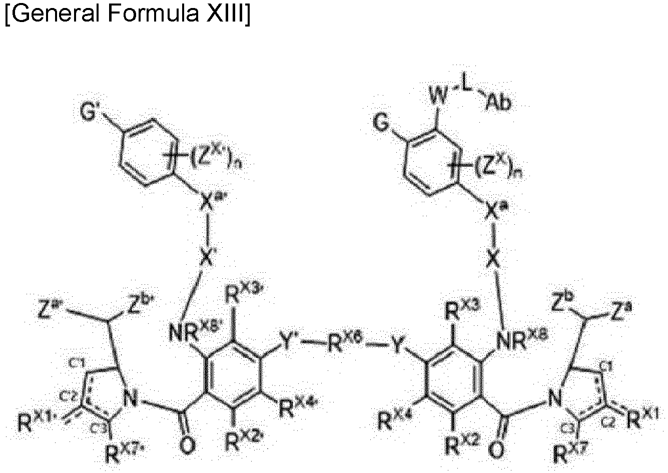
wherein X^{a} and X^{a'} independently selected from a bond or C₁₋₆ alkylene;
Z^{X'} and Z^{X} are independently selected from hydrogen, C₁₋₈ alkyl, halogen, cyano, nitro, 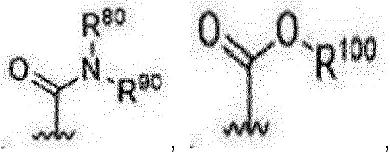 or -(CH₂)ₘ-OCH₃;
R⁸⁰, R⁹⁰, and R¹⁰⁰ are each selected from hydrogen, C₁₋₈ alkyl, C₂₋₆ alkenyl, and C₁₋₆ alkoxy; and
m is an integer from 0 to 12.
Z^{X'} and Z^{X} are independently any one selected from hydrogen, 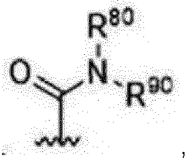 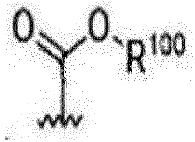 , and -(CH₂)ₘ-OCH₃,
R⁸⁰, R⁹⁰, and R¹⁰⁰ are each any one selected from hydrogen, C₁₋₃ alkyl, and C₁₋₃ alkoxy, and
m is an integer from 1 to 6.

In an embodiment of the present disclosure, the [LINKER-(B)_{I}] prodrug is any one selected from 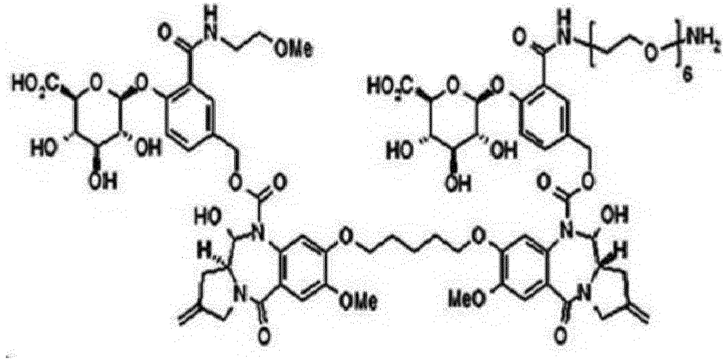 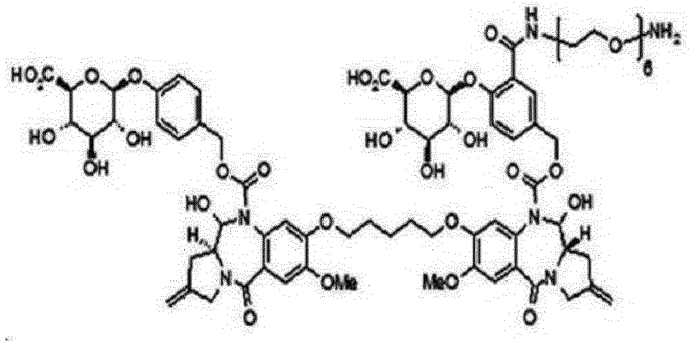 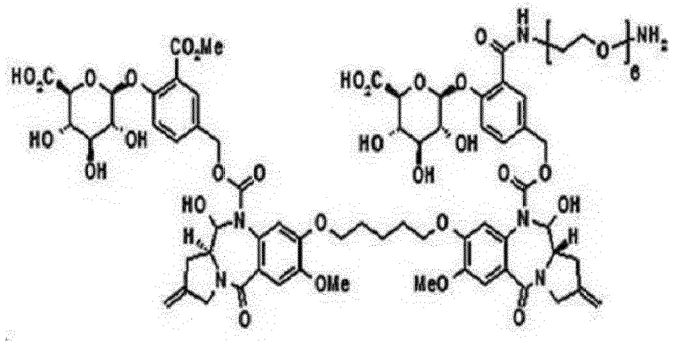 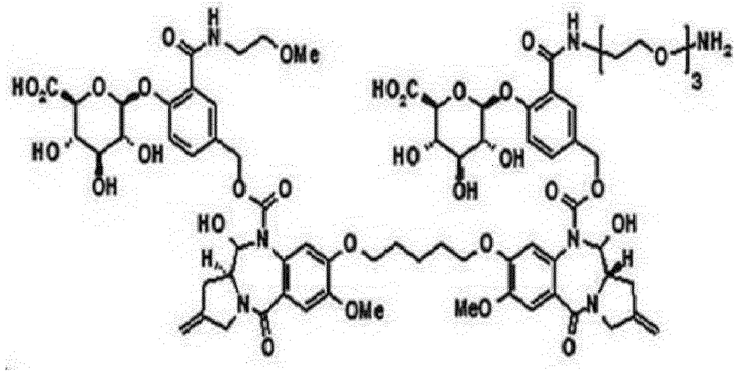 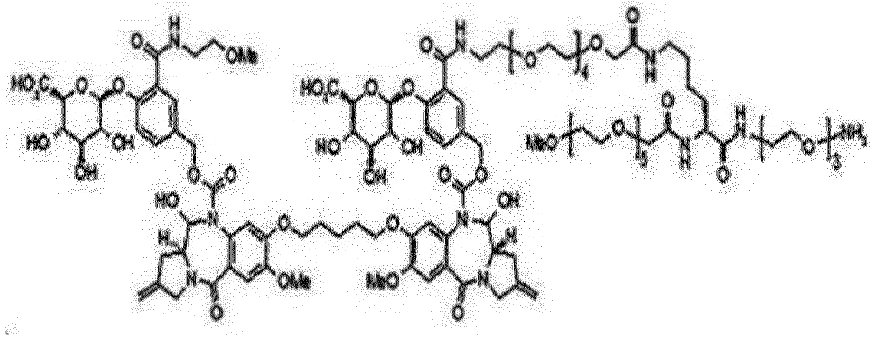 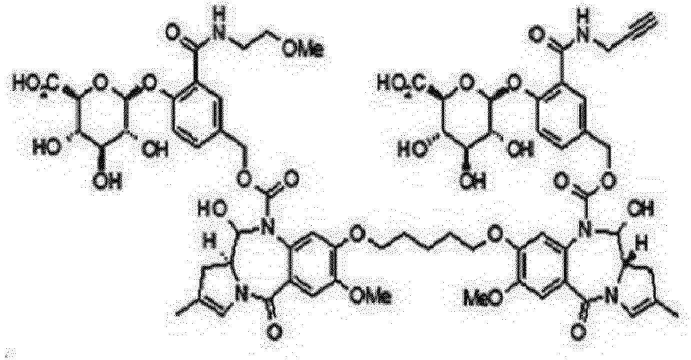 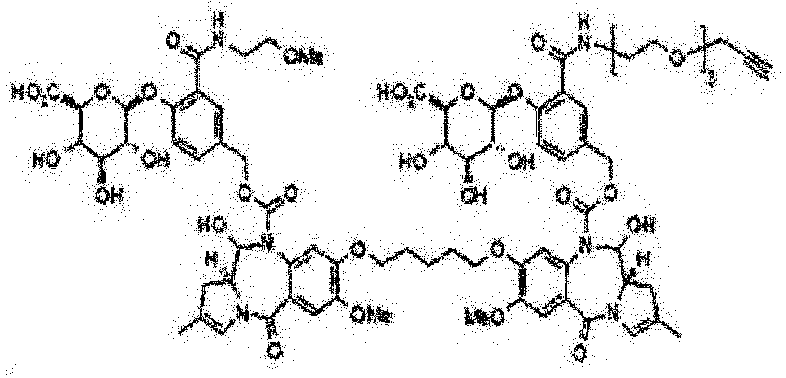 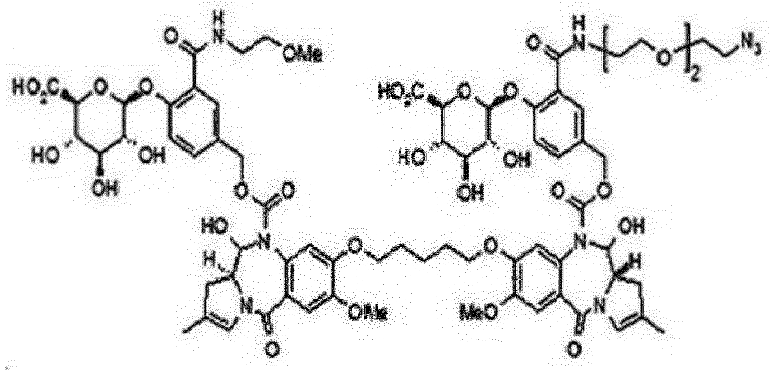 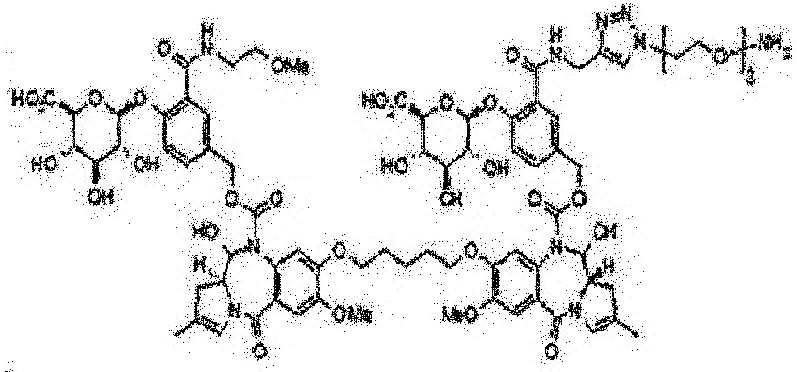 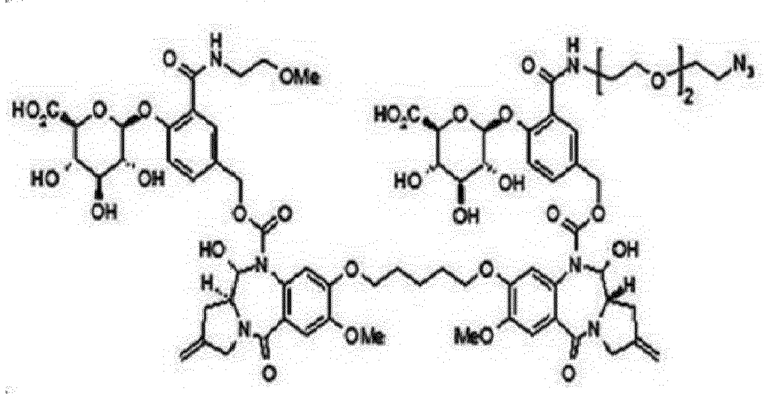 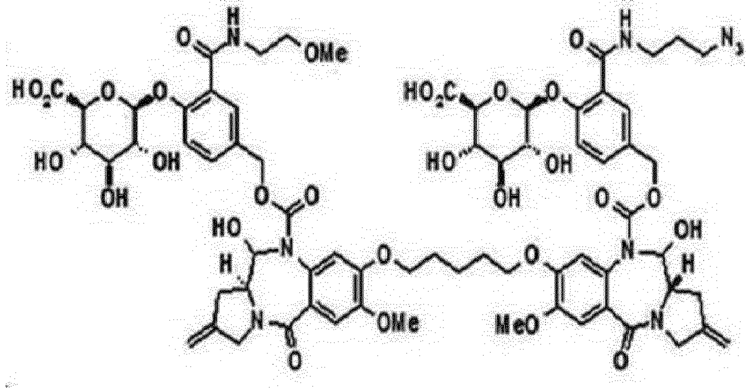 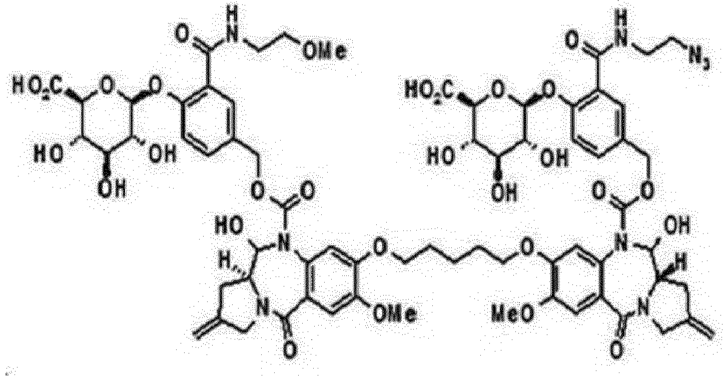 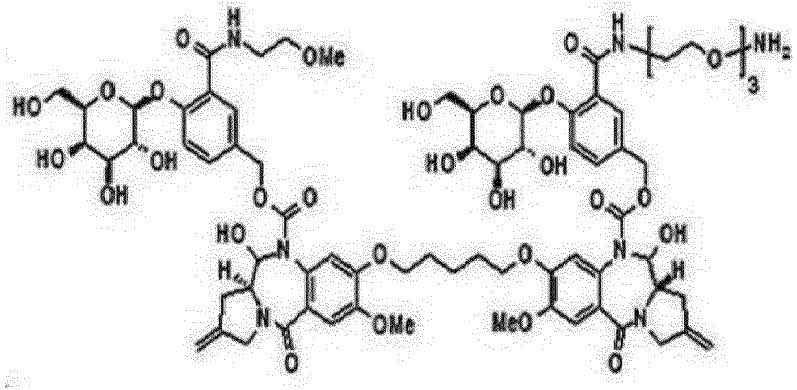 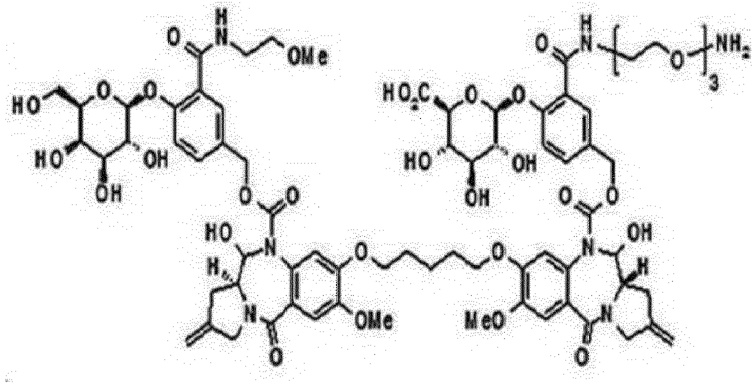 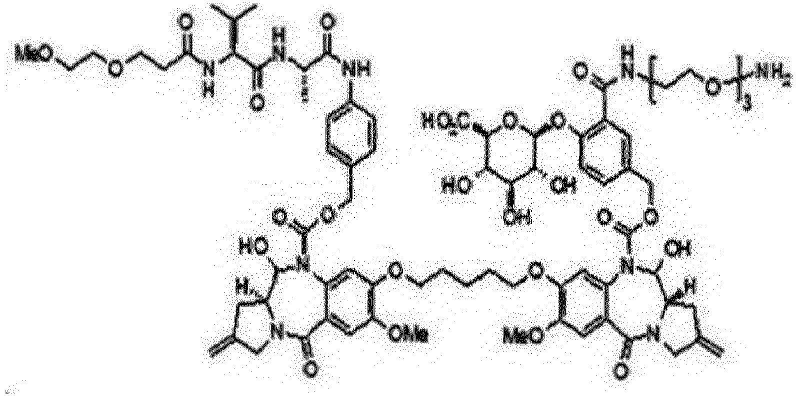 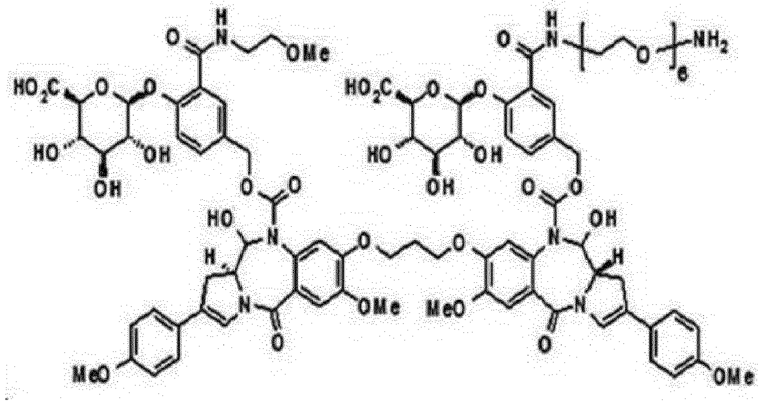 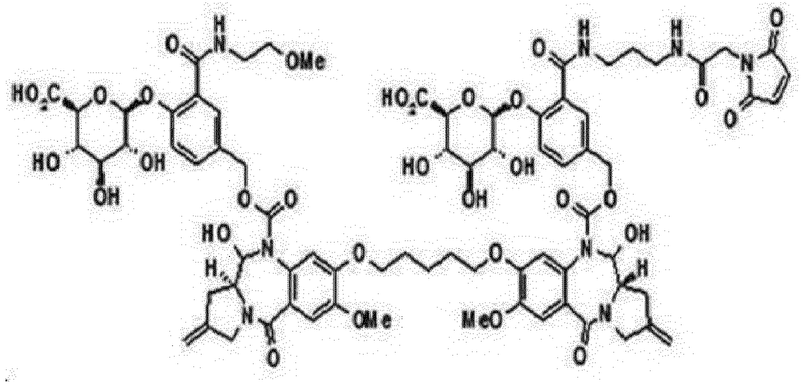 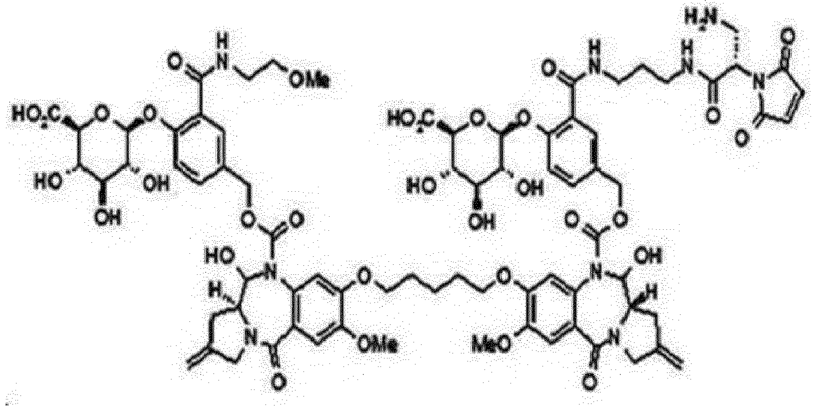 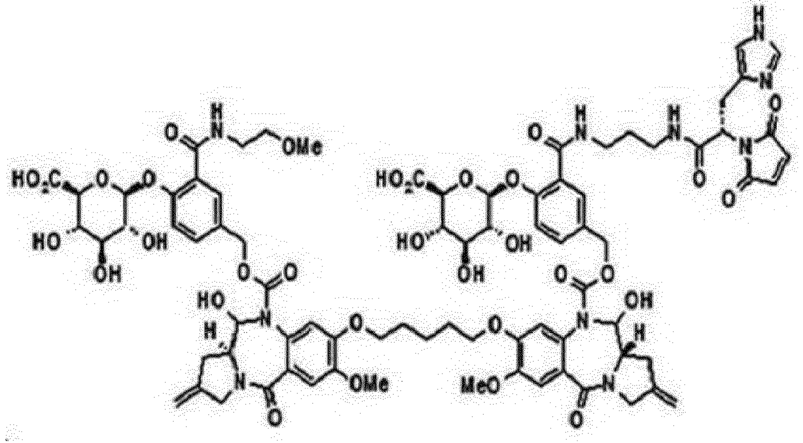 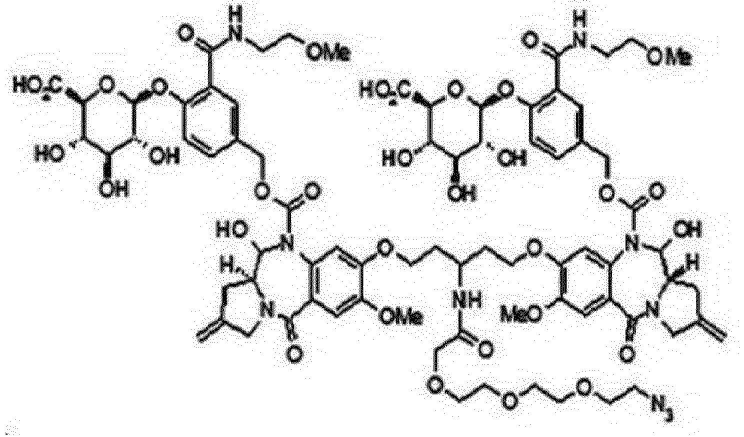 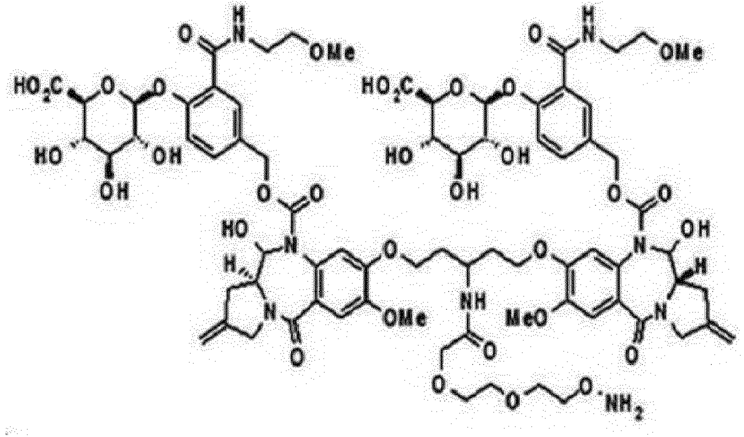 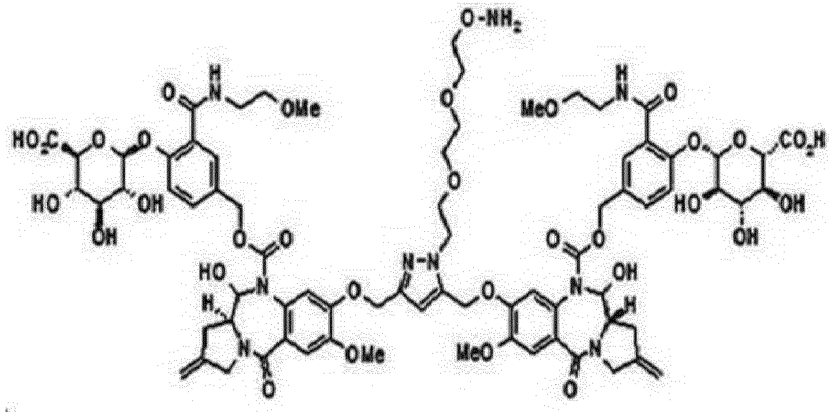 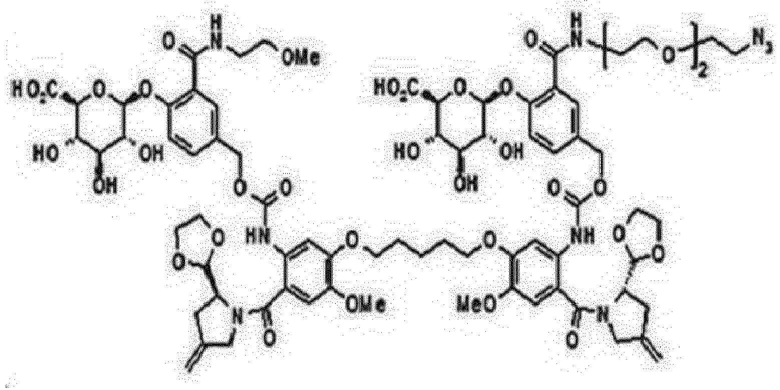 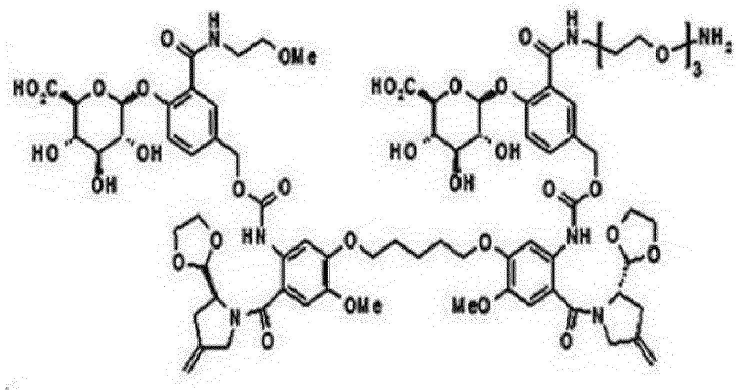 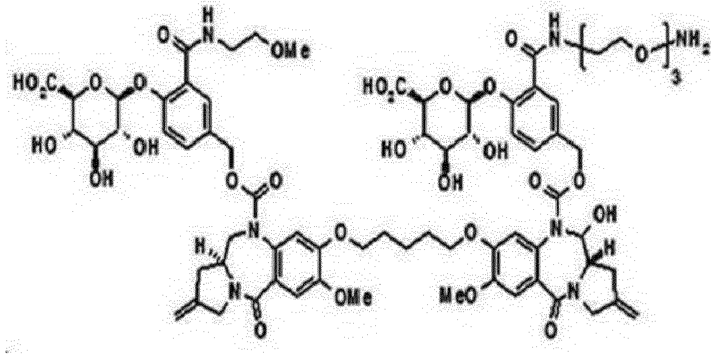 and

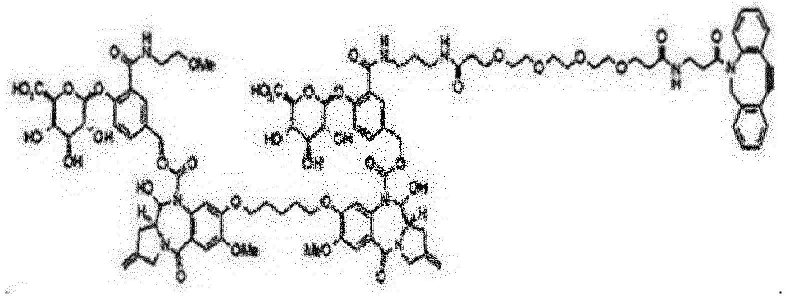

In addition, in an embodiment of the present disclosure, the [LINKER-(B)_{I}] prodrug is 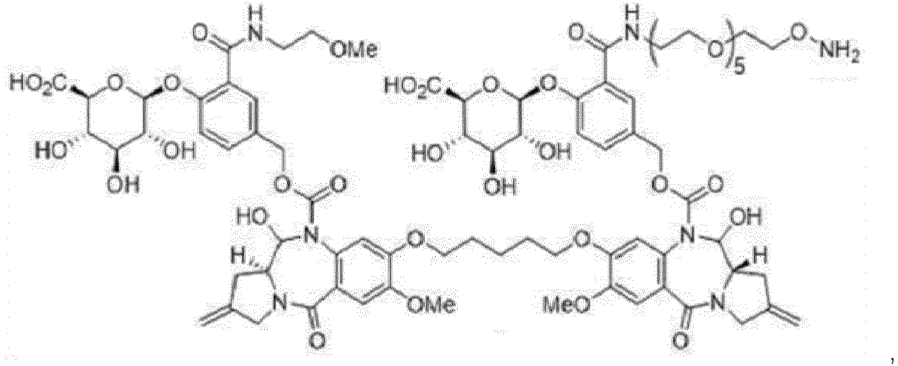 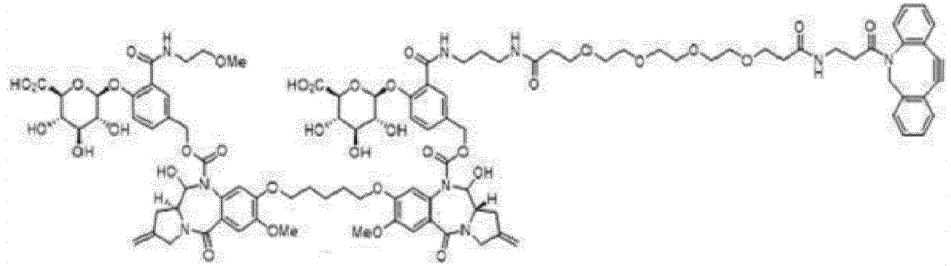 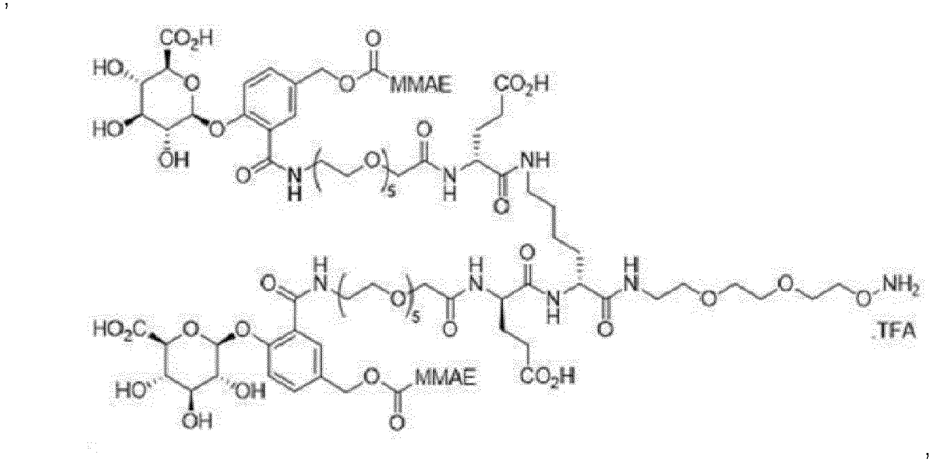 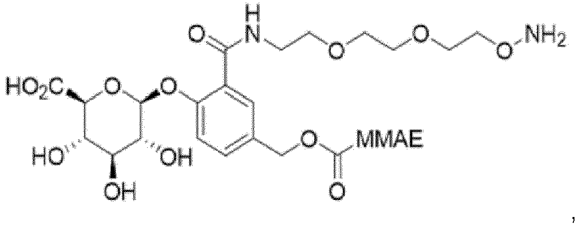 or 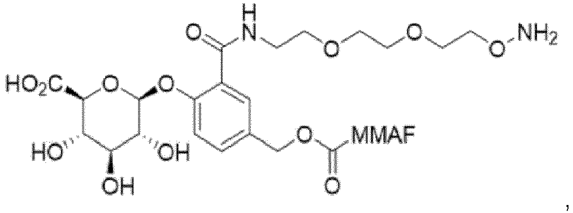
wherein MMAE is monomethyl auristatin E, and
MMAF is monomethyl auristatin F.

In an embodiment of the present disclosure, the [LINKER-(B)_{I}] prodrug has the following structure: 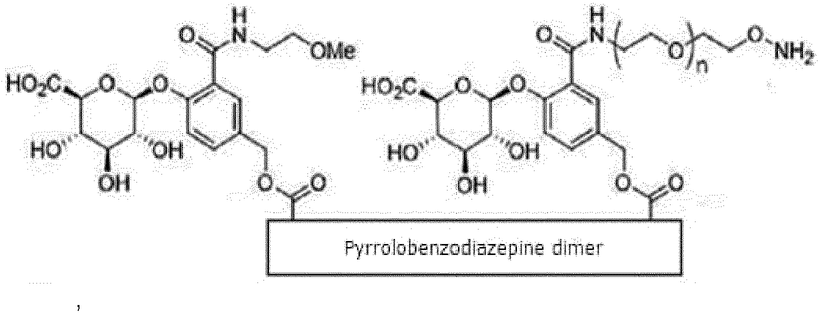 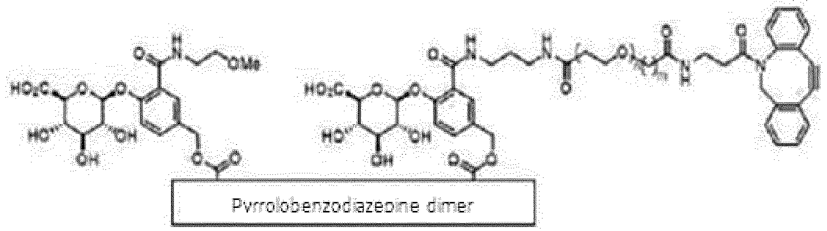 , or 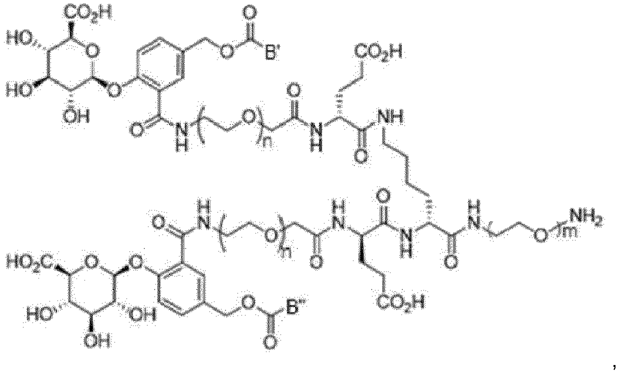
wherein: B' and B" are each active agents which may be identical to or different from each other; and
m and n each independently are an integer from 0 to 30.

Specifically, Pyrrolobenzodiazepine dimer denotes a site which is linked to X and X' of General Formula X, and more specifically, is

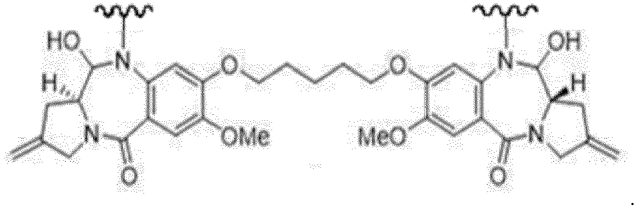

The present disclosure also provides a pharmaceutical composition for the prevention or treatment of hyperproliferation, cancer, or an angiogenic disease, including the conjugate.

In an embodiment of the present disclosure, the pharmaceutical composition further includes a pharmaceutically effective amount of a chemotherapeutic agent.

In an embodiment of the present disclosure, the cancer is any one selected from lung cancer, small cell lung cancer, gastrointestinal cancer, colon cancer, bowel cancer, breast cancer, ovarian cancer, prostate cancer, testicular cancer, liver cancer, kidney cancer, bladder cancer, pancreatic cancer, brain cancer, sarcoma, osteosarcoma, Kaposi's sarcoma, and melanoma.

The present disclosure also provides a pharmaceutical preparation including the conjugate.

The conjugate of the present disclosure may be administered via, for example, an oral or parenteral route. In this regard, parenteral refers to the route of administration in a broad sense, and includes, for example, transdermal, intravenous, intraarterial, intraperitoneal, intramuscular, subcutaneous, intradermal, sublingual, intrathecal, inhalation, ocular, rectal, vaginal, and ventricular administration.

The composition is formulated using generally used diluents or excipients such as fillers, extenders, binders, wetting agents, disintegrating agents, surfactants, and the like.

Solid preparations for oral administration include tablets, pills, powders, granules, capsules, troches, and the like, and such solid preparations are formulated by mixing at least one compound according to the present disclosure with at least one excipient, e.g., starch, calcium carbonate, sucrose, lactose, gelatin, or the like. In addition to simple excipients, lubricants such as magnesium stearate and talc are also used. Examples of liquid preparations for oral administration include suspending agents, liquids for internal use, emulsions, syrups, and the like, and these liquid preparations may include, in addition to simple commonly used diluents, such as water and liquid paraffin, various types of excipients, for example, a wetting agent, a sweetener, a flavoring agent, a preservative, and the like.

Preparations for parenteral administration include an aqueous sterile solution, a non-aqueous solvent, a suspending agent, an emulsion, an injection, a freeze-dried preparation, a suppository, and the like. In preparations for parenteral administration, propylene glycol, polyethylene glycol, a vegetable oil such as olive oil, an injectable ester such as ethyl oleate, and the like may be used as the non-aqueous solvent and the suspending agent. As suppository bases, Witepsol, Macrogol, Tween 61, cacao butter, laurin butter, glycerol, gelatin, and the like may be used.

The conjugate according to the present disclosure is administered in a pharmaceutically effective amount. The term "pharmaceutically effective amount" as used herein refers to an amount sufficient to treat diseases at a reasonable benefit/risk ratio applicable to medical treatment, and an effective dosage level may be determined according to factors including type of diseases of patients, the severity of disease, the activity of drugs, sensitivity to drugs, administration time, administration routes, excretion rate, treatment periods, and simultaneously used drugs, and other factors well known in the medical field. The composition of the present disclosure may be administered as an individual therapeutic agent or in combination with other therapeutic agents, may be administered sequentially or simultaneously with conventional therapeutic agents, and may be administered in a single dose or multiple doses. It is important to administer the composition in the minimum amount that enables achievement of the maximum effects without side effects in consideration of all the above-described factors, and this may be easily determined by those of ordinary skill in the art.

Specifically, an effective amount of the conjugate according to the present disclosure may vary according to the age, gender, and body weight of a patient, and the conjugate may be administered in an amount of 0.01 mg/kg to 150 mg/kg, more particularly 0.1 mg/kg to 100 mg/kg daily or every other day, or may be administered once or three times a day. However, since the effective amount may increase or decrease depending on administration route, severity, gender, body weight, age, and the like, the effective amount is not intended to limit the scope of the present disclosure in any way.

### [ADVANTAGEOUS EFFECTS OF DISCLOSURE]

An antibody-drug conjugate according to the present disclosure includes an anti-CLDN18.2 antibody that specifically binds to cells expressing claudin 18 isoform 2 (CLDN18.2) and is internalized into the cells, and thus can effectively, specifically and selectively deliver a drug, and has a stable bond between an antibody and the drug, thus enabling desired cytotoxicity while maintaining *in vivo* stability. In particular, there is the effect of providing a drug-linker-ligand system which enables a drug and/or a toxin to stably reach a target cell to thereby effectively exhibit efficacy and remarkably reduce toxicity, by applying a technology for a linker containing a self-immolative group, which is more stable in plasma and stable also in body circulation, and enables a drug to be easily released in cancer cells to maximize efficacy.

### [BRIEF DESCRIPTION OF DRAWINGS]

FIG. 1 illustrates the results of a cell binding experiment using a flow cytometer, performed to confirm the ability of an antibody to bind to cancer cells.
FIGS. 2 and 3 are graphs showing the *in-vivo* experimental results of ADC samples.

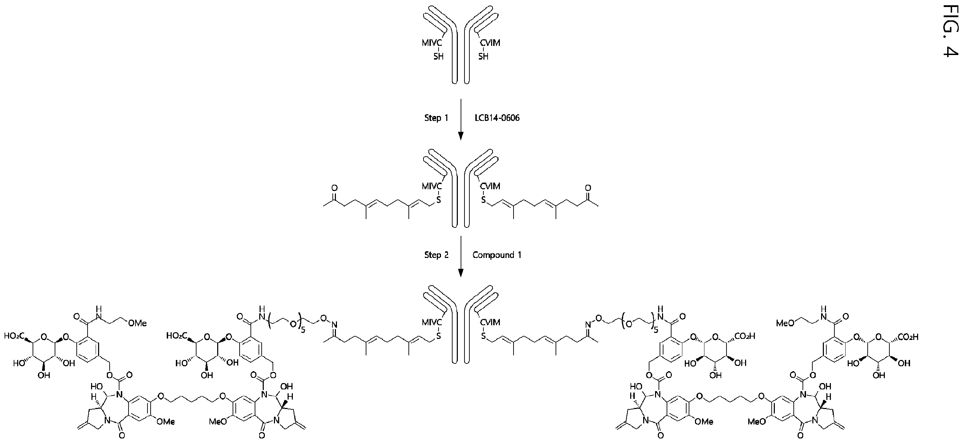

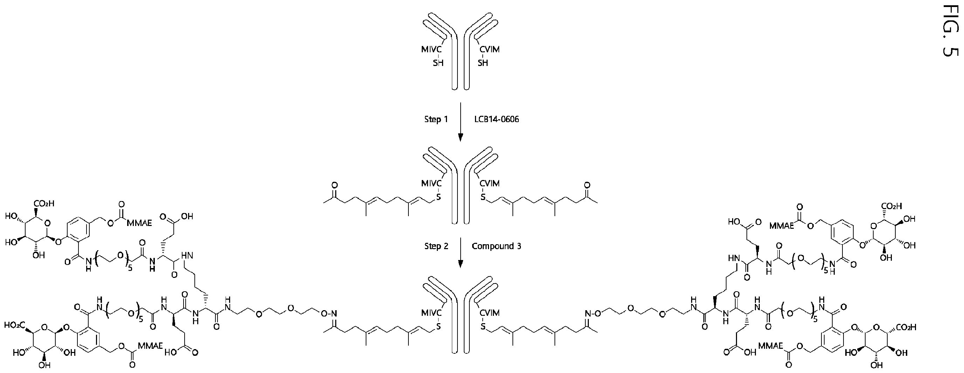

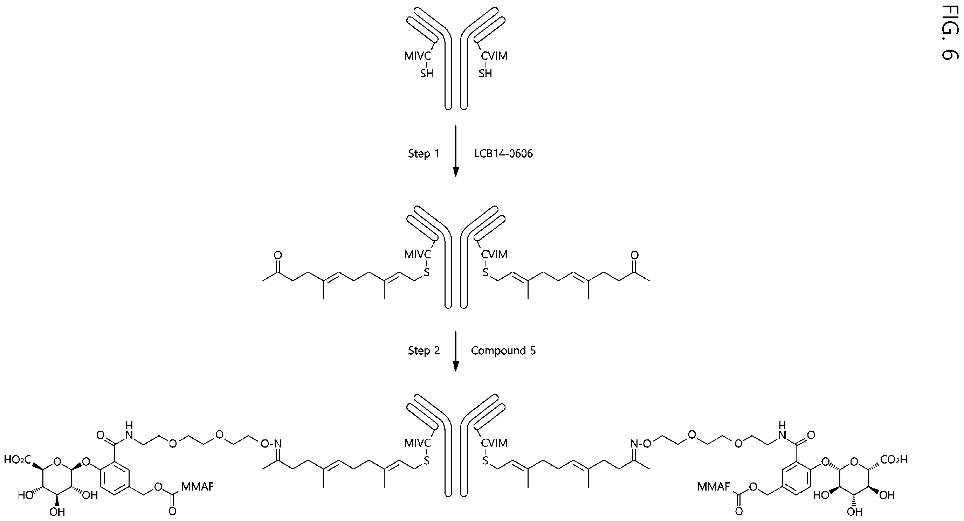

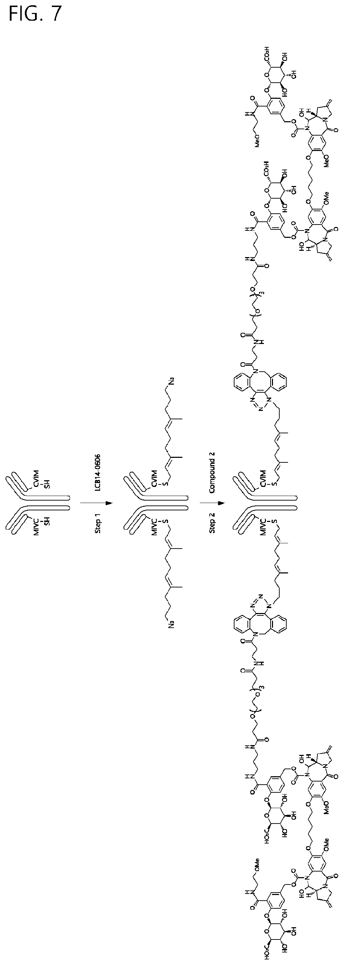

FIGS. 4 to 7 are schematic views illustrating a process of producing ADCs according to the present disclosure.

### [MODE OF DISCLOSURE]

Hereinafter, the present disclosure will be described in more detail with reference to the following examples and experimental examples.

These examples and experimental examples are intended to aid in understanding of the present disclosure and are not intended to limit the scope of the present disclosure.

### <Example 1> Production of Antibody Specific to Claudin 18 Isoform 2 (CLDN18.2)

An antibody that specifically binds to CLDN18.2 was produced by the method described in International Patent Application No. PCT/CN2020/118650. In addition, the amino acid sequences of the antibody that specifically binds to CLDN18.2 are shown in Table 1 below.

**[Table 1]**

| PR002726-heavy chain | | SEQ ID NO: |
|---|---|---|
| FR1 | EVQLLESGGGLVQPGGSLRLSCAAS | 1 |
| CDR 1 | GFTFSSFVMS | 2 |
| FR2 | WVRQAPGKGLEWVS | 3 |
| CDR 2 | TISGSGRSTYYADSVKG | 4 |
| FR3 | RFTISRDNSKNTLHLQMNSLRAEDTAVYYCAK | 5 |
| CDR 3 | DAAAAGTKFDY | 6 |
| FR4 | WGQGTLVTVSS | 7 |
| V_{H} | EVQLLESGGGLVQPGGSLRLSCAASGFTFSSFVMSWVRQAPGKGLEWVSTISG SGRSTYYADSVKGRFTISRDNSKNTLHLQMNSLRAEDTAVYYCAKDAAAAGTKF DYWGQGTLVTVSS | 15 |
| HC | EVQLLESGGGLVQPGGSLRLSCAASGFTFSSFVMSWVRQAPGKGLEWVSTISG SGRSTYYADSVKGRFTISRDNSKNTLHLQMNSLRAEDTAVYYCAKDAAAAGTKF DYWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKV DKKVEPKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWLNGKEY KCKVSNKALPAPIEKTISKAKGQPREPQVYTLPPSREEMTKNQVSLTCLVKGFYP SDIAVEWESNGQPENNYKTTPPVLDSDGSFFLYSKLTVDKSRWQQGNVFSCSV MHEALHNHYTQKSLSLSPGK | 17 |

| PR002726-light chain | | SEQ ID NO: |
|---|---|---|
| FR1 | EIVLTQSPATLSLSPGERATLSC | 8 |
| CDR 1 | RASQSVSSYLA | 9 |
| FR2 | WYQQKPGQAPRLLIY | 10 |
| CDR 2 | DASNRAT | 11 |
| FR3 | GIPARFSGSGSGTDFTLTISSLEPEDFAVYYC | 12 |
| CDR 3 | QQRSNWPLT | 13 |
| FR4 | FGGGTKVEIK | 14 |
| V_{L} | EIVLTQSPATLSLSPGERATLSCRASQSVSSYLAWYQQKPGQAPRLLIYDASNRA TGIPARFSGSGSGTDFTLTISSLEPEDFAVYYCQQRSNWPLTFGGGTKVEIK | 16 |
| LC | EIVLTQSPATLSLSPGERATLSCRASQSVSSYLAWYQQKPGQAPRLLIYDASNRA TGIPARFSGSGSGTDFTLTISSLEPEDFAVYYCQQRSNWPLTFGGGTKVEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC | 18 |

For ADC synthesis, an antibody clone PR301839 was constructed by fusing a CaaX peptide moiety (GGGGGGGCVIM; SEQ ID NO: 19) to the C-terminus of the light chain (SEQ ID NO: 18) of the antibody (PR002726) shown in Table 1, and an antibody was produced through transient expression based on CHO cells. The produced PR301839 antibody was used for ADC synthesis.

**[Table 2]**

| Clone | FR1 | CDR1 | FR2 | CDR2 | FR3 | CDR3 | FR4 | V_{H}/V_{L} |
|---|---|---|---|---|---|---|---|---|
| PR301 839-V_{H} | EVQLL ESGG GLVQP GGSLR LSCAA S | GFTFS SFVMS | WVR QAP GKGL EWV S | TISG SGR STYY ADSV KG | RFTI SRD NSKN TLHL QMN SLRA EDTA VYYC AK | DAAA AGTK FDY | WGQ GTLV TVSS | EVQLLESGGGLVQPGGSLRLSCAA SGFTFSSFVMSWVRQAPGKGLEWV STISGSGRSTYYADSVKGRFTISRD NSKNTLHLQMNSLRAEDTAVYYCAK DAAAAGTKFDYWGQGTLVTVSS |
| SEQ ID NO: | 1 | 2 | 3 | 4 | 5 | 6 | 7 | 15 |
| PR301 839-V_{L} | EIVLTQ SPATL SLSPG ERATL SC | RASQS VSSYL A | WYQ QKP GQA PRLLI Y | DASN RAT | GIPA RFSG SGS GTDF TLTIS SLEP EDFA VYYC | QQR SNW PLT | FGG GTKV EIK | EIVLTQSPATLSLSPGERATLSCRAS QSVSSYLAWYQQKPGQAPRLLIYDA SNRATGIPARFSGSGSGTDFTLTISS LEPEDFAVYYCQQRSNWPLTFGGG TKVEIK |
| SEQ ID NO: | 8 | 9 | 10 | 11 | 12 | 13 | 20 | 21 |

### <Example 2> Preparation of Compounds 1 and 2

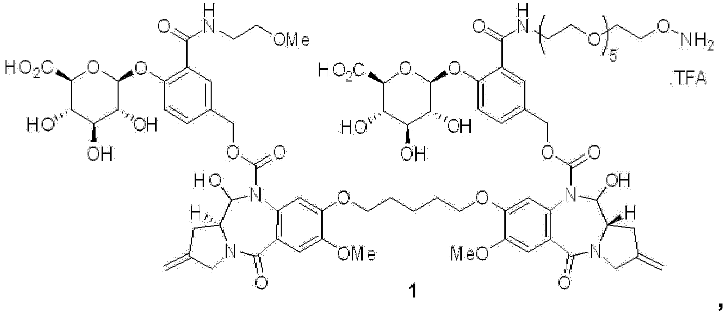

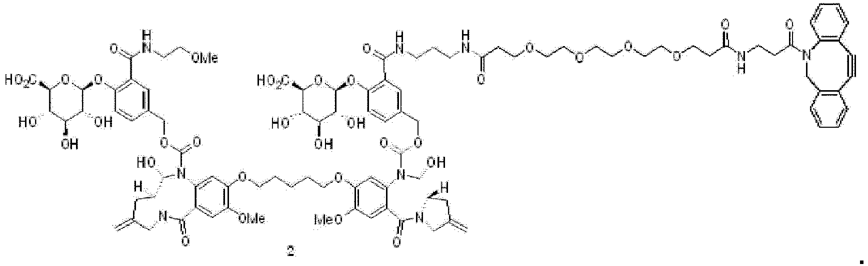

Compounds 1 and 2 were prepared using the method described in Korean Patent Publication No. 10-2018-0110645.

### <Example 3> Preparation of Compound 3

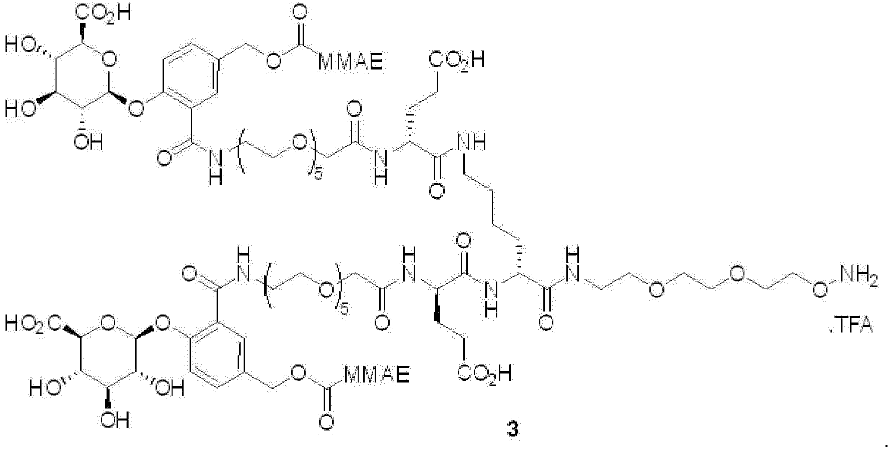

Compound 3 was prepared using the method described in International Patent Publication No. WO2017-089895.

The structure of MMAE in compound 3 is as follows: 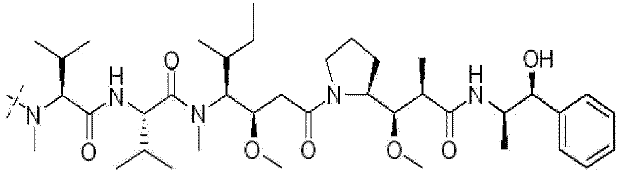 **MMAE**

### <Example 4> Preparation of Compounds 4 and 5

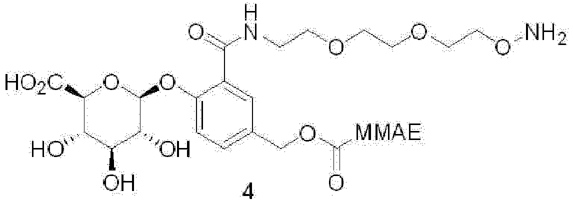

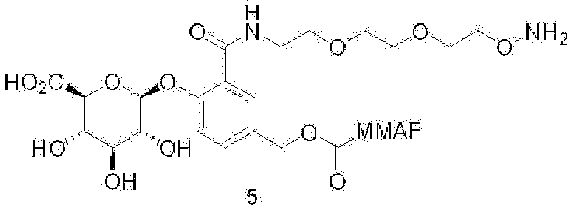

Compounds 4 and 5 were prepared using the method described in WO2017-089890.

The structure of MMAE in compound 5 is as follows:

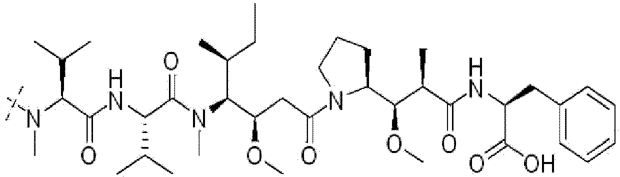

### <Example 5> Production of ADCs

ADCs were produced through the following two steps, and LCB14-0606 and LCB14-0512 used commonly were prepared using the method described in Korean Patent Publication No. 10-2014-0035393. The structural formulae of LCB14-0606 and LCB14-0512 are as follows:

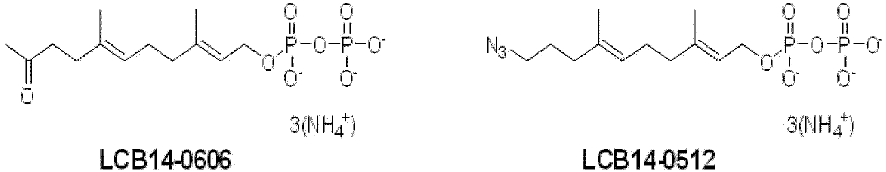

### Step 1: Production of Prenylated Antibodies

A prenylation reaction mixture of the antibodies produced according to Example 1 was prepared and a reaction was allowed to occur at 30 °C for 16 hours. The reaction mixture consisted of a 24 µM antibody, 400 nM FTase (Calbiochem #344145), and a buffer solution (50 mM Tris-HCl (pH 7.4), 5 mM MgCl₂, 10 µM ZnCl₂, and 0.5 mM DTT) containing 0.25 mM LCB14-0511 or LCB14-0606. After the reaction was completed, the prenylated antibody was desalted using a G25 Sepharose column (AKTA purifier, GE healthcare) equilibrated with PBS buffer solution.

### Step 2: Drug Conjugation Method

### <Conjugation by Oxime Bond Formation>

The mixture for oxime bond-formation reaction between the prenylated antibody and linker-drug was prepared by mixing 100 mM Na-acetate buffer solution pH 5.2, 10% DMSO, 24 µM of the prenylated antibody, and 200 µM linker-drug (in house, compounds 1, 2, 3, 4, 5, 7 and 8 of Examples 1 and 2), and stirred lightly at 30 °C. After a reaction was allowed to occur for 6 hours or 24 hours, an FPLC (AKTA purifier, GE healthcare) process was carried out to remove the excess of small-molecule compounds used, and the protein fraction was collected and concentrated.

### <Conjugation by Copper-Free Click Reaction>

For the production of ADCs using the copper-free click coupling reaction between the prenylated antibody and linker-drug, PBS buffer solution (pH 7,4), 1% DMSO, 10 µM of the prenylated antibody, and 100 µM linker-drug (in house, compound 2 of Example 1) were mixed to prepare a reaction mixture, and after reaction at 25 °C for 6 hours, the excess of small-molecule compounds used were removed through FPLC (AKTA purifier, GE healthcare) or a G25 Sepharose column process, and the protein fraction was collected and concentrated.

**[Table 3]**

| List for ADC production | | |
|---|---|---|
| **ADCs** | **Antibody** | **linker-toxin** |
| ADC1 | PR301839 | Compound 1 |
| ADC2 | | Compound 2 |
| ADC3 | | Compound 3 |
| ADC4 | | Compound 4 |
| ADC5 | | Compound 5 |

### <Experimental Example 1> Evaluation of Ability of Antibody to Bind to Cancer Cells

The ability of the anti-CLDN18.2 antibody (PR002726) and the antibody (PR301839), which was used in the production of the ADC in which a CaaX peptide is fused to the C-terminus of the light chain, to bind to cancer cells was examined through a cell binding experiment using a flow cytometer (FACS).

As cancer cells, gastric cancer cell lines SNU-601 and SNU-620, and a pancreatic cancer cell line PATU-8988s, which are known to express CLDN18.2, were used, and a group treated with human IgG and a group treated with only secondary antibodies were used as controls.

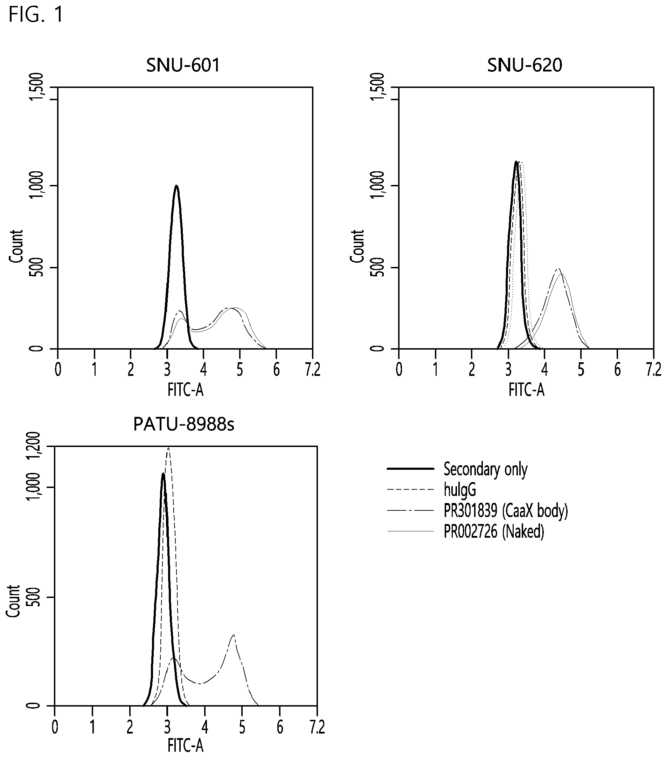

Through the experimental results, it was confirmed that PR002726 and PR301839 were strongly bound 22.95 times and 17.5 times, respectively, in SNU-601 compared to the IgG control. It was confirmed that PR002726 and PR301839 were strongly bound 15.09 times and 11.21 times in SNU-620 compared to the controls. It was confirmed that PR301839 was strongly bound 16.21 times in PATU-8988s compared to the controls. Taken together, it was confirmed that the two antibodies PR002726 and PR301839 can bind to cancer cells expressing CLDN18.2. (see FIG. 1)

### <Experimental Example 2> In Vitro Cytotoxicity Evaluation

The cell proliferation inhibitory activity of drugs shown in Table 4 and the ADCs was measured. Gastric cancer cell lines NUGC-4, SNU-601, and SNU-602, and a pancreatic cancer cell line PATU-8988s, which are commercially available as cancer cell lines, were used, and normal cell lines HaCaT, Fa2N-4, HK-2, and HS738.st/Int were used. MMAE and SG2057 were used as drugs, and the ADCs shown in Table 3 were used as ADCs. As for test groups treated with each cancer cell line for 144 hours, cells were seeded into a 96-well plate at the rate of 2,500 cells/well to 10,000 cells/well and cultured for 24 hours, and then each cancer cell line was treated with each drug and each ADC at a concentration of 0.256 pM to 100 nM (5-fold serial diltuion), and each normal cell line was treated with each drug and each ADC at a concentration of 2.56 pM to 1,000 nM (5-fold serial dilution). The number of cells survived after 144 hours was quantified using a sulforhodamine B (SRB) dye, and the cell line Hs738.st/Int was quantified using Cell titer glo.

**[Table 4]**

| **Test samples** | **IC50 (nM)** | | | | | | | |
|---|---|---|---|---|---|---|---|---|
| | **NUGC-4** | **SNU-601** | **SNU-620** | **PATU-8988s** | **HaCaT** | **Fa2N-4** | **HK-2** | **Hs738.st/Int** |
| MMAE | 0.29 | 0.51 | 0.71 | 0.145 | 0.14 | 3.0 | 21.9 | N/D |
| SG2057 | 0.03 | 0.003 | 0.097 | - | - | - | - | - |
| ADC1 | 17.11 | 0.028 | 20.2 | - | - | - | - | - |
| ADC2 | 9.73 | 0.022 | 20.55 | - | - | - | - | - |
| ADC3 | 6.72 | 0.81 | 18.14 | 2.073 | 32.7 | 365.7 | >1,000 | >1,000 |
| ADC4 | - | 4.122 | - | 9.297 | 420.7 | - | - | - |
| ADC5 | - | 2.278 | - | 27.26 | 562.5 | - | - | - |

In a cytotoxicity test in which the reaction was allowed to occur for the same time period, it was confirmed that, in the NUGC-4 cell line, an auristatin-based antibody-drug conjugates (ADC3) exhibited equivalent or higher cytotoxicity than pyrrolobenzodiazepine-based antibody-drug conjugates (ADC1 and ADC2). Also, in the cytotoxicity test in which the reaction was allowed to occur for the same time period, it was confirmed that, in the SNU-601 cell line exhibiting strong binding to the anti-CLDN18.2 antibody and the strongest cytotoxicity, pyrrolobenzodiazepine-based antibody-drug conjugates (ADC1 and ADC2) exhibited strong cytotoxicity compared to auristatin-based antibody-drug conjugates (ADC3, ADC4, and ADC5). In the SNU-620 cell line to which the antibody was shown to bind strongly, it was confirmed that there was no significant difference in anticancer efficacy between the two types of antibody-drug conjugates. As a result of comparing the cytotoxicity of three types of auristatin-based antibody-drug conjugates in the pancreatic cancer cell line PATU-8988s, it was confirmed that all the three types of ADCs showed strong cytotoxicity.

In the normal cell lines HaCaT, Fa2N-4, HK-2, and Hs738.st/Int, ADC1 to ADC5 showed no cytotoxicity within the effective dose range, and exhibited remarkably reduced cytotoxicity compared to when a toxin was administered alone or when administered to cancer cell lines, through which it can be seen that the ADC according to the present disclosure exhibits cytotoxicity in a cancer cell-specific manner.

### <Experimental Example 3> In Vivo Efficacy Evaluation

A gastric cancer cell line SNU-601 was cultured, and then 100 µl of PBS containing 20,000 cells was mixed with 100 µl of Matrigel, and the mixture was engrafted into Balb/c-nude mice. When the size of a tumor reached 150 mm³ to 200 mm^{3,} each of the antibody-drug conjugates was intravenously administered as shown in Tables 5 and 6 below.

**[Table 5]**

| Comparison in *in vivo* efficacy between ADC samples (first) | | | |
|---|---|---|---|
| Test sample | Population | Dose (mg/kg) | Dose interval |
| PBS | 5 | - | qdx1 |
| ADC1 | 5 | 0.2 | |
| | 5 | 0.4 | |
| ADC3 | 5 | 0.5 | |
| | 5 | 1 | |
| | 5 | 2 | |

**[Table 6]**

| Comparison in *in vivo* efficacy between ADC samples (second) | | | |
|---|---|---|---|
| Test sample | Population | Dose (mg/kg) | Dose interval |
| PBS | 5 | - | qdx1 |
| ADC3 | 5 | 1 | |
| ADC4 | 5 | 2 | |
| | 5 | 4 | |
| ADC5 | 5 | 2 | |
| | 5 | 4 | |

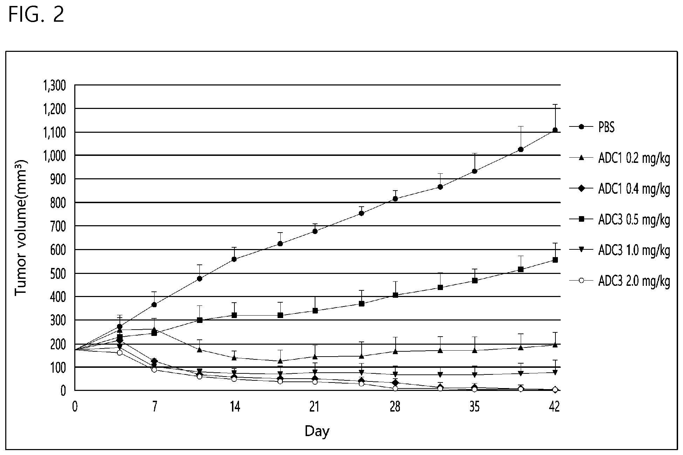

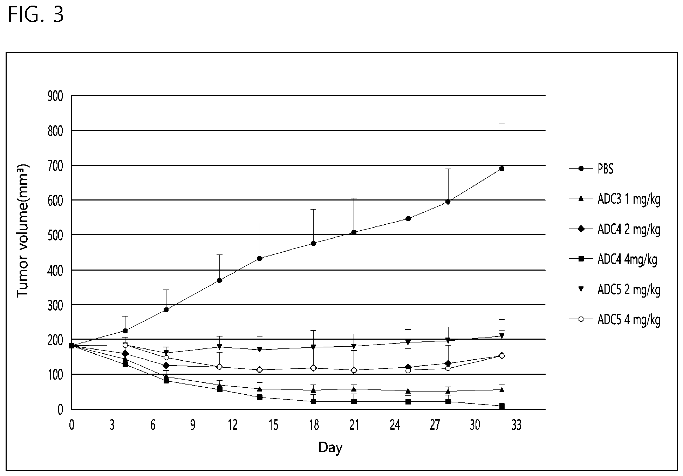

As a result of the first experiment, it was confirmed that the pyrrolobenzodiazepine-based antibody-drug conjugate ADC1 exhibited tumor growth inhibitory efficacy at both 0.2 mg/kg and 0.4 mg/kg compared to the control. It was confirmed that the auristatin-based antibody-drug conjugates ADC3 exhibited tumor growth inhibitory efficacy at all of 0.5 mg/kg, 1.0 mg/kg, and 2.0 mg/kg in a dose-dependent manner. The experimental results (first) of comparison in *in vivo* efficacy between ADC samples are the same as shown in FIG. 2. As a result of the second experiment, it was confirmed that the ADC3 1 mg/kg group exhibited tumor growth inhibitory efficacy similar to the first experimental results. It was confirmed that ADC4 and ADC5 exhibited tumor growth inhibitory efficacy at all the doses in a dose-dependent manner. The experimental results (second) of comparison in *in vivo* efficacy between ADC samples are the same as shown in FIG. 3.

### Cited References

Duncan, A. R., J. M. Woof, L. J. Partridge, D. R. Burton, and G. Winter. 1988. Localisation of the binding site for the human high-affinity Fc receptoron IgG. Nature 332:563-564.

Lund, J., G. Winter, P. T. Jones, J. D. Pound, T. Tanaka, M. R. Walker, P. J. Artymiuk, Y. Arata, D. R. Burton, R. Jefferis, and J. M. Woof. 1991. Human FcyRI and FcγRII interact with distinct but overlapping sites on human IgG. J. Immunol. 147:2657-2662.

Sarmay, G., J. Lund, Z. Rozsnayay, J. Gergeley, and R. Jefferis. 1992. Mapping and comparison of the interaction sites on the Fc region of IgG responsible for triggering antibody dependent cellular cytotoxicity (ADCC) through different types of human Fc receptor. Mol. Immunol. 29:633-639.

### [INDUSTRIAL APPLICABILITY]

The present disclosure can be effectively used in the pharmaceutical field through a novel antibody-drug conjugate targeting claudin 18 isoform 2, an active metabolite thereof, a method of producing same, use thereof, and a pharmaceutical composition including the same.

## Claims

1. A conjugate represented by General Formula I or a pharmaceutically acceptable salt or solvate thereof:
[General Formula I] Ab-[LINKER-(B)_{I}]ₘ
wherein,
Ab is an anti-Claudin 18 isoform 2 (CLDN18.2) antibody or antigen-binding fragment thereof comprising a heavy chain variable region and a light chain variable region, wherein,
the heavy chain variable region comprises a heavy chain CDR1 consisting of the amino acid sequence represented by SEQ ID NO: 2, a heavy chain CDR2 consisting of the amino acid sequence represented by SEQ ID NO: 4, and a heavy chain CDR3 consisting of the amino acid sequence represented by SEQ ID NO: 6, and
the light chain variable region comprises a light chain CDR1 consisting of the amino acid sequence represented by SEQ ID NO: 9, a light chain CDR2 consisting of the amino acid sequence represented by SEQ ID NO: 11, and a light chain CDR3 consisting of the amino acid sequence represented by SEQ ID NO: 13,
LINKER is a linker,
B is an active agent, and
l and m are each independently an integer selected from 1 to 20,
wherein, when l is an integer of 2 or more, at least two of B are identical to or different from each other.

2. The conjugate or the pharmaceutically acceptable salt or solvate thereof of claim 1, wherein the heavy chain variable region comprises a heavy chain FR of a human antibody, and a gene encoding the heavy chain FR is derived from germline V gene IGHV3-23.

3. The conjugate or the pharmaceutically acceptable salt or solvate thereof of claim 2, wherein the heavy chain FR comprises a heavy chain FR1 consisting of the amino acid sequence represented by SEQ ID NO: 1, a heavy chain FR2 consisting of the amino acid sequence represented by SEQ ID NO: 3, a heavy chain FR3 consisting of the amino acid sequence represented by SEQ ID NO: 5, and a heavy chain FR4 consisting of the amino acid sequence represented by SEQ ID NO: 7.

4. The conjugate or the pharmaceutically acceptable salt or solvate thereof of claim 1, wherein the light chain variable region comprises a light chain FR of a human antibody, and a gene encoding the light chain FR is derived from germline V gene IGKV3-11 or IGKV3-15.

5. The conjugate or the pharmaceutically acceptable salt or solvate thereof of claim 4, wherein the light chain FR comprises:
a light chain FR1 consisting of the amino acid sequence represented by SEQ ID NO: 8;
a light chain FR2 consisting of the amino acid sequence represented by SEQ ID NO: 10;
a light chain FR3 consisting of the amino acid sequence represented by SEQ ID NO: 12; and
a light chain FR4 consisting of the amino acid sequence represented by SEQ ID NO: 14, or a light chain FR4 consisting of the amino acid sequence represented by SEQ ID NO: 20.

6. The conjugate or the pharmaceutically acceptable salt or solvate thereof of claim 1, wherein the antibody or antigen-binding fragment thereof that specifically binds to CLDN18.2 comprises:
a heavy chain variable region consisting of the amino acid sequence represented by SEQ ID NO: 15 or an amino acid sequence having at least 80% sequence identity to the amino acid sequence of SEQ ID NO: 15; and
a light chain variable region consisting of the amino acid sequence represented by SEQ ID NO: 16 or an amino acid sequence having at least 80% sequence identity to the amino acid sequence of SEQ ID NO: 16.

7. The conjugate or the pharmaceutically acceptable salt or solvate thereof of claim 1, wherein the antibody or antigen-binding fragment thereof is any one selected from a monoclonal antibody, a domain antibody (dAb), a single chain antibody (scAb), a Fab fragment, a Fab' fragment, a F(ab')2 fragment, an scFab fragment, an Fv fragment, a dsFv fragment, a single chain variable fragment (scFv), an scFv-Fc fragment, a single domain heavy chain antibody, a single domain light chain antibody, a variant antibody, a multimeric antibody, a minibody, a diabody, a bispecific antibody, and a multispecific antibody.

8. The conjugate or the pharmaceutically acceptable salt or solvate thereof of claim 1, wherein the linker between the antibody and an active agent is cleavable.

9. The conjugate or the pharmaceutically acceptable salt or solvate thereof of claim 1, wherein the conjugate has the structure of General Formula IIa: 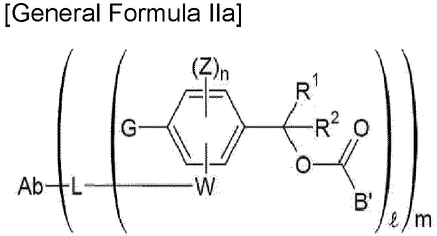 wherein,
Ab is an anti-Claudin 18 isoform 2 (CLDN18.2) antibody or antigen-binding fragment thereof comprising a heavy chain variable region and a light chain variable region;
each B' is independently an active agent, wherein the active agents are identical to or different from each other;
G and G' are each independently a glucuronic acid moiety or 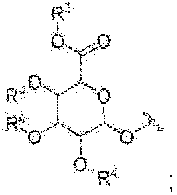
R³ is hydrogen or a carboxyl-protecting group, and each R⁴ is independently hydrogen or a hydroxyl-protecting group;
R¹ and R² are each independently hydrogen, C₁₋₈ alkyl, or C₃₋₈ cycloalkyl;
each W is independently -C(O)-, -C(O)NR'-, -C(O)O-, -SO₂NR'-, -P(O)R"NR', - SONR'-, or -PO₂NR'-, wherein C, S, or P is directly bonded to a phenyl ring, NR' is bonded to L, and R' and R" are each independently hydrogen, C₁₋₈alkyl, C₃₋₈cycloalkyl, C₁₋₈alkoxy, C₁₋₈ alkylthio, mono- or di-C₁₋₈ alkylamino, C₃₋₂₀ heteroaryl, or C₆₋₂₀ aryl;
each Z is independently hydrogen, C₁₋₈ alkyl, halogen, cyano, or nitro;
n is an integer from 0 to 3 wherein, when n is an integer of 2 or more, at least two of Z are identical to or different from each other;
each L is independently any one selected from A) or B) below:
A) C₁₋₅₀ alkylene or 1-50 atom heteroalkylene, satisfying at least one of the following:
(i) L comprises one or more unsaturated bonds;
(ii) two atoms in L are substituted with a bivalent substituent, which completes a heteroarylene;
(iii) L is a 1-50 atom heteroalkylene; or
(iv) the alkylene is further substituted with at least one C₁₋₂₀ alkyl; or
B) at least one isoprenyl derivative unit of General Formula III which is recognizable by an isoprenoid transferase: 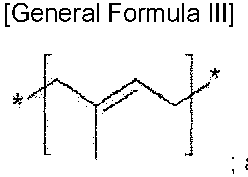 and
m is an integer selected from 1 to 20.

10. A conjugate represented by General Formula IIa or a pharmaceutically acceptable salt or solvate thereof: 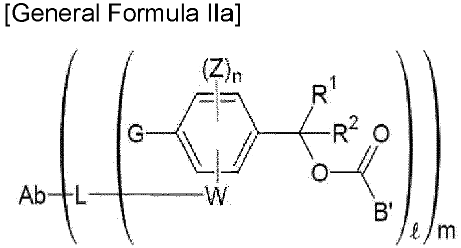 wherein,
Ab is an antibody or antigen-binding fragment thereof that specifically binds to claudin 18 isoform 2 (CLDN18.2);
each G is independently a glucuronic acid moiety or 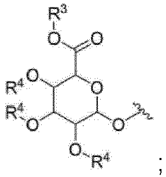
R³ is hydrogen or a carboxyl-protecting group, and each R⁴ is independently hydrogen or a hydroxyl-protecting group;
R¹ and R² are each independently hydrogen, C₁₋₈ alkyl, or C₃₋₈ cycloalkyl;
each W is independently -C(O)-, -C(O)NR'-, -C(O)O-, -SO₂NR'-, -P(O)R"NR', - SONR'-, or -PO₂NR'-, wherein C, S, or P is directly bonded to a phenyl ring, NR' is bonded to L, and R' and R" are each independently hydrogen, C₁₋₈alkyl, C₃₋₈cycloalkyl, C₁₋₈alkoxy, C₁₋₈ alkylthio, mono- or di-C₁₋₈ alkylamino, C₃₋₂₀ heteroaryl, or C₆₋₂₀ aryl;
each Z is independently hydrogen, C₁₋₈ alkyl, halogen, cyano, or nitro;
n is an integer from 0 to 3; and
each L is independently any one selected from A) or B) below:
A) C₁₋₅₀ alkylene or 1-50 atom heteroalkylene, satisfying at least one of the following:
(i) L comprises one or more unsaturated bonds;
(ii) two atoms in L are substituted with a bivalent substituent, which completes a heteroarylene;
(iii) L is a 1- 50 atom heteroalkylene; or
(iv) the alkylene is substituted with at least one C₁₋₂₀ alkyl; or
B) at least one isoprenyl derivative unit of General Formula III which is recognizable by an isoprenoid transferase: 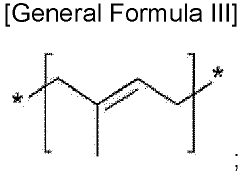
B' is an active agent;
Ab is bonded to a site indicated as a wave symbol; and
l and m are each independently an integer selected from 1 to 20,
wherein, when l is 2 or more, the active agents are identical to or different from each other.

11. The conjugate or the pharmaceutically acceptable salt or solvate thereof of claim 9 or 10, wherein
each G is independently 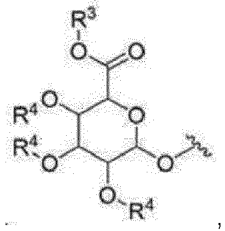
R³ is hydrogen or a carboxyl-protecting group, and
each R⁴ is independently hydrogen or a hydroxyl-protecting group.

12. The conjugate or the pharmaceutically acceptable salt or solvate thereof of claim 9 or 10, wherein:
R¹ and R² are each hydrogen;
n is 0; and
each W is independently -C(O)NR'-, C is directly bonded to a phenyl ring, and R' is hydrogen, C₁₋₈ alkyl, C₃₋₈ cycloalkyl, C₁₋₈ alkoxy, C₁₋₈ alkylthio, mono- or di-C₁₋₈ alkylamino, C₃₋₂₀ heteroaryl, or C₆₋₂₀ aryl, wherein NR' is bonded to L.

13. The conjugate or the pharmaceutically acceptable salt or solvate thereof of claim 9 or 10, wherein
L is a 1-5 atom heteroalkylene comprising nitrogen,
L comprises two or more atoms of a hydrophilic amino acid, and
the nitrogen forms a peptide bond with a carbonyl of the hydrophilic amino acid.

14. The conjugate or the pharmaceutically acceptable salt or solvate thereof of claim 9 or 10, wherein
L is covalently bonded to the antibody by a thioether bond,
wherein the thioether bond comprises a sulfur atom of a cysteine of the antibody.

15. The conjugate or the pharmaceutically acceptable salt or solvate thereof of claim 14, wherein
the antibody comprises an amino acid motif recognizable by an isoprenoid transferase at the C-terminus of the antibody, and
the thioether bond comprises a sulfur atom of a cysteine of the amino acid motif.

16. The conjugate or the pharmaceutically acceptable salt or solvate thereof of claim 15, wherein:
the amino acid motif has a CYYX sequence, wherein C is cysteine, Y is an aliphatic amino acid, and X is any one selected from glutamine, glutamate, serine, cysteine, methionine, alanine, and leucine; and
the thioether bond comprises a sulfur atom of a cysteine of the amino acid motif.

17. The conjugate or the pharmaceutically acceptable salt or solvate thereof of claim 15, wherein
the amino acid motif has a CYYX sequence, wherein Y is any one selected from alanine, isoleucine, leucine, methionine, and valine; or
a CVIM or CVLL sequence.

18. The conjugate or the pharmaceutically acceptable salt or solvate thereof of claim 15, wherein at least one of 1 to 20 amino acids preceding the amino acid motif is glycine.

19. The conjugate or the pharmaceutically acceptable salt or solvate thereof of claim 9 or 10, wherein L comprises the amino acid sequence of GGGGGGGCVIM at the C-terminus.

20. The conjugate or the pharmaceutically acceptable salt or solvate thereof of claim 9 or 10, wherein
L is 3 to 50 heteroalkylene containing an oxime,
the oxygen atom of the oxime is on the side of L linked to W,
the carbon atom of the oxime is on the side of L linked to Ab, or
the carbon atom of the oxime is on the side of L linked to W, and
the oxygen atom of the oxime is on the side of L linked to Ab.

21. The conjugate or the pharmaceutically acceptable salt or solvate thereof of claim 9 or 10, wherein L comprises an oxime, and at least one isoprenyl unit covalently bonds the oxime to Ab.

22. The conjugate or the pharmaceutically acceptable salt or solvate thereof of claim 9 or 10, wherein L further comprises a second unit represented by General Formula VIII or General Formula IX:
[General Formula VIII] -(CH₂)ᵣ(V(CH₂)ₚ)_{q}-
[General Formula IX] -(CH₂CH₂X)_{w}-
V is a single bond, -O-, -S-, -NR²¹-, -C(O)NR²²-, -NR²³C(O)-, -NR²⁴SO₂-, or - SO₂NR²⁵-;
X is -O-, C₁₋₈ alkylene, or -NR²¹-;
R²¹ to R²⁵ are each independently hydrogen, C₁₋₆ alkyl, C₁₋₆ alkyl C₆₋₂₀ aryl, or C₁₋₆ alkyl C₃₋₂₀ heteroaryl;
r is an integer from 0 to 10;
p is an integer from 0 to 10;
q is an integer from 1 to 20; and
w is an integer from 1 to 20.

23. The conjugate or the pharmaceutically acceptable salt or solvate thereof of claim 22, wherein:
q is an integer from 1 to 10;
r and p are each 1 or 2; and
V is -O-.

24. The conjugate or the pharmaceutically acceptable salt or solvate thereof of claim 22, wherein:
X is -O-; and
w is an integer from 1 to 10.

25. The conjugate or the pharmaceutically acceptable salt or solvate thereof of claim 21, wherein L comprises at least one polyethylene glycol unit represented by

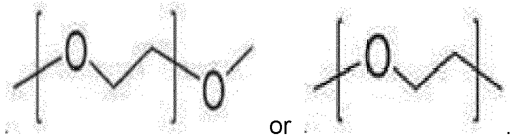

26. The conjugate or the pharmaceutically acceptable salt or solvate thereof of claim 21, wherein
L comprises an oxime, and
at least one polyethylene glycol unit covalently bonds the oxime to an active agent.

27. The conjugate or the pharmaceutically acceptable salt or solvate thereof of claim 9 or 10, wherein L further comprises a third unit formed by a reaction between an alkyne and an azide or between an aldehyde or ketone group and hydrazine or hydroxylamine.

28. The conjugate or the pharmaceutically acceptable salt or solvate thereof of claim 9 or 10, wherein L further comprises L or a third unit represented by General Formula IVa, IVb, IVc, IVd or IVe below: 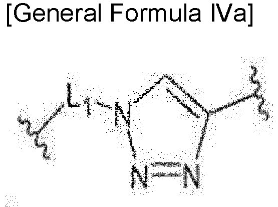 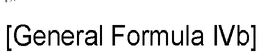 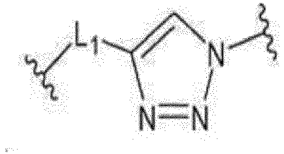 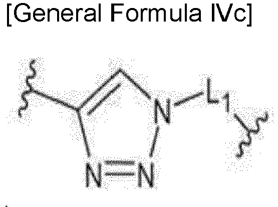 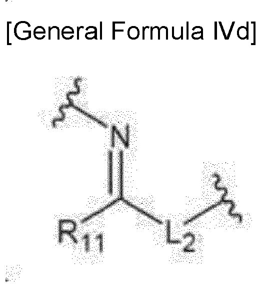 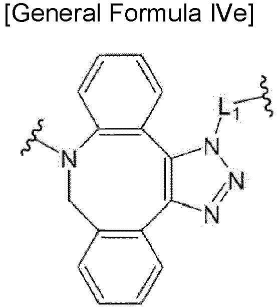 wherein,
L₁ and L₂ are each independently a single bond or C₁₋₃₀ alkylene; and
R₁₁ is hydrogen or C₁₋₁₀ alkyl.

29. The conjugate or the pharmaceutically acceptable salt or solvate thereof of claim 28, wherein L₁ and L₂ are each independently a single bond, C₁₁ alkylene, or C₁₂ alkylene.

30. The conjugate or the pharmaceutically acceptable salt or solvate thereof of claim 9 or 10, wherein the isoprenoid transferase is farnesyl protein transferase (FTase) or geranylgeranyl transferase (GGTase).

31. The conjugate or the pharmaceutically acceptable salt or solvate thereof of claim 9 or 10, wherein
L comprises one or more branched linkers covalently bonded to Ab, wherein:
i) each branched linker comprises a fifth unit covalently bonded to Ab by a primary linker (PL);
ii) each branched linker comprises a first branch (B1) in which a first active agent is covalently bonded to the fifth unit by a secondary linker (SL) and a cleavage group (CG); and
iii) each branched linker comprises: a) a second branch (B2) in which a second active agent is covalently bonded to the fifth unit by a secondary linker (SL) and the cleavage group (CG); or b) a second branch (B2) in which a polyethylene glycol moiety is covalently bonded to the fifth unit, and
each cleavage group is hydrolyzed to release an active agent from the conjugate.

32. The conjugate or the pharmaceutically acceptable salt or solvate thereof of claim 31, wherein
the fifth unit is 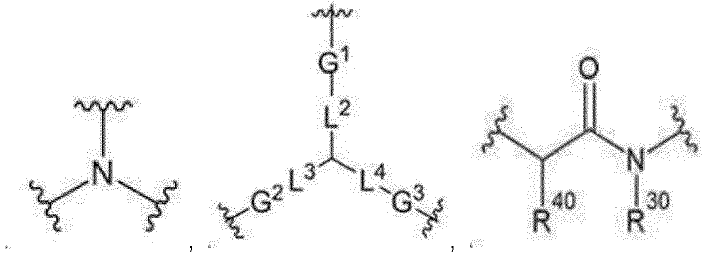 , or 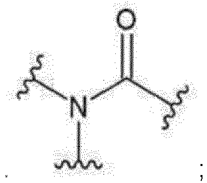
wherein L², L³, and L⁴ are each independently a bond or -CₙH₂ₙ-, wherein n is an integer from 1 to 30;
G¹, G², and G³ are each independently a bond, 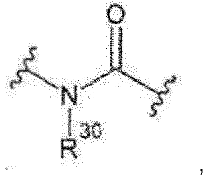 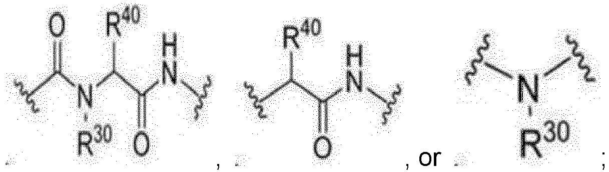
R³⁰ is hydrogen or C₁₋₃₀ alkyl;
R⁴⁰ is hydrogen or L⁵-COOR₆; and
L⁵ is a bond or -C_{n'}H_{2n'}-, wherein n' is an integer from 1 to 10; and
R₆ is hydrogen or C₁₋₃₀ alkyl.

33. The conjugate or the pharmaceutically acceptable salt or solvate thereof of claim 31, wherein the cleavage group is cleavable in a target cell, or is capable of releasing one or more active agents.

34. The conjugate or the pharmaceutically acceptable salt or solvate thereof of claim 33, wherein:
the conjugate comprises Ab, and
1, 2, 3, 4 or more branched linkers covalently bonded to Ab; and
each branched linker is bonded to one or more active agents,
wherein the active agents are identical to or different from each other.

35. The conjugate or the pharmaceutically acceptable salt or solvate thereof of claim 32, wherein each branched linker comprises a fifth unit, each active agent is bonded to the fifth unit via a secondary linker, and the fifth unit is bonded to the antibody via a primary linker.

36. The conjugate or the pharmaceutically acceptable salt or solvate thereof of claim 35, wherein:
the fifth unit is an amide, and the primary linker comprises: a carbonyl of the amide; or
a lysine unit.

37. The conjugate or the pharmaceutically acceptable salt or solvate thereof of claim 33, wherein the conjugate or the pharmaceutically acceptable salt or solvate thereof comprises Structural Formula below: 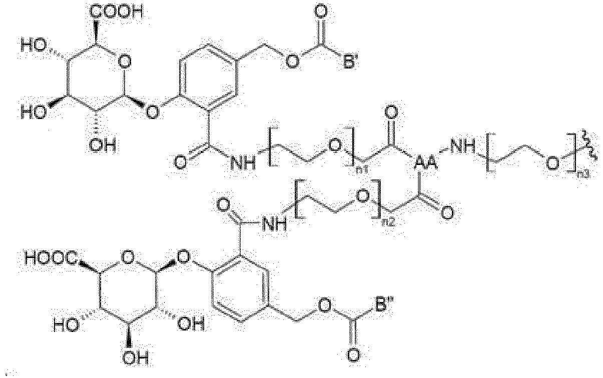
wherein B' and B" are each active agents which are identical to or different from each other;
n1 to n3 each independently are an integer from 0 to 30; and
AA is an amino acid group.

38. A conjugate represented by Structural Formula below and comprising a [LINKER-B] structure, or a pharmaceutically acceptable salt or solvate thereof: 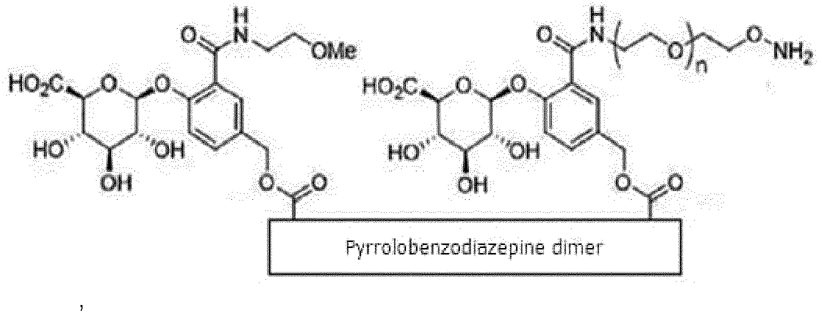 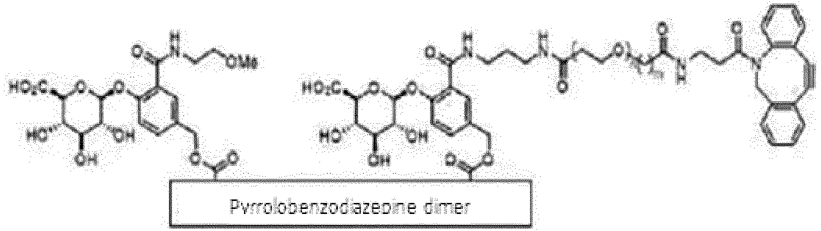 , or 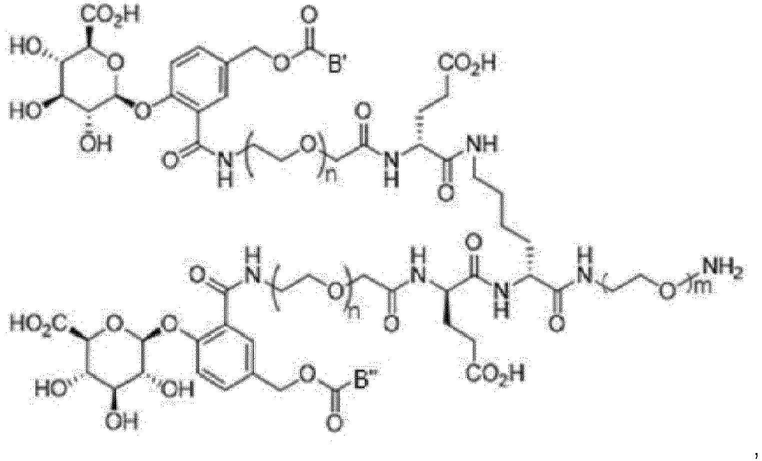
wherein B' and B" are active agents which are identical to or different from each other; and
m and n each independently are an integer from 0 to 30.

39. The conjugate or the pharmaceutically acceptable salt or solvate thereof of claim 9 or 10, wherein the active agent is a chemotherapeutic agent or a toxin.

40. The conjugate or the pharmaceutically acceptable salt or solvate thereof of claim 9 or 10, wherein the active agent is an immunomodulatory compound, an anticancer agent, an anti-viral agent, an anti-bacterial agent, an anti-fungal agent, an anti-parasitic agent, or a combination thereof.

41. The conjugate or the pharmaceutically acceptable salt or solvate thereof of claim 9 or 10, wherein the active agent is any one selected from the following:
(a) erlotinib, bortezomib, fulvestrant, sutent, letrozole, imatinib mesylate, PTK787/ZK 222584, oxaliplatin, 5-fluorouracil, leucovorin, rapamycin, lapatinib, lonafarnib, sorafenib, gefitinib, AG1478, AG1571, thiotepa, cyclophosphamide, busulfan, improsulfan, piposulfan, benzodopa, carboquone, meturedopa, uredopa, ethylenimine, altretamine, triethylenemelamine, triethylenephosphoramide, triethiylenethiophosphoramide, trimethylolomelamine, bullatacin, bullatacinone, camptothecin, topotecan, bryostatin, callystatin, CC-1065, adozelesin, carzelesin, bizelesin, cryptophycin 1, cryptophycin 8, dolastatin, duocarmycin, KW-2189, CB1-TM1, eleutherobin, pancratistatin, sarcodictyin, spongistatin, chlorambucil, chlornaphazine, cholophosphamide, estramustine, ifosfamide, mechlorethamine, melphalan, novembichin, phenesterine, prednimustine, trofosfamide, uracil mustard, carmustine, chlorozotocin, fotemustine, lomustine, nimustine, ranimnustine, calicheamicin, calicheamicin gamma 1, calicheamicin omega 1, dynemicin, dynemicin A, clodronate, esperamicin, neocarzinostatin chromophore, aclacinomysins, actinomycin, antrmycin, azaserine, bleomycins, cactinomycin, carabicin, carninomycin, carzinophilin, chromomycins, dactinomycin, daunorubicin, detorubucin, 6-diazo-5-oxo-L-norleucine, doxorubicin, morpholino-doxorubicin, cyanomorpholino-doxorubicin, 2-pyrrolino-doxorubicin, liposomal doxorubicin, deoxydoxorubicin, epirubicin, esorubicin, marcellomycin, mitomycin C, mycophenolic acid, nogalamycin, olivomycins, peplomycin, potfiromycin, puromycin, quelamycin, rodorubicin, streptomigrin, streptozocin, tubercidin, ubenimex, zinostatin, zorubicin, 5-fluorouracil, denopterin, methotrexate, pteropterin, trimetrexate, fludarabine, 6-mercaptopurine, thiamiprine, thiguanine, ancitabine, azacitidine, 6-azauridine, carmofur, cytarabine, dideoxyuridine, doxifluridine, enocitabine, floxuridine, calusterone, dromostanolone, propionate, epitiostanol, mepitiostane, testolactone, aminoglutethimide, mitotane, trilostane, folinic acid, aceglatone, aldophosphamide glycoside, aminolevulinic acid, eniluracil, amsacrine, bestrabucil, bisantrene, edatraxate, defofamine, demecolcine, diaziquone, elfornithine, elliptinium acetate, etoglucid, gallium nitrate, hydroxyurea, lentinan, lonidainine, maytansine, ansamitocins, mitoguazone, mitoxantrone, mopidanmol, nitraerine, pentostatin, phenamet, pirarubicin, losoxantrone, 2-ethylhydrazide, procarbazine, polysaccharide-k, razoxane, rhizoxin, sizofiran, spirogermanium, tenuazonic acid, triaziquone, 2,2',2"-trichlorotriethylamine, T-2 toxin, verracurin A, roridin A, anguidine, urethane, vindesine, dacarbazine, mannomustine, mitobronitol, mitolactol, pipobroman, gacytosine, arabinoside, cyclophosphamide, thiotepa, paclitaxel, albumin-engineered nanoparticle formulation of paclitaxel, docetaxel, chlorambucil, gemcitabine, 6-thioguanine, mercaptopurine, cisplatin, carboplatin , vinblastine, platinum, etoposide, ifosfamide, mitoxantrone, vincristine, vinorelbine, novantrone, teniposide, edatrexate, daunomycin, aminopterin, xeloda, ibandronate, CPT-11, topoisomerase inhibitor RFS 2000, difluoromethylornithine, retinoic acid, capecitabine, or a pharmaceutically acceptable salt, solvate or acid thereof;
(b) monokines, lymphokines, traditional polypeptide hormones, parathyroid hormones, thyroxine, relaxin, prorelaxin, glycoprotein hormone, follicle stimulating hormone, thyroid stimulating hormone, luteinizing hormone, hepatic growth factor, fibroblast growth factor, prolactin, placental lactogen, tumor necrosis factor, tumor necrosis factor-α, tumor necrosis factor-β, mullerian inhibiting substance, mouse gonadotropin-associated peptide, inhibin, activin, vascular endothelial growth factor, thrombopoietin, erythropoietin, osteoinductive factor, interferon, interferon-α, interferon-β, interferon-γ, colony stimulating factor (CSF), macrophage-CSF, granulocyte-macrophage-CSF, granulocyte-CSF, interleukin (IL), IL-1, IL-1α, IL-2, IL-3, IL-4, IL-5, IL-6, IL-7, IL-8, IL-9, IL-10, IL- 11, IL-12, tumor necrosis factor, polypeptide factor, LIF, kit ligand, or a combination thereof;
(c) diphtheria toxin, botulinum toxin, tetanus toxin, decentretoxin, cholera toxin, amanitin, α-amanitin, pyrrolobenzodiazepines, pyrrolobenzodiazepine derivatives, indolinobenzodiazepines, pyridinobenzodiazepines, tetrodotoxin, brevetoxin, ciguatoxin, ricin, AM toxin, auristatin, tubulysin, geldanamycin, maytansinoid, calicheamycin, daunomycin, doxorubicin, methotrexate, vindesine, SG2285, dolastatin, dolastatin analog, cryptophycin, camptothecin, rhizoxin, rhizoxin derivatives, CC-1065, CC-1065, analogs or derivatives, duocarmycin, enediyne antibiotics, esperamicin, epothilone, toxoid, or a combination thereof;
(d) an affinity ligand, wherein the affinity ligand is a substrate, an inhibitor, an active agent, a neurotransmitter, a radioisotope, or a combination thereof;
(e) a radioactive label, 32P, 35S, a fluorescent dye, an electron dense reagent, an enzyme, biotin, streptavidin, dioxigenin, hapten, an immunogenic protein, a nucleic acid molecule with a sequence complementary to a target, or a combination thereof;
(f) an immunomodulatory compound, an anticancer agent, an anti-viral agent, an anti-bacterial agent, an anti-fungal agent, an anti-parasitic agent, or a combination thereof;
(g) tamoxifen, raloxifene, droloxifene, 4-hydroxytamoxifen, trioxifene, keoxifene, LY117018, onapristone, or toremifene;
(h) 4(5)-imidazole, aminoglutethimide, megestrol acetate, exemestane, letrozole, or anastrozole;
(i) flutamide, nilutamide, bicalutamide, leuprolide, goserelin, or troxacitabine;
(j) an aromatase inhibitor;
(k) a protein kinase inhibitor;
(l) a lipid kinase inhibitor;
(m) an antisense oligonucleotide;
(n) a ribozyme;
(o) a vaccine; and
(p) an anti-angiogenic agent.

42. The conjugate or the pharmaceutically acceptable salt or solvate thereof of claim 1, wherein:
the active agent is a pyrrolobenzodiazepine dimer;
position N10 of the pyrrolobenzodiazepine dimer is substituted with X or position N'10 is substituted with X' wherein X or X' links the pyrrolobenzodiazepine dimer to the linker;
X and X' are each independently -C(O)O-* or -C(O)-*; and
* refers to a binding site between the pyrrolobenzodiazepine dimer and the linker.

43. The conjugate or the pharmaceutically acceptable salt or solvate thereof of claim 42, wherein the pyrrolobenzodiazepine dimer is represented by General Formula X or General Formula XI below: 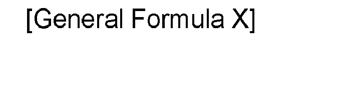 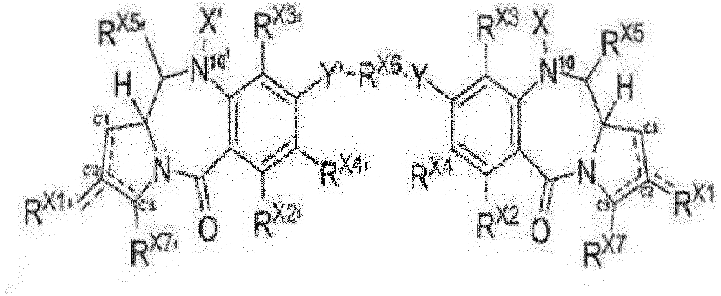 wherein,
a dotted line denotes an optional double bond between C1 and C2, between C2 and C3, between C'1 and C'2, or between C'2 and C'3;
R^{X1} and R^{X1}' are each independently selected from H, OH, =O, =CH₂, CN, R^{m}, OR^{m}, =CH-R^{m}', =C(R^{m}')₂, O-SO₂-R^{m}, CO₂R^{m}, COR^{m}, halo, or dihalo;
R^{X2}, R^{X2}', R^{X3}, and R^{X3}' are each independently selected from H, R^{m}, OH, OR^{m}, NR^{m}₂, NO₂, and halo;
R^{X4} and R^{X4}' are each independently selected from H, R^{m}, OH, OR^{m}, SH, SR^{m}, NH₂, NHR^{m}, NR^{m}₂, halo, and C₁₋₆ alkyl;
R^{X5} and R^{X5}' are each independently selected from H, R^{m}, OH, OR^{m}, SH, SR^{m}, NH₂, NHR^{m}, NR^{m}₂, -NR^{m}R^{m}', NO₂, -NR^{m}C(O)R^{m}', -NR^{m}C(O)OR^{m}', -NR^{m}C(O)NR^{m}R^{m}', - S(O)R^{m}, -S(O)₂R^{m}, -S(O)NR^{m}R^{m}', -S(O)₂NR^{m}R^{m}', -NR^{m}S(O)R^{m}', -NR^{m}S(O)₂R^{m}', -P(O)R^{m}, -P(O)₂R^{m}, -P(O)NR^{m}R^{m}', -P(O)₂NR^{m}R^{m}', -NR^{m}P(O)R^{m}', -NR^{m}P(O)₂R^{m}', and halo;
Y and Y' are each independently O, S, or N(H);
R^{X6} is independently C₃₋₁₂ alkylene, C₃₋₁₂ alkenylene, or C₃₋₁₂ heteroalkylene;
R^{X6} is unsubstituted or substituted with -NH₂, -NHR^{m}, -NHC(O)R^{m}, -NHC(O)CH₂-[OCH₂CH₂]ₙ-R^{XX}, or -[CH₂CH₂O]ₙ-R^{XX};
R^{XX} is H, OH, N₃, CN, NO₂, SH, NH₂, ONH₂, NHNH₂, halo, C₁₋₈alkyl, C₃₋₈cycloalkyl, C₁₋₈ alkoxy, C₁₋₈ alkylthio, C₃₋₂₀ heteroaryl, C₅₋₂₀ aryl, or mono- or di-C₁₋₈ alkylamino, wherein n is an integer from 1 to 6;
R^{X7} and R^{X7}' are each independently H, C₁₋₆ alkyl, C₂₋₆ alkenyl, C₂₋₆ alkynyl, - C(O)R^{r}, -C(O)OR^{s}, or -C(O)NR^{r}R^{r}';
R^{r}, R^{r}', and R^{s} are each independently H, C₁₋₇ alkyl, C₂₋₇ alkenyl, C₂₋₇ alkynyl, C₃₋₁₃ cycloalkyl, 3- to 7-membered heterocycloalkyl, C₅₋₁₀ aryl, or 5- to 7-membered heteroaryl;
each R^{m}' is independently selected from R^{m}, CO₂R^{m}, COR^{m}, CHO, CO₂H, and halo; and
each R^{m} is independently selected from the group consisting of H, OH, C₁₋₁₂ alkyl, C₁₋₁₂ alkoxy, C₂₋₁₂ alkenyl, C₂₋₁₂ alkynyl, C₅₋₂₀ aryl, C₅₋₂₀ heteroaryl, C₃₋₆ cycloalkyl, 3- to 7-membered heterocyclyl, 3- to 7-membered heterocycloalkyl, and 5- to 7-membered heteroaryl, wherein at least one hydrogen atom of C₅₋₂₀ aryl, C₅₋₂₀ heteroaryl, C₃₋₆ cycloalkyl, 3- to 7-membered heterocyclyl, 3- to 7-membered heterocycloalkyl, and 5- to 7-membered heteroaryl is substituted with OH, =O, C₁₋₁₂ alkyl, or C₁₋₁₂ alkoxy.

44. The conjugate or the pharmaceutically acceptable salt or solvate thereof of claim 43, wherein R^{X1} and R^{X1}' are each independently selected from: =CH₂; C₁₋₆ alkyl; C₁₋₆ alkoxy; C₂₋₆ alkenyl; C₅₋₇ aryl; C₃₋₆ heteroaryl; or C₅₋₇ aryl substituted with C₁₋₆ alkyl or C₁₋₆ alkoxy.

45. The conjugate or the pharmaceutically acceptable salt or solvate thereof of claim 43, wherein R^{X2}, R^{X2}', R^{X3}, and R^{X3}' are each independently selected from H or OH.

46. The conjugate or the pharmaceutically acceptable salt or solvate thereof of claim 43, wherein R^{X5} and R^{X5}' are each independently selected from the group consisting of H, OH, -S(O)R^{m}, S(O)₂R^{m}, -P(O)R^{m}, and -P(O)₂R^{m}, wherein R^{m} is H, OH, C₁₋₁₂ alkyl, C₁₋₁₂ alkoxy, C₂₋₁₂ alkenyl, or C₂₋₁₂ alkynyl.

47. The conjugate or the pharmaceutically acceptable salt or solvate thereof of claim 43, wherein R^{X4} and R^{X4}' are each independently C₁₋₆ alkoxy.

48. The conjugate or the pharmaceutically acceptable salt or solvate thereof of claim 43, wherein Y and Y' are O.

49. The conjugate or the pharmaceutically acceptable salt or solvate thereof of claim 43, wherein:
R^{X6} is C₃₋₁₂ alkylene, C₃₋₁₂ alkenylene, or C₃₋₁₂ heteroalkylene, and R^{X6} is substituted with -NH₂, -NHR^{m}, -NHC(O)R^{m}, -NHC(O)CH₂-[OCH₂CH₂]ₙ-R^{XX}, or - [CH₂CH₂O]ₙ-R^{XX};
R^{XX} is H, OH, N₃, CN, NO₂, SH, NH₂, ONH₂, NHNH₂, halo, C₁₋₈ alkyl, C₃₋₈ cycloalkyl, C₁₋₈ alkoxy, C₁₋₈ alkylthio, C₃₋₂₀ heteroaryl, C₅₋₂₀ aryl, or mono- or di-C₁₋₈ alkylamino; and
n is an integer from 1 to 6.

50. The conjugate or the pharmaceutically acceptable salt or solvate thereof of claim 1 or 10, wherein [Linker-(B)ₗ] is selected from the group consisting of: 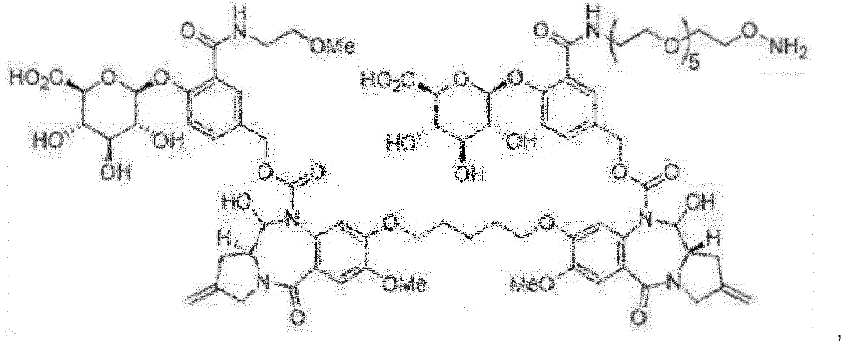 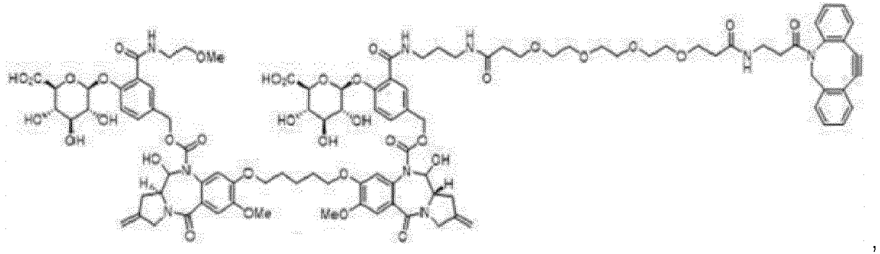 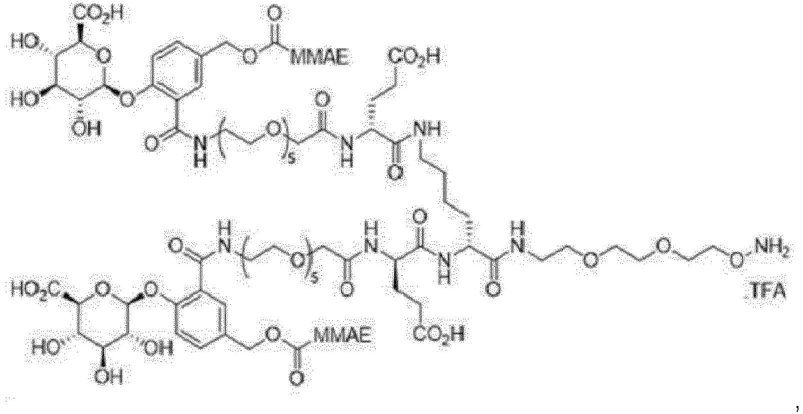 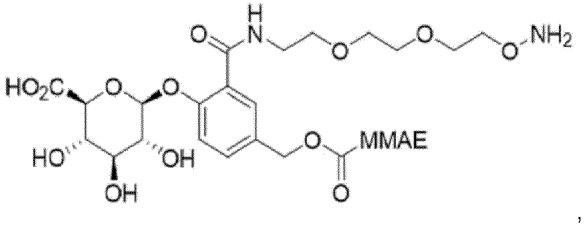 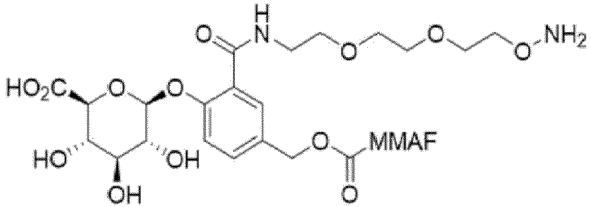 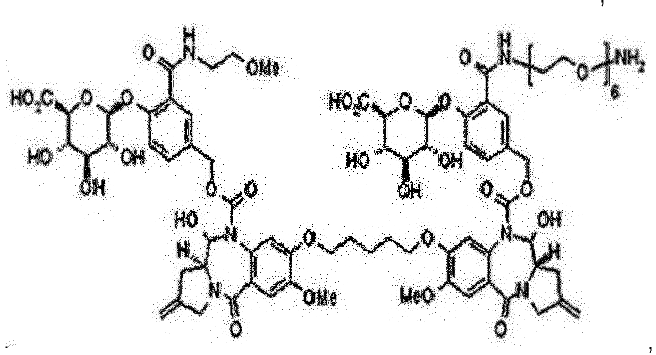 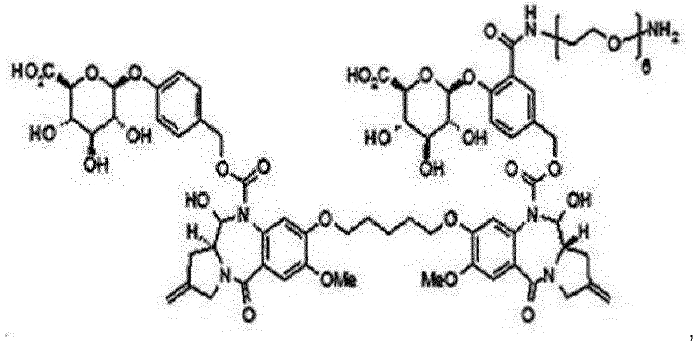 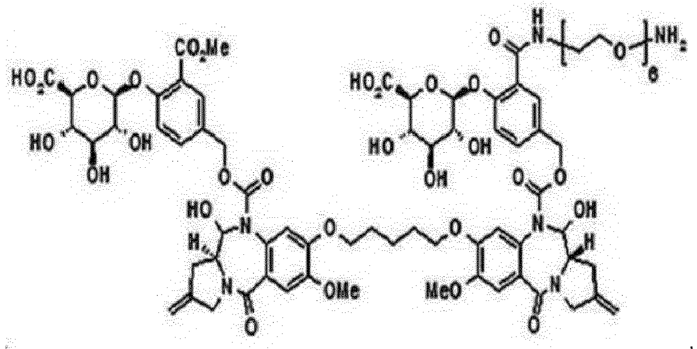 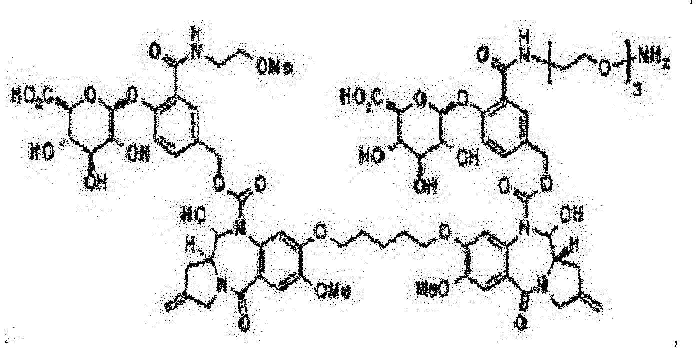 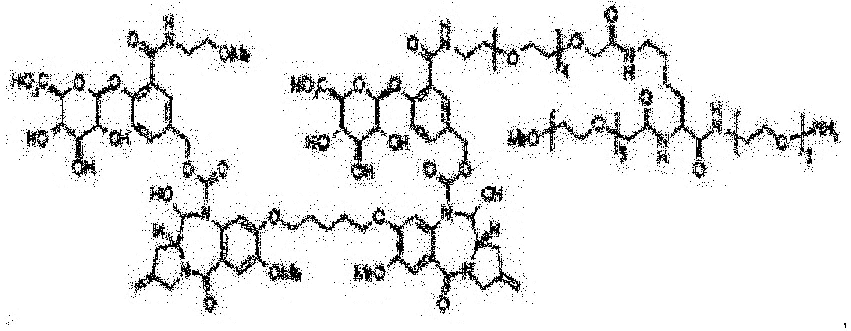 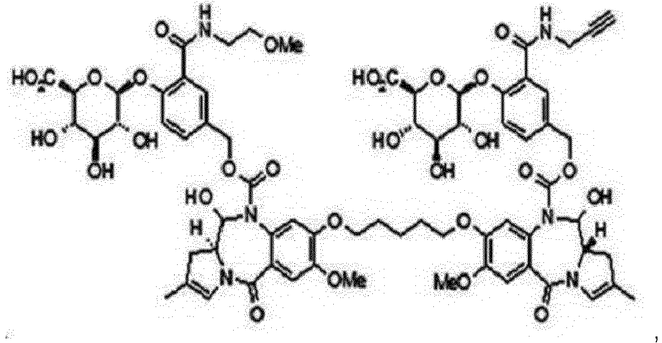 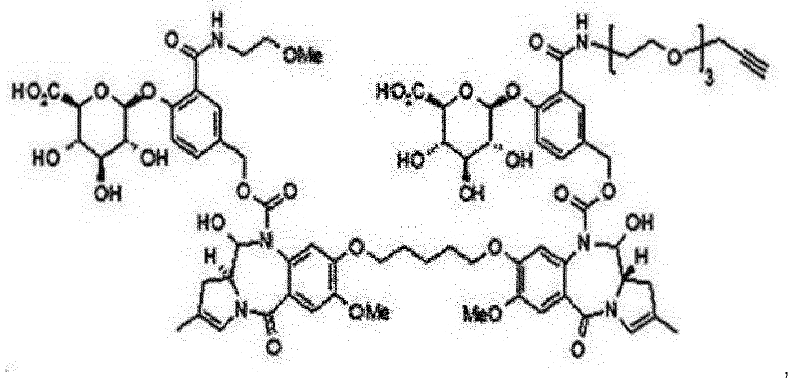 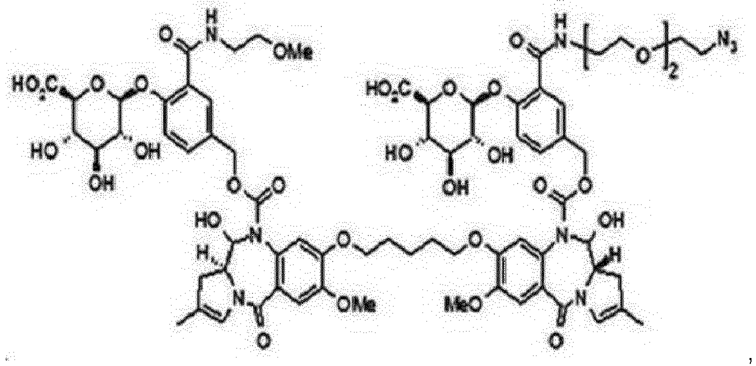 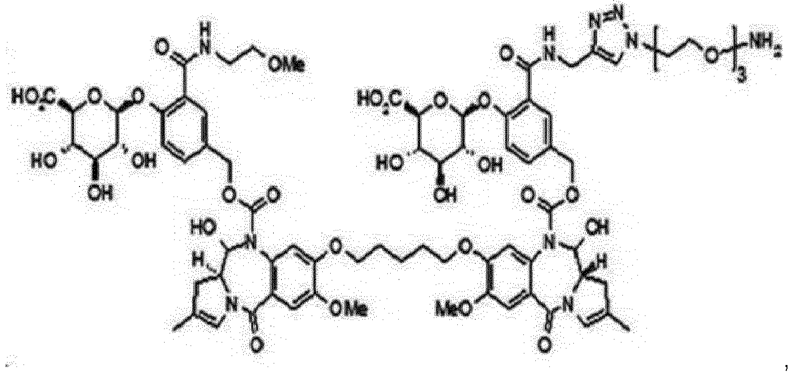 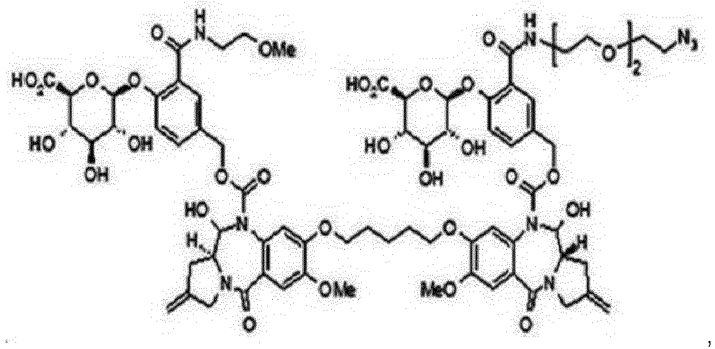 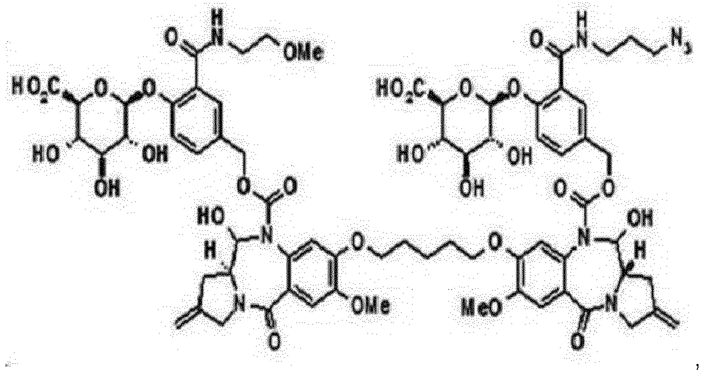 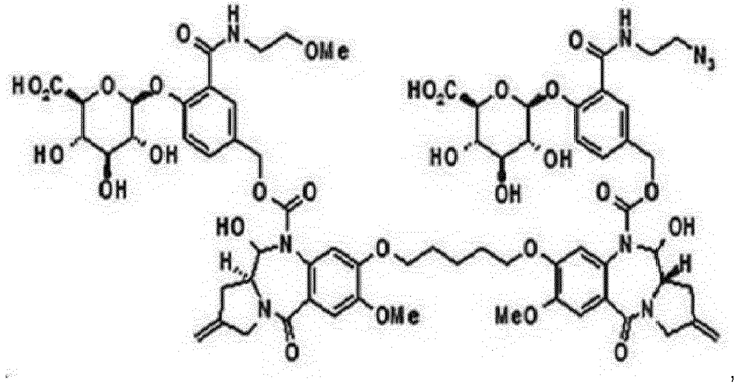 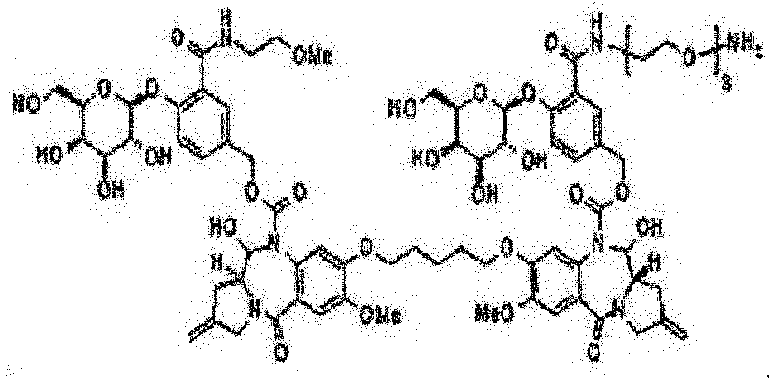 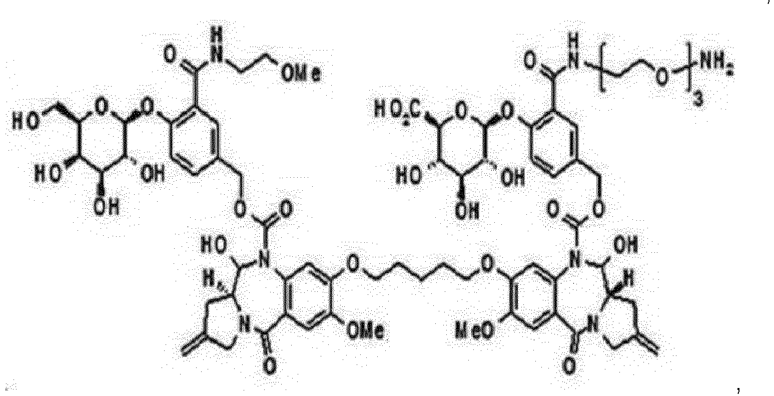 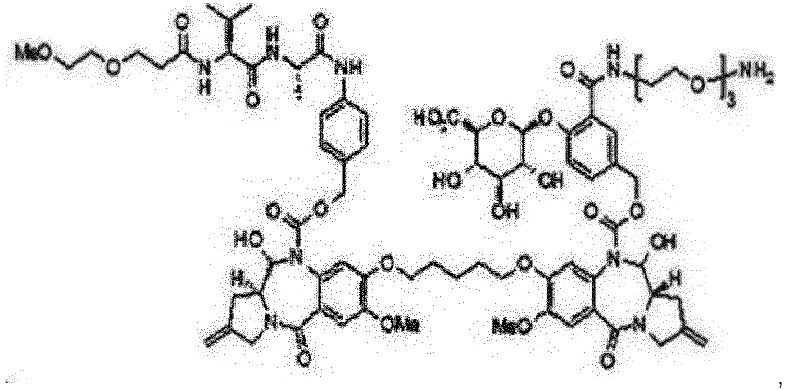 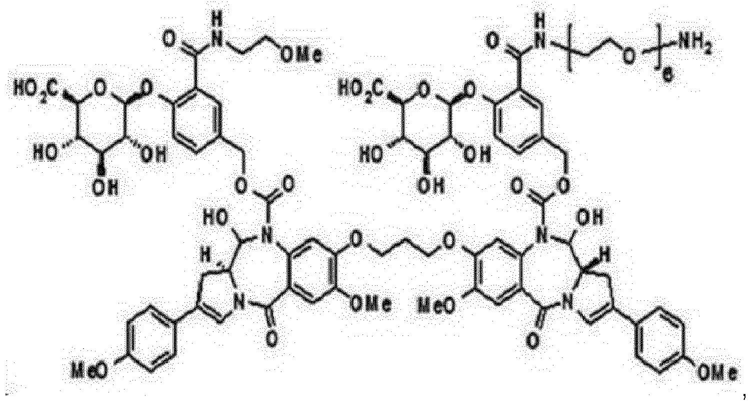 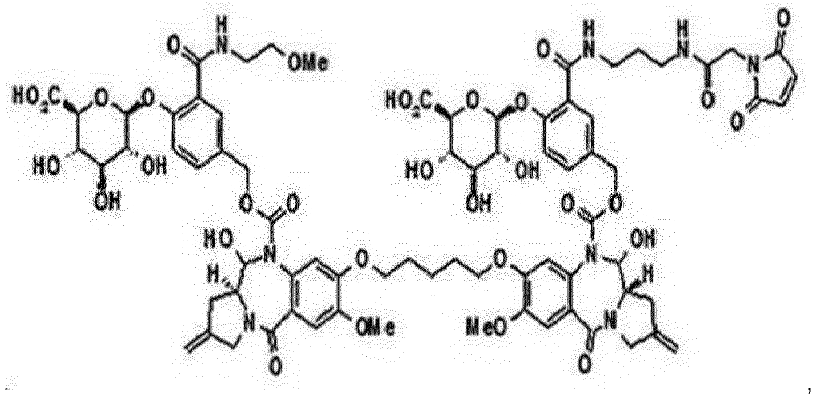 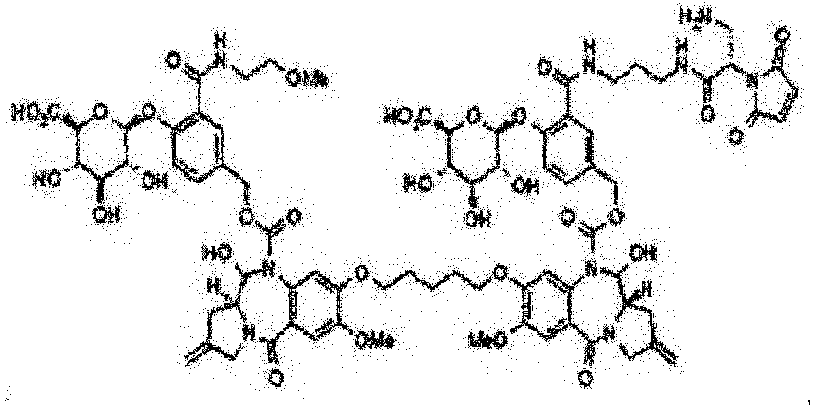 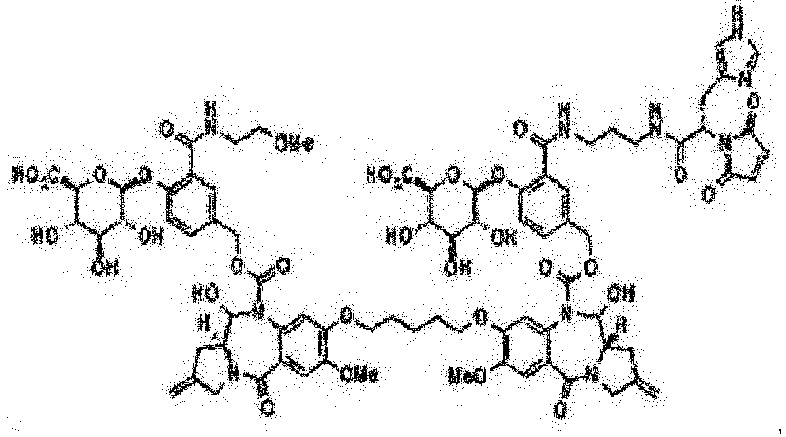 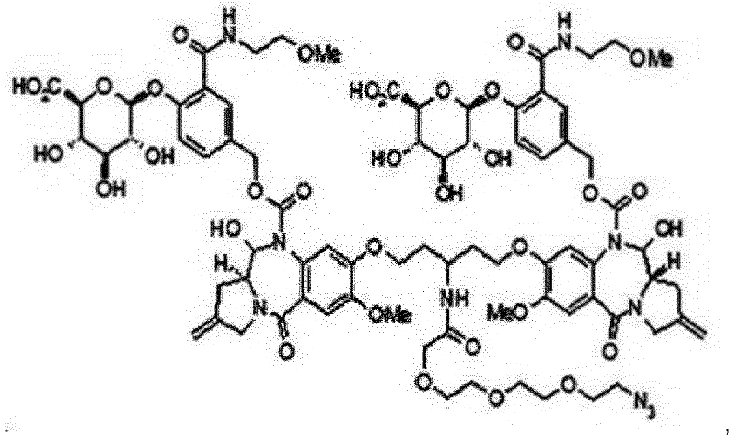 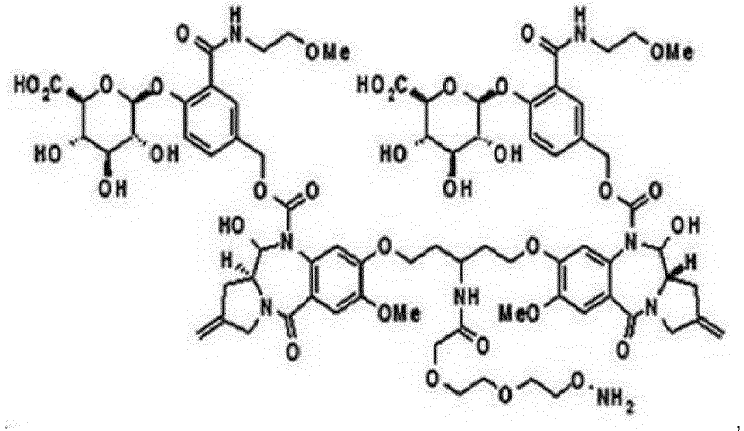 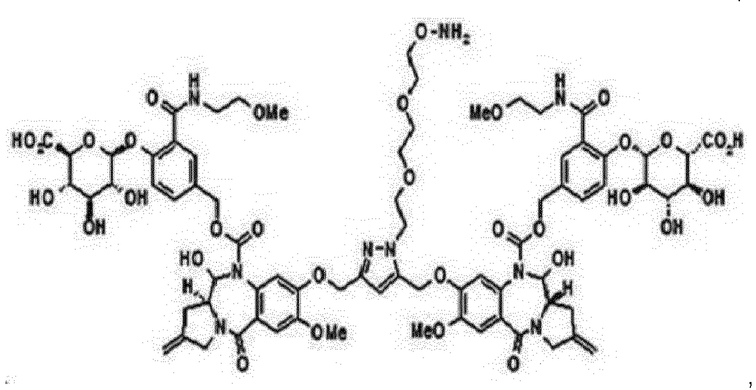 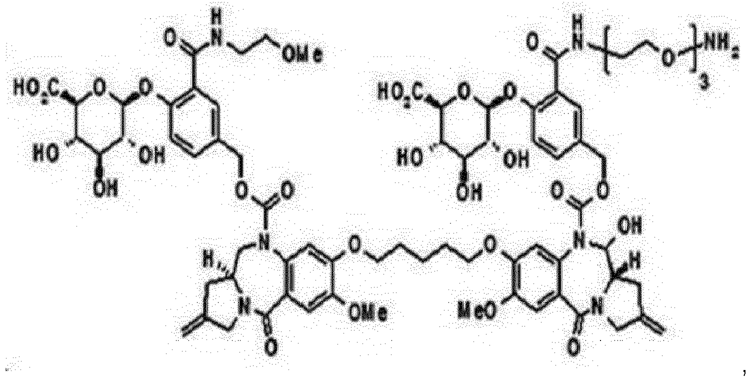 and 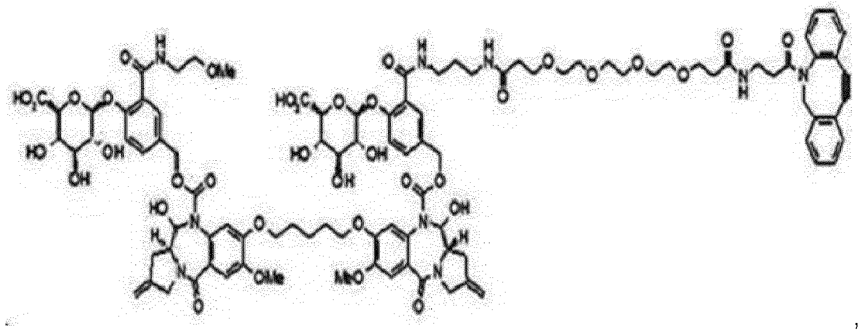
wherein MMAE is monomethyl auristatin E, and
MMAF is monomethyl auristatin F.

51. A pharmaceutical composition for the prevention or treatment of hyperproliferation, cancer, or an angiogenic disease, the pharmaceutical composition comprising the conjugate of claim 1 or 10.

52. The pharmaceutical composition of claim 51, further comprising a pharmaceutically effective amount of a chemotherapeutic agent.

53. The pharmaceutical composition of claim 51, wherein the cancer is any one selected from lung cancer, small cell lung cancer, gastrointestinal cancer, colon cancer, bowel cancer, breast cancer, ovarian cancer, prostate cancer, testicular cancer, liver cancer, kidney cancer, bladder cancer, pancreatic cancer, brain cancer, sarcoma, osteosarcoma, Kaposi's sarcoma, and melanoma.

54. A pharmaceutical preparation comprising the conjugate of claim 1 or 10, and a pharmaceutically acceptable carrier, the pharmaceutical preparation being selected from injections, tablets, pills, powders, granules, capsules, troches, suspending agents, liquids for internal use, emulsions, syrups, freeze-dried preparations, and suppositories.
